(19) Europäisches Patentamt
European Patent Office
Office européen des brevets

(11) Veröffentlichungsnummer: **0 061 425 A2**

# (12) EUROPÄISCHE PATENTANMELDUNG

(21) Anmeldenummer: 82810111.3

(22) Anmeldetag: 12.03.82

(51) Int. Cl.[3]: **C 07 D 277/36**
C 07 D 417/04, C 07 D 417/12
C 07 D 513/04, A 61 K 31/425
//(C07D513/04, 277/00, 277/00)

(30) Priorität: 18.03.81 CH 1838/81

(43) Veröffentlichungstag der Anmeldung: 29.09.82 Patentblatt 82/39

(84) Benannte Vertragsstaaten: AT BE CH DE FR IT LI LU NL SE

(71) Anmelder: CIBA-GEIGY AG
Patentabteilung Postfach
CH-4002 Basel(CH)

(72) Erfinder: Ferrini, Pier Giorgio, Dr.
Im Rehwechsel 22
CH-4102 Binningen(CH)

(72) Erfinder: Göschke, Richard, Dr.
Felixhäglistrasse 21
CH-4103 Bottmingen(CH)

## (54) Neue Thiazolderivate, Verfahren zu ihrer Herstellung, diese enthaltende pharmazeutische Präparate und ihre Verwendung.

(57) 2,4,5-trisubstituierte Thiazole der Formel

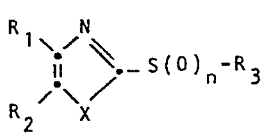
(1),

worin $R_1$ und $R_2$ unabhängig voneinander unsubstituierte oder durch aliphatische Kohlenwasserstoffreste, freies, veräthertes oder verestertes Hydroxy, veräthertes, gegebenenfalls S-oxidiertes Mercapto, aliphatisch substituiertes Amino und/oder Trifluormethyl substituierte Aryl- oder gegebenenfalls N-oxidierte Heteroarylreste bedeuten, X Thio bedeutet, n für 0, 1 oder 2 steht und $R_3$ einen unsubstituierten oder durch veräthertes oder verestertes Hydroxy, veräthertes gegebenenfalls S-oxidiertes Mercapto und/oder in höherer als der α-Stellung durch Oxo und/oder Hydroxy substituierten, gegebenenfalls zusätzlich zu endständig gebundenem Hydroxy in Salzform vorliegendes Sulfo aufweisenden, aliphatischen, einen unsubstituierten oder im Arylteil wie für $R_1$ bzw. $R_2$ angegeben substituierten araliphatischen oder einen unsubstitueierten oder in höherer als der α-Stellung durch freies, veräthertes oder verestertes Hydroxy substituierten cycloaliphatischen oder cycloaliphatisch-aliphatischen Kohlenwasserstoffrest bedeutet oder eine Gruppe der Formel

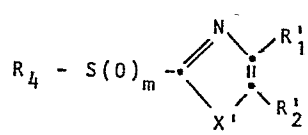
(1a)

darstellt, in der m für 0, 1 oder 2 steht, $R_4$ einen unsubstituierten oder durch veräthertes oder verestertes Hydroxy, gegebenenfalls S-oxidiertes veräthertes Mercapto oder in höherer als der α- und in niederer als der ω-Stellung durch Oxo und/oder Hydroxy substituierten aliphatischen, gegebenenfalls durch Oxa oder Thia, welches auch S-oxidiert sein kann, unterbrochenen Kohlenwasserstoffrest oder, sofern n und m für 0 stehen, eine direkte Bindung darstellt und $R'_1$ und $R'_2$ eine der für $R_1$ bzw. $R_2$ angegebenen Bedeutungen haben, und ihre pharmazeutisch verwendbaren Salze eignen sich zur Bekämpfung rheumatischer Erkrankungen, vorzugsweise als Wirkstoff in anti-rheumatischen Arzneimitteln. Die Erfindung betrifft derartige Arzneimittel und nicht-chemische Verfahren zu ihrer Herstellung, ebenso neue Verbindungen der Formel I und Verfahren zu ihrer Herstellung.

EP 0 061 425 A2

CIBA-GEIGY AG
Basel (Schweiz)

4-13330/+

# Neue Thiazolderivate, Verfahren zu ihrer Herstellung, diese enthaltende pharmazeutische Präparate und ihre Verwendung

Die Erfindung betrifft die Verwendung von 2,4,5-trisubstituierten Thiazolen der Formel

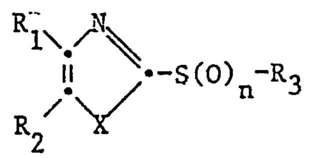

(I),

worin $R_1$ und $R_2$ unabhängig voneinander unsubstituierte oder durch aliphatische Kohlenwasserstoffreste, freies, veräthertes oder verestertes Hydroxy, veräthertes, gegebenenfalls S-oxidiertes Mercapto, aliphatisch substituiertes Amino und/oder Trifluormethyl substituierte Aryl- oder gegebenenfalls N-oxidierte Heteroarylreste bedeuten, X Thio bedeutet, n für 0, 1 oder 2 steht und $R_3$ einen unsubstituierten oder durch veräthertes oder verestertes Hydroxy, veräthertes gegebenenfalls S-oxidiertes Mercapto und/oder in höherer als der α-Stellung durch Oxo und/oder Hydroxy substituierten, gegebenenfalls zusätzlich zu endständig gebundenem Hydroxy in Salzform vorliegendes Sulfo aufweisenden aliphatischen einen unsubstituierten oder im Arylteil wie für $R_1$ bzw. $R_2$ angegeben substituierten araliphatischen oder einen unsubstituierten oder in höherer als der α-Stellung durch freies, veräthertes oder verestertes Hydroxy substituierten cycloaliphatischen oder cycloaliphatisch-aliphatischen Kohlenwasserstoffrest bedeutet oder eine Gruppe der Formel

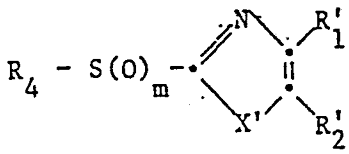 (Ia)

darstellt, in der m für 0, 1 oder 2 steht, $R_4$ einen unsubstituierten oder durch veräthertes oder verestertes Hydroxy, gegebenenfalls S-oxidiertes veräthertes Mercapto oder in höherer als der α- und in niederer als der ω-Stellung durch Oxo und/oder Hydroxy substituierten aliphatischen, gegebenenfalls durch Oxa oder Thia, welches auch S-oxidiert sein kann, unterbrochenen Kohlenwasserstoffrest oder, sofern n und m für 0 stehen, eine direkte Bindung darstellt und $R_1'$ und $R_2'$ eine der für $R_1$ bzw. $R_2$ angegebenen Bedeutungen haben, mit der Massgabe, dass in Verbindungen, worin n für 1 steht, mindestens einer der Reste $R_1$ und $R_2$ von unsubstituierten oder durch Alkyl, Alkoxy, Halogen und/oder Trifluormethyl substituiertem Phenyl verschieden ist, wenn $R_3$ Alkyl bedeutet, und ihre pharmazeutisch verwendbaren Salze zur Anwendung in einem Verfahren zur therapeutischen Behandlung des menschlichen oder tierischen Körpers, ihre Verwendung zur Bekämpfung von Krankheiten, insbesondere als Wirkstoff in oder zur Herstellung von pharmazeutischen Präparaten, diese enthaltende pharmazeutische Präparate und nicht-chemische Verfahren in ihrer Herstellung.

Die Erfindung betrifft gleichermassen Verbindungen der Formel I, worin $R_1$, $R_2$, X, n, $R_3$, $R_4$, m, $R_1'$ und $R_2'$ obige Bedeutungen haben, selbst, mit der Massgabe, dass in Verbindungen, worin n für 0 oder 1 steht, mindestens einer der Reste $R_1$ und $R_2$ von unsubstituierten oder durch Alkyl,

Alkoxy, Halogen und/oder Trifluormethyl substituiertem Phenyl verschieden ist, wenn $R_3$ Alkyl bedeutet, mit der weiteren Massgabe, dass in Verbindungen, worin n für 0 steht, mindestens einer der Reste $R_1$ und $R_2$ einen unsubstituierten oder wie angegeben substituierten Heteroarylrest bedeutet, wenn $R_3$ $\omega$-Hydroxy- oder $\omega$-Oxoniederalkyl mit mehr als 3 Kohlenstoffatomen darstellt, bzw. mindestens einer der Reste $R_1$ und $R_2$ einen unsubstituierten oder wie angegeben substituierten Heteroarylrest oder beide Reste $R_1$ und $R_2$ zugleich p-Methoxyphenyl bedeuten, wenn $R_3$ $\omega$-Hydroxy- oder $\omega$-Oxoniederalkyl mit 2 oder 3 Kohlenstoffatomen darstellt, und ihre Salze sowie Verfahren zu ihrer Herstellung.

Aliphatische Kohlenwasserstoffreste sind einwertig bzw. im Falle von Resten $R_4$ zweiwertig. Einwertige aliphatische Kohlenwasserstoffreste sind beispielsweise Niederalkylreste, im Falle von $R_3$ ferner Niederalkenyl- oder Niederalkinylreste. Zweiwertige aliphatische Kohlenwasserstoffreste sind beispielsweise Niederalkylen- oder Niederalkenylenreste.

Veräthertes Hydroxy ist beispielsweise mit einem aliphatischen Alkohol veräthertes Hydroxy, wie Niederalkoxy, Niederalkenyloxy, Niederalkinyloxy oder, im Falle zweier an einen aliphatischen Rest oder an vicinale Kohlenstoffatome eines Aryl- bzw. Heteroarylrest gebundener verätherter Hydroxygruppen, Niederalkylendioxy.

Verestertes Hydroxy ist beispielsweise mit einer Halogenwasserstoffsäure oder einer organischen Carbonsäure verestertes Hydroxy, wie Halogen, Niederalkanoyloxy oder Benzoyl- bzw. Pyridoyloxy, welches wie für $R_1$ und $R_2$ angegeben z.B. durch Niederalkyl, Niederalkoxy und/oder Halogen, substituiert sein kann.

Veräthertes Mercapto ist beispielsweise aliphatisch veräthertes Mercapto, wie Niederalkylthio oder, im Falle zweier an einen aliphatischen Rest oder an vicinale Kohlenstoffatome eines Aryl-

bzw. Heteroarylrestes gebundener verätherter Mercaptogruppen, Niederalkylendithio. S-oxidiertes veräthertes Mercapto ist beispielsweise Niederalkansulfinyl bzw. Niederalkansulfonyl.

Aliphatisch substituiertes Amino ist beispielsweise durch Niederalkyl oder Niederalkylen bzw. Aza-, Oxa- oder Thia-niederalkylen substituiertes Amino, wie Mono- oder Diniederalkylamino oder 4- bis 7-gliedriges Niederalkylenamino bzw. 3-Aza-, 3-Oxa- oder 3-Thianiederalkylenamino.

Arylreste sind beispielsweise Arylreste mit 6 bis und mit 10 Kohlenstoffatomen (C-Atomen) als Ringgliedern, insbesondere Phenyl, ferner Naphthyl.

Gegebenenfalls N-oxidierte Heteroarylreste sind beispielsweise monocyclische, ein Sauerstoff- oder Schwefelatom und gegebenenfalls zusätzlich ein Stickstoffatom aufweisende 5-gliedrige oder gegebenenfalls N-oxidierte ein oder zwei Stickstoffatom(e) aufweisende 6-gliedrige Heteroarylreste, wie Furyl, z.B. 2-Furyl, Thienyl, z.B. 2-Thienyl, Thiazolyl, z.B. 2-Thiazolyl gegebenenfalls N-oxidiertes Pyridyl, z.B. 2-, 3- oder 4-Pyridyl oder 3- oder 4-(1-Oxido)-pyridyl, oder Pyrimidyl, wie 2-Pyrimidyl.

Unsubstituierte oder durch veräthertes oder verestertes Hydroxy, veräthertes gegebenenfalls S-oxidiertes Mercapto und/oder in höherer als der α-Stellung durch Oxo und/oder Hydroxy substituierte aliphatische Kohlenwasserstoffreste sind im Falle von $R_3$ einwertige, gegebenenfalls wie angegeben substituierte und im Falle von $R_4$ zweiwertige, gegebenenfalls wie angegeben substituierte und gegebenenfalls durch Oxa bzw. gegebenenfalls S-oxidiertes Thia unterbrochene Kohlenwasserstoffreste. Einwertige aliphatische

Kohlenwasserstoffreste sind dabei beispielsweise Niederalkyl-, Niederalkenyl- oder Niederalkinylreste. Als zweiwertige gegebenenfalls durch Oxa oder gegebenenfalls S-oxidiertes Thia unterbrochene aliphatische Kohlenwasserstoffreste kommen z.B. Niederalkylen, Oxaniederalkylen oder gegebenenfalls S-oxidiertes Thianiederalkylen in Betracht. Als Beispiele für Reste $R_3$ dieser Art seine z.B. genannt: Niederalkyl, Niederalkenyl oder Niederalkinyl, Mono- oder Diniederalkoxyniederalkyl, Niederalkylendioxyniederalkyl, Niederalkylidendioxyniederalkyl, die Niederalkanoylgruppe(n) in höherer als der α-Stellung tranges Mono- oder Diniederalkanoyloxyniederalkyl, Polyhalogenniederalkyl, Mono- oder Diniederalkylthioniederalkyl, Niederalkansulfinyl- bzw. Niederalkansulfonylniederalkyl, Niederalkylendithioniederalkyl, die Hydroxygruppe(n) bzw. die Niederalk(anaoyl)-oxygruppe in höherer als der α-Stellung tragenes Mono- oder Dihydroxyniederalkyl, Hydroxyniederalkoxyniederalkyl und Niederalkanoyloxyniederalkoxyniederalkyl, in Salzform vorliegendes ω-Hydroxy-ω-sulfoniederalkyl, ω-Hydroxy-ω-sulfo-niederalkenyl und ω-Hydroxy-ω-sulfoniederalkinyl, ebenso Niederalkoxyniederalkoxyniederalkyl, Niederalkylthioniederalkoxyniederalkyl, Hydroxyniederalkylthioniederalkyl, Niederalkoxyniederalkylthioniederalkyl oder Niederalkanoyloxyniederalkylthioniederalkyl und die Oxogruppe in höherer als der α-Stellung tragendes Oxoniederalkyl. Als Reste $R_4$ der vorstehend bezeichneten Art kommen beispielsweise in Betracht: Niederalkylen, über ein gesätttigtes C-Atom gebundenes Niederalkenylen, Niederalkoxyniederalkylen, die Niederalkanoyloxygruppe in höherer als der α- und in niederer als der ω-Stellung tragendes Mono- oder Diniederalkanoyloxyniederalkylen, Polyhalogenniederalkylen, Niederalkylthioniederalkylen, und die Hydroxy- bzw. Oxogruppe in höherer als der α- und in niederer als der ω-Stellung tragendes Mono- oder Dihydroxy- bzw. Oxoniederalkylen, ferner Oxoniederalkylen und gegebenenfalls S-oxidiertes Thianiederalkylen.

Im Arylteil gegebenenfalls wie für $R_1$ und $R_2$ angegeben substituierte araliphatische Kohlenwasserstoffreste sind beispielsweise unsubstituierte oder wie angegeben, insbesondere durch Niederalkyl, Niederalkoxy, Halogen und/oder Trifluormethyl, substituierte Phenylniederalkylreste.

Gegebenenfalls in höherer als der α-Stellung durch freies, veräthertes oder verestertes Hydroxy substituierte cycloaliphatische bzw. cycloaliphatisch-aliphatische Kohlenwasserstoffreste sind beispielsweise Cycloalkyl- bzw. Cycloalkylniederalkylreste, oder die Hydroxy-, Niederalkoxy- bzw. Niederalkanoyloxygruppe in höherer als der α-Stellung tragendes Hydroxycycloalkyl, Niederalkoxycycloalkyl oder Niederalkanoyloxycycloalkyl oder Cycloalkyl-(hydroxy)-niederalkyl mit jeweils 3 bis 8, vorzugsweise 5 bis 7 Ringgliedern.

Vor- und nachstehend sind unter "niederen" organischen Resten und Verbindungen vorzugsweise solche zu verstehen, die bis und mit 7, vor allem bis und mit 4, Kohlenstoffatome (C-Atome) enthalten.

Niederalkyl ist beispielsweise Methyl, Aethyl, Propyl, Isopropyl, Butyl, Isobutyl, Sekundärbutyl oder Tertiärbutyl, ferner Pentyl, Hexyl oder Heptyl.

Niederalkenyl ist beispielsweise Vinyl, vorzugsweise jedoch über ein gesättigtes C-Atom gebundenes Niederalkenyl, wie Allyl, Methallyl oder But-2-enyl.

Niederalkinyl ist beispielsweise Aethinyl, vorzugsweise jedoch über ein gesättigtes C-Atom gebundenes Niederalkinyl, wie Propargyl oder But-2-inyl.

Niederalkoxy ist beispielsweise Methoxy, Aethoxy, Propyloxy, Isopropyloxy, Butyloxy, Isobutyloxy, Sekundärbutyloxy oder Tertiätbutyloxy, ferner Pentyloxy, Hexyloxy oder Heptyloxy.

Niederalkenyloxy ist beispielsweise Allyloxy, und Niederalkinyloxy ist beispielsweise Propargyloxy.

Niederalkylendioxy ist beispielsweise geminal gebundenes Aethylendioxy, 1,3-Propylendioxy, 2,3-Butylendioxy oder 1,3-(2,2-Dimethyl)-propylendioxy.

Niederalkylidendioxy ist beispielsweise vicinal gebundenes Methylendioxy, Aethylidendioxy oder Isopropylidendioxy.

Niederalkanoyloxy ist beispielsweise Acetoxy, Propionyloxy, Butyryloxy, Isobutyryloxy, ferner Formyloxy oder Pivaloyloxy.

Niederalkylthio ist beispielsweise Methylthio, Aethylthio, Propylthio, Isopropylthio, Butylthio, Isobutylthio, Sekundärbutylthio oder Tertiärbutylthio, ferner Pentylthio, Hexylthio oder Heptylthio.

Niederalkylendithio ist beispielsweise geminal gebundenes Aethylendithio, 1,3-Propylendithio oder 1,3-(2,2-Dimethyl)-propylendithio.

Niederalkylidenthio ist beispielsweise vicinal gebundenes Methylendithio, Aethylidendithio oder Isopropylidendithio.

Niederalkansulfinyl ist beispielsweise Methan-, Aethan-, 1- oder 2-Propan-, Butan- oder Isobutansulfinyl.

Niederalkansulfonyl ist beispielsweise Methan-, Aethan-, 1- oder 2-Propan-, Butan- oder Isobutansulfonyl.

Mono- oder Diniederalkylamino ist beispielsweise Methylamino, Dimethylamino, Aethylamino, Diäthylamino, Propylamino oder Butylamino.

4- bis 7-gliedriges Niederalkylenamino bzw. 3-Aza-, 3-Oxa- oder 3-Thianiederalkylenamino ist beispielsweise Pyrrolidino, Piperidino, Morpholino, Thiomorpholino oder Piperazino bzw. 4-Methyl- oder 4-Aethyl-piperazino.

Niederalkylen ist beispielsweise Aethylen, 1,3-Propylen, 1,4-Butylen oder 1,3-(2,2-Dimethyl)-propylen, ferner 1,5-Pentylen, 1,6-Hexylen oder 1,7-Heptylen.

Oxaniederalkylen ist beispielsweise 1,5-(3-Oxa)-pentylen, 1,6-(3-Oxa)-hexylen oder 1,7-(4-Oxa)-heptylen, ferner 1,4-(2-Oxa)-butylen oder oder 1,5-(2-Oxa)-pentylen.

Gegebenenfalls S-oxidiertes Thianiederalkylen ist beispielsweise gegebenenfalls S-mono- oder S,S-dioxidiertes 1,5-(3-Thia)-pentylen, 1,6-(3-Thia)-hexylen oder 1,7(4-Thia)-heptylen, ferner gegebenenfalls S-mono- oder S,S-dioxidiertes 1,4-(2-Thia)-butylen oder 1,5-(2-Thia)-pentylen.

Mono- oder Diniederalkoxyniederalkyl ist beispielsweise 2-Methoxy-, 2-Aethoxy-, 2-Propyloxy- oder 2-Isopropyloxy-äthyl, 3-Methoxy- oder 3-Aethoxy-propyl oder 3,3-Dimethoxy-, 3,3-Diäthoxy-, 2,3-Dimethoxy- oder 2,3-Diäthoxypropyl oder 4,4-Dimethoxy-butyl, ferner Methoxy-, Aethoxy-, Dimethoxy- oder Propyloxy- oder Isopropyloxymethyl.

Niederalkylendioxyniederalkyl ist beispielsweise 2,2-Aethylendioxy- oder 2,2-Propylendioxy-äthyl oder 3,3- oder 2,3-Aethylendioxy-propyl, ferner Aethylendioxy- oder Propylendioxymethyl.

Niederalkylidendioxyniederalkyl ist beispielsweise 2,3-Methylendioxy-, 2,3-Aethylidendioxy- oder 2,3-Isopropylidendioxy-propyl.

Die Niederalkanoyloxygruppe(n) in höherer als der α-Stellung tragendes Mono- oder Diniederalkanoyloxyniederalkyl ist beispielsweise 2-Acetoxy-äthyl oder 3-Acetoxy- oder 2,3-Diacetoxy-propyl, kann aber auch 2,3-Succinyldioxypropyl sein.

Polyhalogenniederalkyl weist als Halogen insbesondere Fluor auf und bedeutet beispielsweise 1,1,2,2-Tetrafluor- oder 1,1,2,2,2-Pentafluoräthyl, ferner Trifluormethyl.

Mono- oder Diniederalkylthioniederalkyl bedeutet beispielsweise 2-Methylthio-, 2-Aethylthio-, 2-Propylthio oder 2-Isopropylthioäthyl, 3-Methylthio- oder 3-Aethylthio-propyl oder 3,3-Bis(methylthio)-, 3,3-Bis(äthylthio)-, 2,3-Bis(methylthio)- oder 2,3-Bis(äthylthio)-propyl oder 4,4-Bis(methylthio)-butyl, ferner Methylthio-, Aethylthio-, Bis(methylthio)-, Propylthio- oder Isopropylthio-methyl.

Niederalkansulfinylniederalkyl ist beispielsweise 2-Methansulfinyl-, 3-Aethansulfinyl-, 2-Propansulfinyl- oder 2-(2-Propansulfinyl)-äthyl, 3-Methansulfinyl- oder 3-Aethansulfinyl-propyl, ferner Methansulfinyl-, Aethansulfinyl- oder 1- oder 2-Propansulfinyl-methyl.

Niederalkansulfonylniederalkyl ist beispielsweise 2-Methansulfonyl-, 3-Aethansulfonyl-, 2-Propansulfonyl- oder 2-(2-Propansulfonyl)-äthyl, 3-Methansulfonyl- oder 3-Aethansulfonyl-propyl, ferner Methansulfonyl-, Aethansulfonyl- oder 1- oder 2-Propansulfonyl-methyl.

Niederalkylendithioniederalkyl ist beispielsweise 2,2-Aethylendithio- oder 2,2-Propylendithio-äthyl oder 3,3- oder 2,3-Aethylendithiopropyl, ferner Aethylendithio- oder Propylendithio-methyl.

Niederalkylidendithioniederalkyl ist beispielsweise 2,3-Propylidendithio-, 2,3-Aethylidendithio- oder 2,3-Isopropylidendithio-propyl.

Die Hydroxygruppe(n) in höherer als der α-Stellung tragendes Mono- oder Dihydroxyniederalkyl ist beispielsweise 2-Hydroxyäthyl, 3-Hydroxy- oder 2,3-Dihydroxy-propyl, 4-Hydroxy- oder 2,4-Dihydroxybutyl, 5-Hydroxy-, 2,5-Dihydroxy- oder 3,5-Dihydroxy-pentyl, ferner 3,4-Dihydroxybutyl oder 4,5-Dihydroxy-pentyl.

Die Hydroxygruppe(n) in höherer als der α-Stellung tragendes Hydroxy-niederalkoxyniederalkyl bedeutet beispielsweise 2-(2-Hydroxyäthoxy)-2-(3-Hydroxypropyloxy)- oder 2-(2,3-Dihydroxyäthoxy)-äthyl oder 3-(2-Hydroxyäthoxy)- oder 3-(3-Hydroxypropyloxy)-propyl, ferner 2-Hydroxyäthoxy- oder 3-Hydroxypropyloxymethyl.

Die Niederalkanoyloxygruppe in höherer als der α-Stellung tragendes Niederalkanoyloxyniederalkoxyniederalkyl ist beispielsweise 2-(2-Acetoxyäthoxy)- oder 2-(3-Acetoxypropyloxy)-äthyl oder 3-(2-Acetoxyäthoxy)-propyl, ferner 2-Acetoxyäthoxy- oder 3-Acetoxypropyloxy-methyl.

ω-Hydroxy-ω-sulfo-niederalkyl, -niederalkenyl bzw. -niederalkinyl bedeutet z.B. 2-Hydroxy-2-sulfo-äthyl, 3-Hydroxy-3-sulfo-propyl, -1-prop-1-enyl oder -prop-1-inyl oder 4-Hydroxy-4-sulfo-butyl, -but-2-enyl oder -but-2-inyl.

Niederalkoxyniederalkoxyniederalkyl bedeutet beispielsweise 2-(Methoxyäthoxy)-, 2-(Aethoxymethoxy)-, 2-(2-Methoxypropyloxy)- oder 2-(2-Aethoxyäthoxy)-äthyl oder 3-(2-Methoxyäthoxy)-propyl, ferner Methoxyäthoxy-, Aethoxymethoxy- oder Aethoxyäthoxy-methyl .

Die Hydroxygruppe(n) in höherer als der α-Stellung tragendes Hydroxyniederalkylthioniederalkyl bedeutet beispielsweise 2-(2-Hydroxyäthylthio)-, 2-(3-Hydroxypropylthio)- oder 2-(2,3-Dihydroxypropylthio)-äthyl oder 3-(2-Hydroxyäthylthio)- oder 3-(3-Hydroxypropylthio)-propyl, ferner 2-Hydroxyäthylthio- oder 3-Hydroxypropylthio-methyl.

Die Niederalkanoyloxygruppe in höherer als der α-Stellung tragendes Niederalkanoyloxyniederalkylthioniederalkyl bedeutet beispielsweise 2-(2-Acetoxyäthylthio)- oder 2-(3-Acetoxypropylthio)-äthyl oder 3-(2-Acetoxyäthylthio)-propyl, ferner 2-Acetoxyäthylthio- oder 3-Acetoxypropylthio-methyl.

Die Oxogruppe in höherer als der α-Stellung tragendes Oxoniederalkyl bedeutet beispielsweise 2-Oxoäthyl, 2- oder 3-Oxopropyl oder 2-, 3- oder 4-Oxobutyl, ferner entsprechendes Oxopentyl, Oxohexyl oder Oxoheptyl.

Ueber ein gesättigtes C-Atom gebundenes Niederalkenylen bedeutet ferner But-2-enylen, Pent-2-enylen, Pent-3-enylen oder Hex-3-enylen.

Die Hydroxygruppe(n) in höherer als der α- und in niederer als der ω-Stellung tragendes Mono- oder Dihydroxyniederalkylen bedeutet beispielsweise 1,3-(2-Hydroxy)-propylen, 1,4-(2- bzw. 3-Hydroxy)- oder 1,4-(2,3-Dihydroxy)-butylen oder 1,5-(3-Hydroxy)- oder 1,5-(2,4-Dihydroxy)-pentylen.

Niederalkoxyniederalkylen ist beispielsweise 1,3-(2-Methoxy)- oder 1,3-(2-Aethoxy)-propylen oder 1,4-(2- bzw. 3-Methoxy)-butylen, ferner 2-Methoxy- oder 2-Aethoxyäthylen.

Die Niederalkanoyloxygruppe(n) in höherer als der α- und in niederer als der ω-Stellung tragendes Mono- oder Diniederalkanoyloxyniederalkylen bedeutet beispielsweise 1,3-(2-Acetoxy)-propylen, 1,4-(2- bzw. 3-Acetoxy)-butylen oder 1,4-(2,3-Diacetoxy)-butylen, ferner 1,5-(3-Acetoxy)-pentylen.

Polyhalogenniederalkylen weist als Halogen insbesondere Fluor auf und bedeutet beispielsweise 1,1,2,2-Tetrafluoräthylen.

Niederalkylthioniederalkylen ist beispielsweise 1,3-(2-Methylthio)- oder 1,3-(2-Aethylthio)-propylen.

Die Oxogruppe in höherer als der α- und in niederer als der ω-Stellung tragendes Oxoniederalkylen bedeutet beispielsweise 1,3-(2-Oxo)-propylen-, 1,4-(2- bzw. 3-Oxo)-butylen oder 1,5-(3-Oxo)-pentylen.

Phenylniederalkyl ist beispielsweise Benzyl, 2-Phenyläthyl oder 3-Phenylpropyl.

Cycloalkyl mit 3 bis 8 Ringgliedern ist beispielsweise Cyclopentyl, Cyclohexyl oder Cycloheptyl, ferner Cyclopropyl oder Cyclooctyl.

Cycloalkylniederalkyl ist beispielsweise 1-Cycloalkyl-niederalkyl, wie Cyclopentyl-, Cyclohexyl- oder Cycloheptylmethyl.

Die Hydroxygruppe in höherer als der α-Stellung tragendes Hydroxycycloalkyl ist beispielsweise 2-Hydroxycycloalkyl, wie 2-Hydroxycyclopentyl, 2-Hydroxycyclohexyl oder 2-Hydroxycycloheptyl. Ebenso ist die Niederalkoxy- bzw. Niederalkanoyloxygruppe in höherer als der α-Stellung tragendes Niederalkoxy- bzw. Niederalkanoyloxycycloalkyl beispielsweise 2-Methoxy- oder 2-Aethoxy- bzw.

2-Acetoxycyclopentyl, 2-Methoxy- oder 2-Aethoxy- bzw. 2-Acetoxycyclohexyl oder 2-Methoxy- oder 2-Aethoxy- bzw. 2-Acetoxycycloheptyl.

Cycloalkyl-(hydroxy)-niederalkyl ist z.B. 2-Cyclohexyl-, 2-Cyclopentyl- oder 2-Cycloheptyl-2-hydroxy-äthyl.

Halogen ist beispielsweise Halogen der Atomnummer bis und mit 35, wie Fluor, Chlor oder Brom.

Salze von Verbindungen der Formel I sind vor allem pharmazeutisch verwendbare Säureadditionssalze mit starken Säuren, wie Mineralsäure, z.B. Salze mit Halogenwasserstoffsäuren, vor allem Chlor- oder Bromwasserstoffsäure, d.h. Hydrohalogenide, vor allem Hydrochloride und Hydrobromide, oder Schwefelsäuresalze, d.h. Hydrogensulfate und Sulfate, ferner Salze mit geeigneten organischen Säuren, wie Dicarbonsäuren oder organischen Sulfonsäuren, z.B. Maleinate, Fumarate, Maleate, Tartrate oder Methansulfonate, ferner N-Cyclohexylsulfaminate. Als weitere pharmazeutisch verwendbare Salze kommen Metallsalze, wie Alkalimetallsalze, von ω-Hydroxy-ω-sulfoniederalkyl, -niederalkenyl bzw. -niederalkinyl $R_4$ aufweisenden Verbindungen der Formel I, vor allem deren Kaliumsalze, in Frage.

Die Verbindungen der Formel I weisen wertvolle pharmakologische Eigenschaften auf. Insbesondere zeigen sie eine ausgeprägte antinociceptive Wirksamkeit sowie eine Hemmwirkung auf die Prostaglandinsynthese, ebenso eine ausgeprägte antiinflammatorische Wirkung. So erweisen sie sich an der Maus im durch Phenyl-p-benzochinon ausgelösten Writhing-Syndrom nach J. Pharmacol. exp. Therap. 125, 237 (1959) im Dosisbereich von etwa 4-40 mg/kg p.o. als ausgezeichnet wirksam.

Ebenso zeigen sie bei Dosen ab etwa 1 bis 10 mg/kg p.o. eine deutliche Hemmwirkung auf das experimentelle Carrageneen-Pfotenoedem der Ratte. Auch hemmen sie in vivo die Synthese von Leukotrien $B_4$.

Ferner zeigen sie in vitro im Konzentrationsbereich von etwa 0,1 bis 1,0 µM/L eine deutliche Hemmwirkung auf die Prostaglandinsynthese aus Arachidonsäure, nachgewiesen in der Versuchsanordnung nach Prostaglandin 7, 123 (1974).

Die Verbindungen der Formel I sind dementsprechend vorzüglich geeignet als Wirkstoffe von pharmazeutischen Präparaten zur Behandlung schmerzhaft-entzündlicher Erkrankungen, vor allem chronischer Erkrankungen des rheumatischen Formenkreises, wie der chronischen Arthritis.

Die Erfindung betrifft in erster Linie die Verwendung von Verbindungen der Formel I, worin $R_1$ und $R_2$ unabhängig voneinander unsubstituiertes oder durch Niederalkyl, Niederalkoxy bzw. an vicinalen C-Atomen Niederalkylidendioxy, Hydroxy, Halogen, Niederalkanoyloxy, unsubstituiertes oder durch Niederalkyl, Niederalkoxy und/oder Halogen substituiertes Benzoyl- oder Pyridoyloxy, Niederalkylthio bzw. an vicinalen C-Atomen Niederalkylidendithio, Niederalkansulfinyl, Niederalkansulfonyl, Amino, Mono- oder Diniederalkylamino, 3- bis 7-gliedriges Alkylen- bzw. 3-Aza-, 3-Oxa- oder 3-Thianiederalkylenamino, Nitro und/oder Trifluormethyl substituiertes Aryl mit 6 bis und mit 10 C-Atomen oder monocyclische, ein Sauerstoff- oder Schwefelatom und gegebenenfalls zusätzlich ein Stickstoffatom aufweisende 5-gliedrige bzw. gegebenenfalls N-oxidierte ein oder zwei Stickstoffatom(e) aufweisende 6-gliedriges Heteroaryl bedeuten, X Thio darstellt, n für 0, 1 oder 2 steht, $R_3$ Niederalkyl, vorzugsweise über ein gesättigtes C-Atom gebundenes Niederalkenyl oder Niederalkinyl, Mono- oder Diniederalkoxyniederalkyl, Niederalkylendioxyniederalkyl, Niederalkylidendioxyniederalkyl, die Niederalkanoyloxygruppe(n) in höherer als der α-Stellung tragendes Mono- oder Diniederalkanoyloxyniederalkyl, Polyhalogenniederalkyl, Mono- oder Diniederalkylthioniederalkyl, Niederalkansulfinyl- bzw. Niederalkansulfonylniederalkyl, Niederalkylendithioniederalkyl, die Hydroxygruppe(n) bzw. die Niederalk(anoyl)oxygruppe in höherer als der α-Stellung tragendes Mono- oder Dihydroxyniederalkyl, Hydroxyniederalkoxyniederalkyl oder Niederalkanoyloxyniederalkoxyniederalkyl, in Salzform vorliegendes ω-Hydroxy-ω-sulfoniederalkyl, ω-Hydroxy-ω-sulfoniederalkenyl und ω-Hydroxy-ω-sulfo-niederalkinyl, ebenso Niederalkoxyniederalkoxyniederalkyl, Niederalkylthioniederalkoxyniederalkyl, Hydroxyniederalkylthioniederalkyl, Niederalkoxyniederalkylthioniederalkyl oder Niederalkanoyloxyniederalkylthioniederalkyl, die Oxogruppe in höherer als der α-Stellung tragendes Oxoniederalkyl, unsubstituiertes oder wie für $R_1$ und $R_2$ angegeben substituiertes Phenylniederalkyl, 3- bis 8-gliedriges Cycloalkyl bzw. Cycloalkylniederalkyl oder die Hydroxy- bzw. Niederalk(anoyl)oxygruppe in höherer als der α-Stellung tragendes Hydroxycycloalkyl, Niederalkoxycycloalkyl, Niederalkanoyloxyniederalkyl, Cycloalkyl-(hydroxy)-niederalkyl bedeutet oder einen Rest der Formel Ia darstellt in der m für 0,1 oder 2 steht, $R_4$ Niederalkylen, über ein gesättigtes C-Atom gebundenes Niederalkenylen, Niederalkoxyniederalkylen, die Niederalkanoyloxygruppe in höherer als der α- und in niederer als der ω-Stellung tragendes Mono- oder Diniederalkanoyloxyniederalkylen, Polyhalogenniederalkylen, Niederalkylthioniederalkylen, oder die Hydroxy- bzw. Oxogruppe in höherer als der α- und in niederer als der ω-Stellung tragendes Mono- oder Dihydroxy- bzw. Oxoniederalkylen, Oxaniederalkylen oder gegebenenfalls S-oxidiertes Thianiederalkylen bedeutet oder, sofern n und m für 0 stehen, eine direkte Bindung darstellt und $R_1'$ und $R_2'$ eine der für $R_1$ bzw. $R_2$ angegebenen Bedeutungen haben,

mit der Massgabe, dass in Verbindungen, worin n für 1 steht, mindestens einer der Reste $R_1$ und $R_2$ von unsubstituierten oder durch Niederalkyl, Niederalkoxy, Halogen und/oder Trifluormethyl substituiertem Phenyl verschieden ist, wenn $R_3$ Niederalkyl bedeutet, und ihre pharmazeutisch verwendbaren Salze zur Anwendung in einem Verfahren zur therapeutischen Behandlung des menschlichen oder tierischen Körpers, ihre Verwendung zur Bekämpfung von Krankheiten, insbesondere als Wirkstoff in oder zur Herstellung von pharmazeutischen Präparaten, diese enthaltende pharmazeutischen Präparate und nicht-chemische Verfahren in ihrer Herstellung, und gleichermassen Verbindungen der Formel I, worin $R_1$, $R_2$, X, n, $R_3$, $R_4$, m, $R_1'$ und $R_2'$ obige Bedeutungen haben, selbst, mit der Massgabe, dass in Verbindungen, worin n für 0 oder 1 steht, mindestens einer der Reste $R_1$ und $R_2$ von unsubstituierten oder durch Niederalkyl, Niederalkoxy, Halogen und/oder Trifluormethyl substituiertem Phenyl verschieden ist, wenn $R_3$ Niederalkyl bedeutet, und mit der weiteren Massgabe, dass in Verbindungen, worin n für 0 steht, mindestens einer der Reste $R_1$ und $R_2$ unsubstituiertes oder wie angegeben substituiertes Heteroaryl gemäss der vorstehenden Definition bedeutet, wenn $R_3$ $\omega$-Hydroxy- oder $\omega$-Oxoniederalkyl mit mehr als 3 C-Atomen darstellt, bzw. mindestens einer der Reste $R_1$ und $R_2$ Heteroaryl gemäss der vorstehenden Definition bedeutet oder beide Reste $R_1$ und $R_2$ zugleich für p-Methoxyphenyl stehen, wenn $R_3$ $\omega$-Hydroxy- oder $\omega$-Oxoniederalkyl mit 2 oder 3 C-Atomen darstellen, und ihre Salze, insbesondere ihre pharmazeutisch verwendbaren Salze sowie Verfahren zu ihrer Herstellung.

Die Erfindung betrifft insbesondere die Verwendung von Verbindungen der Formel I, worin $R_1$ und $R_2$ unabhängig voneinander unsubstituiertes oder durch Niederalkyl, Niederalkoxy, Hydroxy, Halogen, Niederalkanoyloxy, Niederalkylthio, Niederalkansulfonyl, Diniederalkylamino, 3- bis 7-gliedriges Alkylen- bzw. 3-Aza-, 3-Oxa- oder 3-Thianiederalkylenamino und/oder Trifluormethyl substituiertes Phenyl oder unsubstituiertes oder durch Niederalkyl, Niederalkoxy oder Hydroxy

substituiertes Furyl, wie 2-Furyl, Thienyl, wie 2-Thienyl, Pyridyl, wie 2-, 3- oder 4-Pyridyl, bzw. 1-Oxidopyridyl, wie 2-, 3- oder 4-(1-Oxido)-pyridyl, bedeutet, X Thio darstellt, n für 0, 1 oder 2 steht, $R_3$ Niederalkyl, über ein gesättigtes C-Atom gebundenes Niederalkenyl oder Niederalkinyl, Mono- oder Diniederalkoxyniederalkyl, Niederalkylendioxyniederalkyl, Niederalkylidendioxyniederalkyl, die Niederalkanoyloxygruppe(n) in höherer als der α-Stellung tragendes Mono- oder Diniederalkanoyloxyniederalkyl, Mono- oder Diniederalkylthioniederalkyl, die Hydroxygruppe(n), die Niederalk(anoyl)-oxygruppe bzw. die Niederalkylendioxygruppe in höherer als der α-Stellung tragendes Mono- oder Dihydroxyniederalkyl, Hydroxyniederalkoxyniederalkyl oder Niederalkanoyloxyniederalkoxyniederalkyl, Niederalkoxyniederalkoxyniederalkyl, Niederalkylthioniederalkoxyniederalkyl, Hydroxyniederalkylthioniederalkyl, Niederalkoxyniederalkylthioniederalkyl oder Niederalkanoyloxyniederalkylthioniederalkyl, in Salzform vorliegendes ω-Hydroxy-ω-sulfoniederalkyl, ω-Hydroxy-ω-sulfo-niederalkenyl und ω-Hydroxy-ω-sulfo-niederalkinyl, ebenso/oder die Oxogruppe in höherer als der α-Stellung tragendes Oxoniederalkyl bedeutet oder einen Rest der Formel Ia darstellt in der m für 0, 1 oder 2 steht, $R_4$ Niederalkylen, die Hydroxy-, Niederalkanoyloxy- oder Oxogruppe in höherer als der α- und in niederer als der ω-Stellung tragendes Hydroxyniederalkylen, Niederalkanoyloxyniederalkylen oder Oxoniederalkylen bedeutet oder, sofern n und m für 0 stehen, eine direkte Bindung darstellt und $R_1'$ und $R_2'$ eine der für $R_1$ bzw. $R_2$ angegebenen Bedeutungen haben, mit der Massgabe, dass in Verbindungen, worin n für 1 steht, mindestens einer der Reste $R_1$ und $R_2$ von unsubstituiertem oder durch Niederalkyl, Niederalkoxy, Halogen und/oder Trifluormethyl substituiertem Phenyl verschieden ist,

wenn $R_3$ Niederalkyl bedeutet, und ihre pharmazeutisch verwendbaren Salze zur Anwendung in einem Verfahren zur therapeutischen Behandlung des menschlichen oder tierischen Körpers, ihre Verwendung zur Bekämpfung von Krankheiten, insbesondere als Wirkstoff in oder zur Herstellung von pharmazeutischen Präparaten, diese enthaltende pharmazeutische Präparate und nicht-chemische Verfahren in ihrer Herstellung, und gleichermassen Verbindungen der Formel I, worin $R_1$, $R_2$, X, n, $R_3$, $R_4$, X', m, $R_1'$ und $R_2'$ obige Bedeutungen haben, selbst, mit der Massgabe, dass in Verbindungen, worin n für 0 oder 1 steht, mindestens einer der Reste $R_1$ und $R_2$ von unsubstituiertem oder durch Niederalkyl, Niederalkoxy, Halogen und/oder Trifluormethyl substituiertem Phenyl verschieden ist, wenn $R_3$ Niederalkyl bedeutet, und mit der weiteren Massgabe, dass in Verbindungen, worin n für 0 steht, mindestens einer der Reste $R_1$ und $R_2$ unsubstituiertes oder wie angegeben substituiertes Furyl, Thienyl, Pyridyl oder 1-Oxidopyridyl bedeutet, wenn $R_3$ $\omega$-Hydroxy- oder $\omega$-Oxoniederalkyl mit mehr als 3 C-Atomen darstellt, bzw. mindestens einer der Reste $R_1$ und $R_2$ unsubstituiertes oder wie angegeben substituiertes Furyl, Thienyl, Pyridyl oder 1-Oxidopyridyl bedeutet, oder beide Reste $R_1$ und $R_2$ zugleich p-Methoxyphenyl darstellt, wenn $R_3$ $\omega$-Hydroxy- oder $\omega$-Oxoniederalkyl mit 2 oder 3 C-Atomen bedeutet, und ihre Salze, insbesondere ihre pharmazeutisch verwendbaren Salze, sowie Verfahren zu ihrer Herstellung.

Die Erfindung betrifft speziell die Verwendung von Verbindungen der Formel I, worin $R_1$ und $R_2$ unabhängig voneinander unsubstituiertes oder durch Niederalkoxy, Halogen, Niederalkylthio, Niederalkansulfinyl, Diniederalkylamino und/oder Trifluormethyl substituiertes Phenyl oder unsubstituiertes Thienyl, wie 2-Thienyl, oder unsubstituiertes oder durch Hydroxy substituiertes, gegebenenfalls N-oxidiertes Pyridyl, wie 3- oder 4-Pyridyl bzw. 3- oder 4- bzw. (1-Oxido)-pyridyl, bedeuten, X Thio darstellt, n für 0, 1 oder 2 steht, $R_3$ Niederalkyl, über ein gesättigtes C-Atom gebundenes Niederalkenyl oder Niederalkinyl,

Niederalkoxyniederalkyl, die Niederalkanoyloxygruppe in höherer als der α-Stellung tragendes Niederalkanoyloxyniederalkyl, Niederalkylthioniederalkyl, die Hydroxygruppe bzw. die Niederalk(anoyl)oxygruppe in höherer als der α-Stellung tragendes Hydroxyniederalkyl, Hydroxyniederalkoxyniederalkyl oder Niederalkanoyloxyniederalkoxyniederalkyl, in Salzform vorliegendes ω-Hydroxy-ω-sulfoniederalkyl, Niederalkoxyniederalkoxyniederalkyl, Niederalkylthioniederalkoxyniederalkyl, oder die Oxogruppe in höherer als der α-Stellung tragendes Oxoniederalkyl bedeutet, mit der Massgabe, dass in Verbindungen, worin n für 1 steht, mindestens einer der Reste $R_1$ und $R_2$ von unsubstituiertem oder durch Niederalkyl, Niederalkoxy, Halogen und/oder Trifluormethyl substituiertem Phenyl verschieden ist, wenn $R_3$ Niederalkyl bedeutet, und ihre pharmazeutisch verwendbaren Salze zur Anwendung in einem Verfahren zur therapeutischen Behandlung des menschlichen oder tierischen Körpers, ihre Verwendung zur Bekämpfung von Krankheiten, insbesondere als Wirkstoff in oder zur Herstellung von pharmazeutischen Präparaten, diese enthaltende pharmazeutische Präparate und nicht-chemische Verfahren in ihrer Herstellung,und gleichermassen Verbindungen der Formel I, worin $R_1$, $R_2$, X, n, $R_3$, $R_4$, m, X', $R_1'$ und $R_2'$ obige Bedeutungen haben, selbst, mit der Massgabe, dass in Verbindungen, worin n für 0 oder 1 steht, mindestens einer der Reste $R_1$ und $R_2$ von unsubstituierten oder durch Niederalkyl, Niederalkoxy, Halogen und/oder Trifluormethyl substituiertem Phenyl verschieden ist, wenn $R_3$ Niederalkyl bedeutet, und mit der weiteren Massgabe, dass in Verbindungen, worin n für 0 steht, $R_1$ und $R_2$ und/oder Thienyl oder Pyridyl ist, wenn $R_3$ ω-Hydroxy- oder ω-Oxoniederalkyl mit mehr als 3 C-Atomen ist bzw. $R_1$ und/oder $R_2$ Thienyl oder Pyridyl oder $R_1$ sowie $R_2$ p-Methoxyphenyl ist, wenn $R_3$ ω-Hydroxy- oder ω-Oxoniederalkyl mit 2 oder 3 C-Atomen bedeutet, und ihre Salze sowie Verfahren zu ihrer Herstellung.

Die Erfindung betrifft vor allem einerseits Verbindungen der Formel I, worin mindestens einer der Reste $R_1$ und $R_2$ gegebenenfalls N-oxidiertes Pyridyl, wie 3- oder 4-Pyridyl bzw. 3- oder 4-(1-Oxido)-pyridyl oder Thienyl, wie 2-Thienyl, und der andere unsubstituiertes oder durch Niederalkoxy mit bis und mit 4 C-Atomen, wie Methoxy, Niederalkylthio mit bis und mit 4 C-Atomen, wie Methylthio, Halogen der Atomnummer bis und mit 35, wie Fluor oder Chlor, Hydroxy und/oder Trifluormethyl substituiertes Phenyl bedeutet, X Thio darstellt, n für 0, 1 oder 2 steht, und worin $R_3$ entweder Niederalkyl bis und mit 4 C-Atomen, wie Aethyl, Polyhalogenniederalkyl, worin Halogen die Atomnummer bis und mit 35 und Niederalkyl bis und mit 4 C-Atome aufweist, wie Trifluormethyl oder 1,1,2,2-Tetrafluoräthyl, die Hydroxy-, Niederalkanoyloxy-, Niederalkoxy-, Niederalkylthio-, Niederalkansulfinyl- bzw. Niederalkansulfonylgruppe in höhere als der α-Stellung tragendes Hydroxyniederalkyl mit 2 bis und mit 7 C-Atomen, wie 2-Hydroxyäthyl, Niederalkanoyloxyniederalkyl mit jeweils bis und mit 4 C-Atomen, wie 2-Acetoxyäthyl, Niederalkoxyniederalkyl mit jeweils bis und mit 4 C-Atomen, wie 2-Methoxyäthyl, oder Niederalkylthio-, Niederalkansulfinyl- oder Niederalkansulfonyl mit jeweils bis und mit 4 C-Atomen, wie 2-Methylthio-, 2-Methansulfinyl- oder 2-Methansulfonyläthyl, oder die Hydroxygruppe sowie die Hydroxyniederalkoxy- bzw. Hydroxyniederalkylthiogruppe in höherer als der α-Stellung tragendes Hydroxyniederalkoxyniederalkyl, wie 2-(2-Hydroxyäthoxy)-äthyl, bzw. Hydroxyniederalkylthioniederalkyl, wie 2-(2-Hydroxyäthylthio)-äthyl, bedeutet, oder $R_3$ Niederalkyl bis und mit 4 C-Atomen, wie Aethyl, die Oxo-, Hydroxy-, Niederalkanoyloxy-, Niederalkoxy- oder Niederalkylendioxygruppe(n) in höherer als der α-Stellung tragendes Oxo- oder Hydroxyniederalkyl mit jeweils bis und mit 4 C-Atomen, wie 2-Hydroxyäthyl, 3-Hydroxypropyl, 2-Oxoäthyl oder 3-Oxopropyl, Niederalkanoyloxyniederalkyl mit jeweils bis und mit 4 C-Atomen, wie 2-Acetoxyäthyl, Mono- oder Diniederalkoxyniederalkyl mit jeweils bis und mit 4 C-Atomen, wie 2,2-Dimethoxyäthyl oder 3,3-Dimethoxypropyl, oder Niederalkylendioxyniederalkyl mit jeweils bis und mit 4 C-Atomen, wie 2,2-Aethylendioxyäthyl, oder in Salzform vorliegendes ω-Hydroxy-ω-sulfo-niederalkyl mit 2 bis 4 C-Atomen, wie 2-Hydroxy-2-sulfo-äthyl, bedeutet und ihre Salze, insbesondere pharmazeutisch verwendbare Salze, Verfahren zu ihrer Herstellung, diese bzw. ihre pharmazeutisch verwendbaren Salze enthaltene pharmazeutische Präparate und ihre Verwendung.

Die Erfindung betrifft andererseits vor allem Verbindungen der Formel I, worin $R_1$ und $R_2$ unabhängig voneinander unsubstituiertes oder durch Niederalkoxy mit bis und mit 4 C-Atomen, wie Methoxy, Niederalkylthio mit bis und mit 4 C-Atomen, wie Methylthio, Halogen der Atomnummer bis und mit 35, wie Fluor oder Chlor, Hydroxy und/oder Trifluormethyl substituiertes Phenyl bedeuten, X Thio darstellt, n für 0 oder 2 steht, und worin $R_3$ entweder Niederalkyl bis und mit 4 C-Atomen, wie Aethyl, Polyhalogenniederalkyl, worin Halogen die Atomnummer bis und mit 35 und Niederalkyl bis und mit 4 C-Atome aufweist, wie Trifluormethyl oder 1,1,2,2- Tetrafluoräthyl,die Hydroxy-, Niederalkanoyloxy-, Niederalkoxy-, Niederalkylthio-, Niederalkansulfinyl- bzw. Niederalkansulfonylgruppe in höherer als der α-Stellung tragendes Hydroxyniederalkyl mit 2 bis und mit 7 C-Atomen, wie 2-Hydroxyäthyl, Niederalkanoylniederalkyl mit jeweils bis und mit 4 C-Atomen, wie 2-Acetoxyäthyl, Niederalkoxyniederalkyl mit jeweils bis und mit 4 C-Atomen, wie 2-Methoxyäthyl, oder Niederalkylthio-, Niederalkansulfinyl- oder Niederalkansulfonyl mit jeweils bis und mit 4 C-Atomen, wie 2-Methylthio-, 2-Methansulfinyl- oder 2-Methansulfonyläthyl, oder die Hydroxygruppe sowie die Hydroxyniederalkoxy- bzw. Hydroxyniederalkylthiogruppe in höherer als der α-Stellung tragendes Hydroxyniederalkoxyniederalkyl, wie 2-(2-Hydroxyäthoxy)-äthyl, bzw. Hydroxyniederalkylthioniederalkyl, wie 2-(2-Hydroxyäthylthio)-äthyl, bedeutet oder $R_3$ Niederalkyl bis und mit 4 C-Atomen, wie Aethyl, die Oxo-, Hydroxy-, Niederalkanoyloxy-, Niederalkyloxy- oder Niederalkylendioxygruppe(n) in höherer als der α-Stellung

tragendes Hydroxyniederalkyl mit 2 bis und mit 4 C-Atomen, wie 2-Hydroxyäthyl oder 3-Hydroxypropyl, Niederalkanoyloxyniederalkyl mit jeweils bis und mit 4 C-Atomen, wie 2-Acetoxyäthyl, Oxoniederalkyl mit bis und mit 4 C-Atomen, wie 2-Oxoäthyl oder 3-Oxopropyl, oder Mono- oder Diniederalkoxyniederalkyl bzw. Niederalkylendioxyniederalkyl mit jeweils bis und mit 4 C-Atomen in jedem Alkyl(en)teil, wie 2-Methoxyäthyl, 2,2-Dimethoxyäthyl, 3,3-Dimethoxypropyl oder 2,2-Aethylendioxyäthyl, oder in Salzform vorliegendes ω-Hydroxy-ω-sulfo-niederalkyl mit 2 bis 4 C-Atomen, wie 2-Hydroxy-2-sulfo-äthyl, bedeutet, und ihre pharmazeutisch verwendbaren Salze zur Anwendung in einem Verfahren zur Behandlung des menschlichen oder tierischen Körpers, insbesondere zur Verwendung als Wirkstoff in oder zur Herstellung von pharmazeutischen Präparaten, und solche Präparate, ebenso Verbindungen der Formel I, worin $R_1$, $R_2$ und X obige Bedeutungen haben, n für 0, 1 oder 2 steht, und worin $R_3$ entweder Polyhalogenniederalkyl, worin Halogen die Atomnummer bis und mit 35 und Niederalkyl bis und mit 4 C-Atomen aufweist, wie Trifluormethyl oder 1,1,2,2-Tetrafluoräthyl, die Niederalkanoyloxy-, Niederalkoxy-, Niederalkylthio-, Niederalkansulfinyl- bzw. Niederalkansulfonylgruppe in höhere als der α-Stellung tragendes Niederalkanoyloxyniederalkyl mit jeweils bis und mit 4-C-Atomen, wie 2-Acetoxyäthyl, Niederalkoxyniederalkyl mit jeweils bis und mit 4 C-Atomen, wie 2-Methoxyäthyl, oder Niederalkylthio-, Niederalkansulfinyl- oder Niederalkansulfononyl mit jeweils bis und mit 4 C-Atomen, wie 2-Methylthio-, 2-Methansulfinyl- oder 2-Methansulfonyläthyl, oder die Hydroxygruppe sowie die Hydroxyniederalkyl- bzw. Hydroxyniederalkylthiogruppe in höherer als der α-Stellung tragendes Hydroxyniederalkoxyniederalkyl, wie 2-(Hydroxyäthoxy)-äthyl, bzw. Hydroxyniederalkylthioniederalkyl, wie 2-(2-Hydroxyäthylthio)-äthyl, oder die Hydroxygruppe in höherer als der α- und in niederer als der ω-Stellung tragendes Hydroxyniederalkyl mit 2 bis und mit 4 C-Atomen, wie 2-Hydroxypropyl, bedeutet oder, sofern n für 2 steht, Niederalkyl mit 2 bis und mit 7 C-Atomen, wie Aethyl, oder, sofern n für 1 oder 2 steht, ω-Hydroxyniederalkyl mit 2 bis und mit 7 C-Atomen, wie 2-Hydroxyäthyl, darstellt oder $R_3$ die Niederalkanoyloxy-,

Niederalkoxy- bzw. Niederalkylendioxygruppe(n) in höhere als der α-Stellung tragendes Niederalkanoyloxyniederalkyl mit jeweils bis und mit 4 C-Atomen, wie 2-Acetoxyäthyl, Mono- oder Di-niederalkoxyniederalkyl bzw. Niederalkylendioxyniederalkyl mit jeweils bis und mit 4 C-Atomen, wie 2-Methoxyäthyl, 2,2-Dimethoxyäthyl, 3,3-Dimethoxypropyl oder 2,2-Aethylendioxyäthyl, oder in Salzform vorliegendes ω-Hydroxy-ω-sulfo-niederalkyl mit 2 bis 4 C-Atomen, wie 2-Hydroxy-2-sulfo-äthyl bedeutet, oder, sofern n für 2 steht, Niederalkyl mit 2 bis und mit 7 C-Atomen, wie Aethyl, oder, sofern n für 1 oder 2 steht, ω-Hydroxyniederalkyl oder ω-Oxoniederalkyl mit 2 bis und mit 4 C-Atomen, wie 2-Hydroxyäthyl oder 2-Oxoäthyl, darstellt, oder, sofern n für 0 steht und beide Reste $R_1$ und $R_2$ p-Methoxyphenyl darstellen, ω-Hydroxy- oder ω-Oxoniederalkyl mit 2 bis 3 C-Atomen, wie 2-Hydroxyäthyl, 3-Hydroxypropyl, 2-Oxoäthyl oder 3-Oxopropyl, bedeuten und ihre Salze, insbesondere pharmazeutisch verwendbaren Salze und Verfahren zu ihrer Herstellung.

Die Erfindung betrifft in allererster Linie einerseits Verbindungen der Formel I, worin mindestens einer der Reste $R_1$ und $R_2$ Pyridyl, z.B. 3- oder 4-Pyridyl, oder Thienyl, z.B. 2-Thienyl, und der andere unsubstituiertes oder, vorzugsweise in p-Stellung, durch Niederalkoxy mit bis und mi 4 C-Atomen, wie Methoxy, oder Halogen der Atomnummer bis und mit 35, wie Fluor oder Chlor, substituiertes Phenyl bedeutet, X Thio oder Oxy darstellt, n für 0, 1 oder 2 steht, und worin $R_3$ entweder Niederalkyl mit bis und mit 4 C-Atomen, wie Aethyl, ω-Hydroxyniederalkyl mit 2 bis 4 C-Atomen, wie 2-Hydroxyäthyl, 2- oder 3-Niederalkoxyniederalkyl bzw. 2- oder 3-Niederalkylthioniederalkyl mit bis und mit 4 C-Atomen, wie 2-Methoxyäthyl bzw. 2-Methylthioäthyl, oder 2- oder 3-(ω-Hydroxyniederalkoxy)- bzw. 2- oder 3-(ω-Hydroxyniederalkylthio)-niederalkyl mit bis und mit 4 C-Atomen, wie 2-(2-Hydroxyäthoxy)-äthyl bzw. 2-(2-Hydroxyäthylthio)-äthyl,bedeutet, oder $R_3$ Niederalkyl mit bis und mit 4 C-Atomen, wie Aethyl, in Salzform vorliegendes ω-Hydroxy-ω-sulfo-niederalkyl mit 2 bis 4 C-Atomen, wie 2-Hydroxy-2-sulfo-äthyl, ω-Hydroxyniederalkyl mit 2 bis 4 C-Atomen, wie 2-Hydroxyäthyl, ω,ω-Di-

niederalkoxyniederalkyl mit bis und mit 4 C-Atomen, wie 2,2-Dimethoxyäthyl oder 3,3-Dimethoxypropyl, ω-Niederalkanoyloxyniederalkyl mit je bis und mit 4 C-Atomen, wie 2-Acetoxyäthyl, oder ω-Oxoniederalkyl mit bis und mit 4 C-Atomen, wie 2-Oxoäthyl oder 3-Oxopropyl, bedeutet, und ihre Salze, insbesondere ihre pharmazeutisch verwendbaren Salze, Verfahren zu ihrer Herstellung, diese bzw. ihre pharmazeutisch verwendbaren Salze enthaltenden pharmazeutischen Präparate und ihre Verwendung.

Die Erfindung betrifft in allererster Linie andererseits Verbindungen der Formel I, worin $R_1$ und $R_2$ unabhängig voneinander unsubstituiertes oder insbesondere in p-Stellung, durch Niederalkoxy mit bis und mit 4 C-Atomen, wie Methoxy, oder Halogen der Atomnummer bis und mit 35, wie Fluor oder Chlor, substituiertes Phenyl bedeuten, X Thio darstellt, n für 0, 1 oder 2 steht, und worin $R_3$ entweder ω-Hydroxyniederalkyl mit 2 bis 4 C-Atomen, wie 2-Hydroxyäthyl, 2- oder 3-Niederalkoxyniederalkyl bzw. 2- oder 3-Niederalkylthioniederalkyl mit bis und mit 4 C-Atomen, wie 2-Methoxyäthyl bzw. 2-Methylthioäthyl, oder 2- oder 3-(ω-Hydroxyniederalkoxy)- bzw. 2- oder 3-(ω-Hydroxyniederalkylthio)-niederalkyl mit bis und mit 4 C-Atomen, wie 2-(2-Hydroxyäthoxy)-äthyl bzw. 2-(2-Hydroxyäthylthio)-äthyl, bedeutet oder, sofern n für 0 oder 2 steht, Niederalkyl mit bis und mit 4 C-Atomen, wie Aethyl, darstellt, oder ω,ω-Diniederalkoxyniederalkyl mit jeweils bis und mit 4 C-Atomen in jedem Alkylteil, wie 2,2-Dimethoxyäthyl oder 2,2-Dimethoxypropyl oder ω-Niederalkanoyloxyniederalkyl mit je bis und mit 4 C-Atomen, wie 2-Acetoxyäthyl bedeutet oder, sofern n für 0 oder 2 steht, Niederalkyl mit bis und mit 4 C-Atomen, wie Aethyl, darstellt, und ihre pharmazeutisch verwendbaren Salze zur Anwendung in einem Verfahren zur therapeutischen Behandlung des menschlichen oder tierischen Körpers, insbesondere zur Verwendung als Wirkstoff in bzw. zur Herstellung von pharmazeutischen Präparaten, und solche pharmazeutischen Präparate, ebenso Verbindungen der Formel I, worin $R_1$, $R_2$, X und n obige Bedeutungen haben, und worin $R_3$ entweder 2- oder 3-Niederalkoxyniederalkyl bzw. 2- oder 3-Niederalkylthioniederalkyl mit bis und mit 4 C-Atomen, wie 2-Methoxyäthyl bzw. 2-Methylthioäthyl, oder 2- oder 3-(ω-Hydroxyniederalkoxy)-

bzw. 2- oder 3-(ω-Hydroxyniederalkylthio)-niederalkyl mit bis und mit 4 C-Atomen, wie 2-(2-Hydroxyäthoxy)-äthyl bzw. 2-(2-Hydroxyäthylthio)-äthyl, bedeutet oder, sofern n für 2 steht, Niederalkyl mit bis und mit 4 C-Atomen, wie Aethyl, bedeutet oder, sofern n für 1 oder 2 steht, ω-Hydroxyniederalkyl mit 2 bis und mit 4 C-Atomen, wie 2-Hydroxyäthyl, darstellt, oder $R_3$ in Salzform vorliegendes ω-Hydroxy-ω-sulfo-niederalkyl mit 2 bis 4 C-Atomen, wie 2-Hydroxy-2-sulfo-äthyl, ω-Diniederalkoxyniederalkyl bzw. ω-Niederalkanoyloxyniederalkyl mit je bis und mit 4 C-Atomen, wie 2,2-Dimethoxyäthyl bzw. 2-Acetoxyäthyl, oder ω-Niederalkylendioxyniederalkyl mit je bis und mit 4 C-Atomen, wie 2,2-Aethylendioxyäthyl, bedeutet oder, sofern n für 2 steht, Niederalkyl mit bis und mit 4 C-Atomen, wie Aethyl, bedeutet oder, sofern n für 1 oder 2 steht, ω-Hydroxyniederalkyl bzw. ω-Oxoniederalkyl mit 2 bis und mit 4 C-Atomen, wie 2-Hydroxyäthyl oder 2-Oxoäthyl, darstellt, oder, sofern n für 0 steht, und $R_1$ und $R_2$ zugleich p-Methoxyphenyl bedeuten, ω-Hydroxy- oder ω-Oxoniederalkyl mit 2 oder 3 C-Atomen, wie 2-Hydroxyäthyl oder 2-Oxoäthyl bedeuten, und ihre Salze, insbesondere pharmazeutisch verwendbaren Salze, und Verfahren zu ihrer Herstellung.

Die Erfindung betrifft namentlich die in den Beispielen genannten neuen Verbindungen der Formel I und ihre pharmazeutisch verwendbaren Salze, Verfahren zu ihrer Herstellung, diese enthaltende pharmazeutische Präparate und ihre Verwendung.

Die Verbindungen der Formel I können nach an sich bekannten Methoden hergestellt werden, beispielsweise indem man aus einer Verbindung der Formel

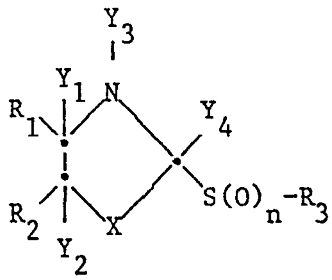

(II),

worin einer der Reste $Y_1$ und $Y_2$ einen abspaltbaren Rest Y und der andere ein Wasserstoffatom bedeutet und $Y_3$ und $Y_4$ gemeinsam eine zusätzliche Bindung darstellen, oder worin $Y_4$ einen abspaltbaren Rest Y und $Y_3$ Wasserstoff bedeutet und $Y_1$ und $Y_2$ eine zusätzliche Bindung darstellen, oder aus einem Salz davon unter Einführung einer zusätzlichen Bindung HY abspaltet, erforderlichenfalls ein verfahrensgemäss erhältliches Isomerengemisch in die reinen Isomeren auftrennt und gewünschtenfalls eine verfahrensgemäss erhältliche Verbindung in eine andere Verbindung der Formel I überführt und/oder eine verfahrensgemäss erhältliche freie Verbindung in ein Salz oder ein verfahrensgemäss erhältliches Salz in die freie Verbindung umwandelt.

Abspaltbare Reste Y sind beispielsweise gegebenenfalls veresterte oder verätherte Hydroxy- oder Mercaptogruppen, ferner Amino-, Ammonio- und Sulfoniumgruppen. Als verestertes Hydroxy kommt beispielsweise mit einer anorganischen Säure oder mit einer organischen Carbonsäure verestertes Hydroxy in Betracht, wie Halogen, z.B. Chlor oder Brom, oder Niederalkanoyloxy, z.B. Acetoxy. Verätherte Hydroxygruppen sind beispielsweise Niederalkoxygruppen, z.B. Methoxy oder Aethoxy. Veresterte Mercaptogruppen sind beispielsweise mit einer Niederalkancarbonsäure veresterte Mercaptogruppen, wie Acetylthio. Verätherte Mercaptogruppen bzw. Sulfoniumgruppen sind beispielsweise Niederalkylthio- bzw. Diniederalkylsulfoniumgruppen, wie Methylthio, Aethylthio oder Dimethylsulfonium. Aminogruppen sind beispielsweise Diniederalkyl- oder Alkylen- bzw. Aza-, Oxa- oder Thia-niederalkylenaminogruppen, z.B. Dimethylamino, Diäthylamino, Pyrrolidino, Piperidino, Morpholino oder Thiomorpholino, ferner Anilino. Ammoniumgruppen sind beispielsweise den vorstehend genannten Aminogruppe entsprechende tertiäre Ammoniumgruppen oder quaternäre Ammoniumgruppen, wie Triniederalkylammonio oder Pyridinio.

Die Abspaltung von HY erfolgt in üblicher Weise spontan oder durch gelindes Erwärmen, z.B. auf etwa 40 bis 100°C, erforderlichenfalls in Gegenwart eines Kondensationsmittels und/oder eines inerten Lösungsmittels. Als Kondensationsmittel kommen ausgehend von Verbindungen der Formel II, worin Y gegebenenfalls veräthertes oder mit einer organischen Carbonsäure verestertes Hydroxy bzw. Mercapto, Sulfonium oder eine Amino- bzw. Ammoniumgruppe bedeutet, vorzugsweise saure Kondensationsmittel in Betracht. Solche sind beispielsweise Mineralsäuren oder deren Anhydride bzw. sauren Salze, z.B. Halogenwasserstoffsäuren, vor allem Chlor-, Brom- oder Jodwasserstoffsäure, Schwefelsäure, Alkalimetallhydrogensulfate, Phosphorsäure, Polyphosphorsäure, Phosphorpentoxid bzw. zur Herstellung von Verbindungen der Formel I, worin X Thio ist, Phosphorpentasulfid, Phosphortrichlorid, Phosphoroxychlorid oder Phosphortribromid, organische Sulfonsäuren, wie p-Toluolsulfonsäure, oder Carbonsäuren bzw. deren Anhydride, wie Niederalkansäuren und deren Anhydride oder Halogenide, z.B. Essigsäure, Acetanhydrid oder Acetylchlorid, ferner Phosgen.

Ausgehend von Verbindungen der Formel II, worin Y mit einer Halogenwasserstoffsäure verestertes Hydroxy ist, werden vorzugsweise basische Kondensationsmittel, wie Hydroxide, Carbonate oder Niederalkanolate von Alkali- oder Erdalkalimetallen, z.B. Natrium-, Kalium- oder Calciumhydroxid, Natrium- oder Kaliumcarbonat oder Natriummethanolat, verwendet, ferner organische Stickstoffbasen, wie Triniederalkylamine, z.B. Triäthylamin, oder aromatische Tertiärbasen, wie Pyridin.

Die Ausgangsstoffe der Formel II können nach an sich bekannten Methoden hergestellt werden.

So erhält man Verbindungen der Formel II, worin $Y_1$ freies, veräthertes oder raktionsfähiges verestertes Hydroxy, veräthertes Mercapto oder gegebenenfalls substituiertes Amino ist, $Y_2$ Wasserstoff ist, n für 0 steht und $Y_3$ und $Y_4$ eine zusätzliche Bindung bedeuten, beispielsweise, indem man eine Verbindung der Formel $R_1$-C(=O)-CH($R_2$)-XH (III) oder ein funktionelles Oxoderivat davon mit einer Verbindung der Formel N≡C-$SR_3$ (IV) umsetzt. Funktionelle Oxoderivate von Verbindungen der Formel III sind beispielsweise deren Ketale, wie Diniederalkylketale, Thioketale, wie Diniederalkylthioketale, Thioxoderivate, Iminderivate, wie Imine oder Anile, Enamine, wie N,N-Diniederalkyl- oder N,N-Alkylen- bzw. N,N-Oxa- oder N,N-Thia-alkylen-enamine, oder Enoläther, wie Enolniederalkyläther. Die Umsetzung, bei der intermediär eine Verbindung der Formel $R_1$-C(=O)-C($R_2$)-X-C(=NH)-$SR_3$ (V) bzw. ein funktionelles Oxoderivat davon gebildet wird, erfolgt vorzugsweise in einem inerten Lösungsmittel, erforderlichenfalls in Gegenwart eines Kondensationsmittels, unter Kühlen oder Erwärmen und/oder unter Inertgas. Als Kondensationsmittel kommen beispielsweise saure Kondensationsmittel, wie Mineralsäuren, z.B. Chlorwasserstoffsäure, Schwefelsäure, Phosphorsäure und dergleichen, und ihre sauren Salze, z.B. Natriumhydrogensulfat, Phosphorpentoxid, Polyphosphorsäure, Phosphoroxychlorid, Phosphortribromid oder Triniederalkylphosphite, ferner Carbonsäureanhydride wie Acetylchlorid oder Acetanhydrid, und organische Sulfonsäuren, z.B. p-Toluolsulfonsäure, in Betracht. Saure Kondensationsmittel gestatten, die gesamte Reaktionsfolge (III + IV ⟶ V ⟶ II ⟶ I) in einer "Eintopf-Umsetzung", ohne Isolierung von Zwischenprodukten z.B. der Formeln V und II, durchzuführen. Die Grundverbindungen der Formel III sind, soweit nicht bekannt, durch Kondensation von Verbindungen der Formeln $R_1$-C(=O)-Cl (VI) und $R_2$-H (VII) in Gegenwart von Aluminiumchlorid, Bromierung des Produktes der Formel $R_1$-C(=O)-$CH_2$-$R_2$ (VIII), z.B. mit Brom in Essigsäure, Umsetzung mit Natriumthiolacetat sowie Hydrolyse des

Produktes der Formel $R_1$-C(=O)-CH($R_2$)-X-C(=O)-$CH_3$ (IX) und gewünschtenfalls funktionelle Abwandlung der Oxogruppe zugänglich.

In analoger Weise kann man auch ein Aminoketon der Formel $R_1$-CH($NH_2$)-C(=O)-$R_2$ (X) oder ein funktionelles Oxoderivat, wie Ketal, Thioketal, geminales Dihalogenid oder Thioxoderivat davon mit einer Verbindung der Formel Hal-C(=X')-S-$R_3$ (XI), worin Hal Halogen und X' Thioxo bedeuten, oder zunächst mit einem reaktionsfähigen Kohlensäure- bzw. Thiokohlensäurederivat, wie einem Diester, Esterhalogenid oder Dihalogenid der Thiokohlensäure oder Trithiokohlensäure, z.B. mit einem Thionkohlensäureniederalkylester, einem Thionkohlensäureesterhalogenid oder Thiophosgen, und anschliessend mit einem Mercaptan der Formel $R_3$-$Y_6$ (XII, $Y_6$ = Mercapto) zu einer Verbindung der Formel $R_1$-CH[NH-C(=X')-S$R_3$]-C(=O)-$R_2$ (XIII) bzw. einem funktionellen Oxoderivat davon umsetzen, das spontan oder durch gelindes Erwärmen,erforderlichenfalls in Gegenwart eines Kondensationsmittels,zu einer Verbindung der Formel II cyclisiert,worin $Y_1$ Wasserstoff,$Y_2$ gegebenenfalls veräthertes Hydroxy bzw.Mercapto oder Halogen und $Y_3$ gemeinsam mit $Y_4$ eine zusätzliche Bindung bedeutet oder $Y_1$ gemeinsam mit $Y_2$ eine zusätzliche Bindung, $Y_3$ Wasserstoff und $Y_4$ Hydroxy oder Mercapto darstellt. Als Kondensationsmittel kommen dabei ausgehend von Verbindungen der Formel XIII selbst und ihrer funktionellen Thioxoderivate, ausgehend von geminalen Dihalogenderivaten von Verbindungen der Formel XIII, beispielsweise basische, und ausgehend von Verbindungen der Formel XIII abgeleiteten Ketalen bzw. Thioketalen sowohl saure als auch basische Kondensationsmittel in Betracht. Saure Kondensationsmittel sind beispielsweise die genannten, und als basische Kondensationsmittel kommen beispielsweise Metallbasen, wie Hydroxide, Carbonate oder Niederalkanolate von Alkalimetallen oder Erdalkalimetallen, z.B. Natrium-, Kalium- oder Calciumhydroxid, Natrium- oder Kaliumcarbonat oder Natriummethanolat, ebenso tertiäre oder quaternäre Stickstoffbasen, wie Triniederalkylamine, z.B. Triäthylamin, oder heteroaromatische Basen,

z.B. Pyridin. Man kann aber auch von Verbindungen der Formel XIII, worin X' Oxo ist, ausgehen und diese mit Phosphorpentasulfit behandeln. Auch die vorstehend genannten Reaktionen lassen sich, insbesondere in Gegenwart eines sauren Kondensationsmittels, in einer Eintopf-Umsetzung, ohne Isolierung von Zwischenstufen (z.B. der Formeln XIII und II) durchführen. Die als Ausgangsstoffe dienenden Aminoketone der Formel X können z.B. hergestellt werden, indem man eine Verbindung der Formel $R_1$-CH-C(=O)-$R_2$ (XIV) mit Hydroxylamin zum Oxim umsetzt, dieses mit p-Toluolsulfochlorid verestert und den erhaltenen Oximester mit einem Alkalimetallalkoholat, z.B. mit Kaliumtertiärbutanolat, behandelt.

Eine andere Verfahrensweise besteht darin, dass man eine Verbindung der Formel $R_1$-C(=O)-CH(Z)-$R_2$ (XVII) mit einer Verbindung der Formel X=C($NH_2$)-$SR_3$ XV, worin X' Thioxo ist, umsetzt, wobei in der Formel XVII Z reaktionsfähiges verestertes Hydroxy, wie Halogen oder Sulfonyloxy, z.B. Methan, Aethan-, Aethen-, Benzol-, p-Brombenzol- oder vor allem p-Toluolsulfonyloxy, ferner Fluorsulfonyloxy, bedeutet. Dabei wird ebenfalls unter primärer Bildung einer Zwischenstufe, z.B. der Formel V (X = Thio) oder XIII (X' = Oxo) und Cyclisierung zu einer Verbindung der Formel II erhalten, worin einer der Reste $Y_1$ und $Y_2$ Hydroxy und der andere Wasserstoff ist und $Y_3$ und $Y_4$ eine zusätzliche Bindung darstellen.

Die Umsetzung von Verbindungen einer Verbindung der Formel XVII mit einer Verbindung der Formel XV erfolgt vorzugsweise in Gegenwart eines basischen oder sauren Kondensationsmittels, wobei die Reaktion im ernstgenannten Fall auf der Stufe des Zwischenproduktes der Formel II aufgehalten und dieses isoliert werden kann. Basische bzw. saure Kondensationsmittel sind vorzugsweise die genannten. Statt der Verwendung eines sauren Kondensationsmittels kann man aber auch die Komponente der Formel XV in Form eines Säureadditionssalzes verwenden.

Die Verbindungen der Formel I können ferner hergestellt werden, indem man Verbindungen der Formel

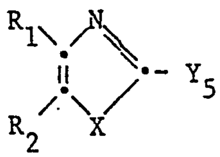 (XVI) und $Y_6-R_3$ (XII),

worin $Y_5$ eine gegebenenfalls in Salzform vorliegende Gruppe der Formel $-S(O)_n-H$ oder -S-SH und $Y_6$ reaktionsfähiges verestertes Hydroxy oder, sofern $R_3$ in α,β-Stellung mindestens eine zusätzliche C-C-Bindung aufweist, veräthertes Hydroxy, bedeutet oder $Y_5$ reaktionsfähiges verestertes Hydroxy und $Y_6$ eine gegebenenfalls in Salzform vorliegende Gruppe der Formel -SH- oder -S-SH darstellt, miteinander kondensiert, erforderlichenfalls ein verfahrensgemäss erhältliches Isomerengemisch in die reinen Isomeren auftrennt, und gewünschtenfalls eine verfahrensgemäss erhältliche Verbindung in eine andere Verbindung der Formel I überführt und/oder eine verfahrensgemäss erhältliche freie Verbindung in ein Salz oder ein verfahrensgemäss erhältliches Salz in die freie Verbindung umwandelt.

Reaktionsfähige veresterte Hydroxygruppen sind beispielsweise mit Mineralsäuren oder organischen Sulfonsäuren veresterte Hydroxygruppen, wie Halogen, z.B. Chlor, Brom oder Jod, Niederalkansulfonyl, z.B. Methan-, Aethan- oder Aethansulfonyl, oder gegebenenfalls substituiertes Benzolsulfonyl, z.B. Benzol-, p-Toluol- oder p-Brombenzolsulfonyl, ferner Fluorsulfonyl. Veräthertes Hydroxy $Y_6$ ist z.B. Niederalkoxy, wie Isopropyloxy oder vor allem tert.-Butyloxy.

Als Salzformen der Gruppe $-S(O)_n-H$ bzw. -S-SH kommen beispielsweise deren Metallsalzformen, wie Alkalimetall- oder Erdalkalimetallsalzformen, im Falle von Gruppen $-S(O)_n-H$, worin n 1 oder 2 ist, ferner deren Ammoniumsalzformen mit Ammoniak oder organischen Aminen in Betracht. Derartige Salze werden vorzugsweise durch Einwirkung der äquivalenten Menge der betreffenden Base auf die die freie Gruppe $-S(O)_n-H$

enthaltende Reaktionskomponente bzw. durch Umsetzung einer Verbindung der Formel XVI bzw. XII, worin $Y_5$ bzw. $Y_6$ Halogen ist, mit Natriumdisulfid in situ erzeugt und ohne Isolierung verwendet.

Die Kondensation erfolgt in üblicher Weise, vorzugsweise in einem inerten Lösungsmittel, erforderlichenfalls unter Kühlen oder Erwärmen, z.B. im Temperaturbereich von etwa 0 bis 100°C, in einem geschlossenen Gefäss und/oder unter Inertgas, wie Stickstoff. Als inerte Lösungsmittel kommen insbesondere polare Lösungsmittel in Betracht, wie Niederalkanole, Diniederalkylketone, N,N-Diniederalkylalkancarbonsäureamide bzw. N-Niederalkyl-lactame oder Diniederalkylsulfoxide, sofern $Y_6$ veräthertes Hydroxy ist, in Gegenwart von Dilithiumpalladiumtetrachlorid.
Die Ausgangsstoffe der Formel XVI können nach an sich bekannten Methoden hergestellt werden.

So stellt man Verbindungen der Formel XVI, worin $Y_5$ Halogen oder organisches Sulfonyloxy bedeutet, beispielsweise her, indem man eine Verbindung der Formel $R_1$-C(=O)-CH(Z)-$R_2$ (XVII), worin Z reaktionsfähiges verestertes Hydroxy, z.B. Halogen ist, mit einem anorganischen Isothiocyanat umsetzt, vorzugsweise in Aethanol, und das Kondensationsprodukt der Formel $R_1$-C(=O)-CH[X-(C≡N)]-$R_2$ (XIX) mit einer Halogenwasserstoffsäure oder organischen Sulfonsäure behandelt, vorzugsweise in Benzol oder Diäthyläther.

Verbindungen der Formel XVI, worin $Y_5$ eine Gruppe $-S(O)_n-H$ und n 0 bedeutet, können z.B. erhalten werden, indem man eine Verbindung der Formel XVII mit Ammoniumdithiocarbaminat umsetzt oder eine entsprechende Verbindung der Formel XVI, worin $Y_5$ Halogen ist, mit Thioharnstoff oder Natriumthiolacetat umsetzt und daraus solvolytisch oder reduktiv das gewünschte Mercaptan freisetzt.

Verbindungen der Formel XVI, worin $Y_5$ eine Gruppe $-S(O)_n-H$ und n 0 ist, werden ferner erhalten, indem man eine Verbindung der Formel $R_1-C(=O)-CH(R_2)-XH$ (III) mit Ammonium- oder einem Alkalimetallrhodanid umsetzt, vorzugsweise unter Erwärmen in äthanolischer Salzsäure.

Verbindungen der Formel XVI, worin $Y_5$ eine Gruppe $-S(O)_n-H$ und n 1 oder 2 bedeutet, können beispielsweise durch Oxidation der entsprechenden Verbindungen, worin n 0 ist, hergestellt werden. Als Oxidationsmittel kommt dabei zur Herstellung von Sulfinsäuren ($Y_5 = SO_2-H$) beispielsweise Wasserstoffperoxid in Gegenwart einer Säure und zur Herstellung von Sulfonsäuren ($Y_5 = SO_3-H$) beispielsweise Kaliumpermanganat, ebenso Stickstoffdioxid in Betracht.

Die Verbindungen der Formel I können ferner hergestellt werden, indem man Verbindungen der Formeln

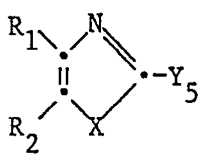 (XVI) und $Y_6-R_3$ , (XII),

worin einer der Reste $Y_5$ und $Y_6$ einen metallischen Rest und der andere eine Gruppe $-S(O)_n-Y_7$ bedeutet, in der $Y_7$ reaktionsfähiges verestertes Hydroxy darstellt oder, sofern n für 1 oder 2 steht, veräthertes Hydroxy oder, sofern n für 0 steht, veräthertes Mercapto bedeutet, und worin $R_3$ einen unsubstituierten oder durch verätherte Hydroxy- und/oder Mercaptogruppen substituierten, gegebenenfalls durch Oxa oder Thia unterbrochenen aliphatischen, einen unsubstituierten oder ein Arylteil gegebenenfalls wie für $R_1$ bzw. $R_2$ angegebenen substituierten aliphatischen oder einen unsubstituierten oder durch veräthertes Hydroxy substituierten cycloaliphatischen oder cycloaliphatisch-aliphatischen Kohlenwasserstoffrest bedeutet oder aufweist, miteinander kondensiert und gewünschtenfalls eine verfahrensgemäss erhältliche Verbindung in eine andere Verbindung der Formel I überführt und/oder eine verfahrensgemä-s erhältliche freie Verbindung in ein Salz oder ein verfahrensgemäss erhältliches Salz in die freie Verbindung umwandelt.

Metallische Reste sind beispielsweise Gruppen der Formeln $-M^{I}$ $-M^{II}/2$ oder $-M^{II}$-Hal, worin $M^{I}$ ein Metallatom der Gruppe 1A und $M^{II}$ ein Metallatom der Gruppen 2A und 2B des Periodischen Systems der Elemente bedeutet und Hal für ein Halogenatom, wie Chlor, Brom oder Jod steht.

Reaktionsfähiges verestertes Hydroxy ist beispielsweise Halogen, wie Chlor, Brom oder Jod. Veräthertes Hydroxy bzw. Mercapto ist beispielsweise Niederalkoxy bzw. Niederalkylthio, Niederalkylidendioxy bzw. Niederalkylidendithio oder Niederalkylendioxy bzw. Niederalkylendithio, kann aber auch gegebenenfalls durch Niederalkyl und/oder Niederalkoxy substituiertes Phenoxy bzw. Phenylthio sein. Als verätherte Mercaptogruppen kommen aber ebenso Gruppen $Y_5$ der Formel

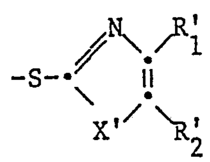

bzw. Gruppen $Y_5$ der Formel $-S-R_3$ in Betracht, worin $R_1'$ und $R_2'$ eine der für $R_1$ bzw. $R_2$ angegebenen Bedeutungen haben.

Die Umsetzung erfolgt in üblicher Weise, vorzugsweise in einem inerten Lösungsmittel, erforderlichenfalls unter Kühlen oder Erwärmen und/oder unter Inertgas, wie Stickstoff. Als inerte Lösungsmittel kommen beispielsweise Aether, wie Diniederalkyläther, z.B. Diäthyläther oder Tertiärbutoxymethan, bzw. Niederalkylenäther, z.B. Tetrahydrofuran, oder tertiäre Amide, z.B. Hexamethylphosphorsäuretriamid, ferner Kohlenwasserstoffe, wie Benzol, in Betracht. Die Reaktion wird beispielsweise im Temperaturbereich von -80 bis etwa + 60°C, vorzugsweise von etwa -25 bis etwa +40°C, durchgeführt. In einer bevorzugten Ausführungsform dieses Verfahrens geht man beispielsweise von einer Verbindung der Formel XVI aus, worin $Y_5$

eine Halogensulfenyl- oder Halogensulfonylgruppe ist und setzt diese bei etwa -10 bis + 10°C in Tetrahydrofuran oder Hexamethylphosphorsäuretriamid mit einer Verbindung der Formel XII um, worin $Y_6$ ein Alkalimetallatom bedeutet. In einer anderen bevorzugten Ausführungsform setzt man beispielsweise eine Verbindung der Formel XVI, worin $Y_5$ ein Alkalimetallatom bedeutet, mit einer Verbindung der Formel $R_3$-S-S-$R_3$ (XIIa) um.

Ausgangsstoffe der Formel XVI, worin $Y_5$ eine Gruppe $-S(O)_n-Y_7$ und $Y_7$ Halogen ist, erhält man beispielsweise, indem man eine Verbindung der Formel XVI, worin $Y_5$ eine Gruppe $-S(O)_n-H$ ist in üblicher Weise halogeniert, ausgehend von Mercaptanen (n=0) beispielsweise durch Umsetzung mit Chlor, z.B. in Tetrachlormethan, gegebenenfalls nach vorhergehender Oxidation zum Disulfid, und ausgehend von Sulfin- bzw. Sulfonsäuren (n = 1 bzw. 2) durch trockenes Erhitzen des Natriumsalzes mit Phosphorpentachlorid. Aus den so erhältlichen Sulfinyl- bzw. Sulfonylchloriden erhält man durch Umsetzung mit einem Alkohol oder Alkoholat, z.B. mit einem Niederalkanol bzw. Alkalimetallniederalkanolat, die entsprechenden Ester ($Y_5$ = veräthertes Hydroxy).

Verbindungen der Formel XVI, worin $Y_5$ einen metallischen Rest bedeutet, kann beispielsweise hergestellt werden, indem man eine Verbindung der Formel

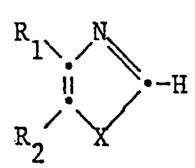 (XVIII),

die beispielsweise durch Umsetzung einer Verbindung der Formel $R_1$-C(=O)-CH(Z)-$R_2$ (XVII, Z = reaktionsfähiges verestertes Hydroxy, z.B. Halogen) mit Thioformamid zugänglich ist, mit einer metallorganischen Verbindung, wie einer Alkalimetall- oder

Erdalkalimetallkohlenwasserstoffverbindung, z.B. mit Butyllithium, Phenylnatrium oder Butylmagnesiumbromid, umsetzt. Man kann aber auch von einer Verbindung der Formel XVI, worin $Y_5$ Halogen ist, ausgehen, und diese mit einem Alkali- oder Erdalkalimetall, z.B. mit Magnesium oder Lithium, umsetzen.

Disulfide der Formel

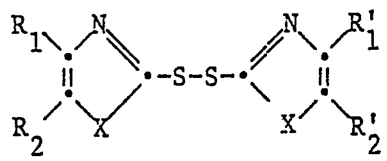

(XVIa)

bzw. der Formel $R_3-S-S-R_3$ (XIIa) erhält man beispielsweise durch Oxidation der entsprechenden Mercaptane der Formel XVI bzw. XII ($Y_5$ bzw. $Y_6$ = $-S(O)_n-H$, n = 0), z.B. mittels Luft oder durch Behandeln mit Jod.

Ein weiteres Verfahren zur Herstellung von Verbindungen der Formel I ist dadurch gekennzeichnet, dass man eine Verbindung der Formel

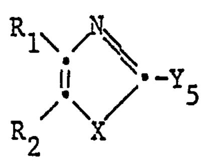

(XVI),

worin $Y_5$ Mercapto ist, oder ein Salz davon mit einem von einer Verbindung der Formel $R_3$-H (XIX) abgeleiteten vicinalen Epoxiderivat zu einer Verbindung der Formel I umsetzt, worin $R_3$ in β-Stellung eine Hydroxygruppe aufweist und n für 0 steht, erforderlichenfalls ein verfahrensgemäss erhältliches Isomerengemisch in die reinen Isomeren auftrennt und gewünschtenfalls eine verfahrensgemäss erhältliche Verbindung in eine andere Verbindung der Formel I überführt und/oder eine verfahrensgemäss erhältliche freie Verbindung in ein Salz oder ein verfahrensgemäss erhältliches Salz in die freie Verbindung umwandelt.

Als Salze von Verbindungen der Formel XVI kommen beispielsweise deren Metallsalze, vorzugsweise Alkalimetall- oder Erdalkalimetallsalze in Betracht.

Von Verbindungen der Formel XIX abgeleitete vicinale Epoxide sind beispielsweise unsubstituierte oder wie für $R_3$ angegeben substituierte vicinale aliphatische, araliphatische cycloaliphatische oder cycloaliphatisch-aliphatische Epoxide, wie 1,2-Epoxyniederalkane, 1,2-Epoxycycloalkane oder Cycloalkyl-1,2-epoxy-niederalkane.

Die Umsetzung erfolgt in üblicher Weise, beispielsweise in einem inerten Lösungsmittel, wie einem Niederalkanol, oder einem tertiären Amid, z.B. in Hexamethylphosphorsäuretriamid, erforderlichenfalls in Gegenwart eines Kondensationsmittel, beispielsweise ausgehend von freien Mercaptanen der Formel XVI eines zur Salzbildung befähigten basischen Mittels, wie eines Alkalimetallalkoholates oder -hydroxides, z.B. von Natriummethanolat oder Natriumhydroxid, unter Kühlen oder Erwärmen und/oder unter Inertgas, wie Stickstoff.

Ein weiteres Verfahren zur Herstellung von Verbindungen der Formel I ist dadurch gekennzeichnet, dass man eine Verbindung der Formel

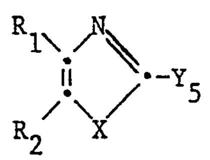

(XVI),

worin $Y_5$ Mercapto ist, oder ein Salz davon mit einer Verbindung der Formel $R_3'$-H (XX), worin $R_3'$ ein von $R_3$ abgeleiteter mindestens eine C-C-Doppel- oder C-C-Dreifachbindung aufweisenden Rest bedeutet, zu einer Verbindung der Formel I umsetzt, worin $R_3$ α,β-gesättigt oder α,β-einfach ungesättigt ist und n für 0 steht, erforderlichenfalls ein verfahrensgemäss erhältliches Isomerengemisch in die reinen

Isomeren auftrennt und gewünschtenfalls eine verfahrensgemäss erhältliche Verbindung in eine andere Verbindung der Formel I überführt und/oder eine verfahrensgemäss erhältliche freie Verbindung in ein Salz oder ein verfahrensgemäss erhältliches Salz in die freie Verbindung umwandelt.

Als Salze von Mercaptanen der Formel XVI kommen insbesondere deren Metallsalze, vorzugsweise Alkalimetall- oder Erdalkalimetallsalze in Betracht.

Mindestens eine C-C-Doppel- oder C-C-Dreifachbindung aufweisende Reste $R_3'$ sind beispielsweise unsubstituierte oder wie für $R_3$ angegeben substituierte mindestens eine C-C-Doppel oder C-C-Dreifachbindung aufweisende aliphatische, mindestens eine nicht aromatische C-C-Doppelbindung oder eine C-C-Dreifachbindung aufweisende araliphatische, mindestens eine C-C-Doppelbindung aufweisende cycloaliphatische oder im aliphatischen Teil mindestens eine C-C-Doppel- oder C-C-Dreifachbindung aufweisende cycloaliphatische Kohlenwasserstoffreste, ferner mindestens eine nicht-aromatische C-C-Doppelbindung oder eine C-C-Dreifachbindung aufweisende von Resten der Formel Ia abgeleitete Reste. Als Verbindungen der Formel XX kommen beispielsweise in Betracht: Niederalkene, Niederalkandiene, Niederalkine, Niederalkin-ene; die Niederalkoxy-, Niederalkylendioxy-, Niederalkylidendioxy-, Niederalkylthio-, Niederalkansulfinyl-, Niederalkansulfonyl-, Hydroxy-, Hydroxyniederalkoxy-, Niederalkanoyloxyniederalkoxy, Niederalkoxyniederalkoxy-, Niederalkylthioniederalkoxy-, Hydroxyniederalkylthio-, Niederalkoxyniederalkylthio-, Niederalkanoyloxyniederalkylthio- bzw. Oxogruppe(n) in höherer als der β-Stellung tragendes Mono- oder Diniederalkoxyniederalkene, Niederalkylendioxyniederalkene, Niederalkylidendioxyniederalkene, oder Diniederalkanoyloxyniederalkene, Polyhalogenniederalkene, Mono- oder Diniederalkylthioniederalkene, Niederalkansulfinyl- bzw. Niederalkansulfonylniederalkene, Niederalkylendithioniederalkene, Mono-oder Dihydroxyniederalkene, Hydroxyniederalkoxyniederalkene, Niederalkanoyloxyniederalkoxyniederalkene, Niederalkoxyniederalkoxyniederalkene, Niederalkylthioniederalkoxyniederalkene, Hydroxyniederalkylthioniederalkene, Niederalkoxyniederalkylthioniederalkene, Niederalkanoyloxyniederalkylthioniederalkyene und Oxoniederalkene, im Phenylteil wie für $R_3$ angegeben substituierte Phenylniederalkene, bzw. -alkine, Cycloalkene und Cycloalkylniederalkene bzw. -alkine, ferner als Rest $R_4'$ Niederalkenylen, Niederalkadienylen, 3-Niederalkoxyniederalk-1-enylen, 3-Niederalkanoyloxyniederalk-1enylen, Polyhalogenniederalkenylen, 3-Niederalkylthioniederalk-1-enylen, oder die Hydroxy- bzw.Oxogruppe in höherer als der β- und in niederer als der ω-Stellung tragendes Mono- oder Dihydroxy- bzw. Oxoniederalkenylen, ferner 4-Oxaniederalk-1-enylen oder gegebenenfalls S-oxidiertes 4-Thianiederalk-1-enylen aufweisende Verbindungen der Formel

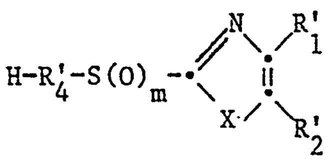

(XXa).

Die Umsetzung erfolgt in üblicher Weise, beispielsweise in einem inerten Lösungsmittel, wie einem tertiären Amid, z.B. in Dimethylformamid oder N-Methylpyrrolidon, erforderlichenfalls in Gegenwart eines Kondensationsmittels, unter Kühlen oder Erwärmen und/oder unter Inertgas, wie Stickstoff. Als Kondensationsmittel kommen ausgehend von Mercaptanen der Formel XVI beispielsweise organische Stickstoffbasen, wie Di- oder Triniederalkylamine, z.B. Diisopropylamin oder Triäthylamin, bzw. Alkylen- bzw. Aza-, Oxa- oder Thiaalkylenamine, z.B. Pyrrolidin, Piperidin, Morpholin, Thiomorpholin oder Piperazin, in Betracht.

Verbindungen der Formel I, worin $R_3$ eine Gruppe der Formel Ia darstellt, n und m für 0 stehen und $R_4$ eine direkte Bindung darstellt, können ferner hergestellt werden, indem man Verbindungen der Formeln

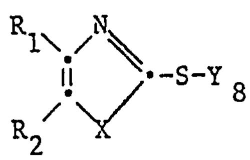 und 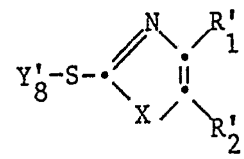

(XXI) (XXII),

worin die Reste $Y_8$ und $Y'_8$ unter Ausbildung einer Bindung abspaltbare Gruppen bedeuten, miteinander kondensiert, erforderlichenfalls ein verfahrensgemäss erhältliches Isomerengemisch in die reinen Isomeren auftrennt und gewünschtenfalls eine verfahrensgemäss erhältliche Verbindung in eine andere Verbindung der Formel I überführt und/oder eine verfahrensgemäss erhältliche freie Verbindung in ein Salz oder ein verfahrensgemäss erhältliches Salz in die freie Verbindung umwandelt.

Unter Ausbildung einer Bindung abspaltbare Gruppen $Y_3$ und $Y'_8$ sind beispielsweise Wasserstoffatome. Als weitere unter Ausbildung einer Bindung abspaltbare Paare $Y_8$ und $Y'_8$ kommen beispielsweise gegebenenfalls in Salzform, z.B. in Alkalimetallsalzform vorliegendes Mercapto und Halogen, z.B. Chlor, und in Salzform, z.B. in Alkalimetallsalzform vorliegende Sulfonsäuregruppen der Formel $-SO_3H$ in Betracht. Die Varianten, worin $Y_8$ und $Y'_8$ gleich sind und beispielsweise Wasserstoff oder in Salzform vorliegende Sulfonsäuregruppen darstellen, sind insbesondere zur Herstellung symmetrischer Disulfide, $R_1 = R'_1$, $R'_2 = R_2$) geeignet, während die Umsetzung von Verbindungen der Formeln XXI und XXII, worin $Y_8$ und $Y'_8$ verschieden sind und $Y_8$ ($Y'_8$) z.B. in Salzform vorliegendes Mercapto und $Y'_8$ ($Y_8$) z.B. Halogen ist, ebenso zur Herstellung unsymmetrischer Disulfide herangezogen werden kann.

Die Abspaltung von $Y_8$ und $Y'_8$ unter Ausbildung einer Bindung erfolgt in üblicher Weise, ausgehend von Verbindungen, worin $Y_8$ und $Y'_8$ Wasserstoff oder in Salzform vorliegende Sulfonsäuregruppen bedeuten, beispielsweise durch Einwirkung eines geeigneten Oxidationsmittels vorteilhaft in einem inerten Lösungsmittel, erforderlichenfalls unter

Kühlen oder Erwärmen und/oder unter Inertgas, wie Stickstoff. Geeignete Oxidationsmittel sind beispielsweise Halogene, wie Brom oder vor allem Jod, vorzugsweise in essigsaurer, alkoholischer und/oder wässriger Lösung, Alkalimetallhypohalogenite, z.B. Natriumhypojodid, welches vorzugsweise, z.B. durch Eintragung von Jod in wässrige und/oder alkoholische Natronlauge, in situ erzeugt wird, zur oxidativen Abspaltung von Wasserstoff ferner Wasserstoffperoxid, z.B. in etwa 10%-iger wässriger Lösung, oder Eisentrichlorid in Aether.

Die Ausgangsstoffe der Formeln XXI und XXII können nach an sich bekannten Methoden hergestellt werden, Buntesalze ($Y_8$ = $Y_8'$ = in Salzform vorliegende Sulfonsäuregruppen) beispielsweise durch Umsetzung der entsprechenden Verbindungen der Formel

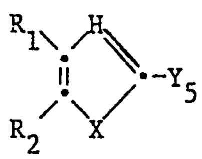 (XVI),

worin $Y_5$ Halogen ist, mit einem Salz der Thioschwefelsäure, beispielsweise mit Natriumthiosulfat, vorzugsweise in situ.

Ein weiteres Verfahren zur Herstellung von Verbindungen der Formel I, worin der Rest $R_3$ eine Gruppe der Formel Ia, in der $R_1'$ $R_1'$ einen Rest $R_1$, $R_2'$ einen Rest $R_2$ und m 0 bedeutet, ist dadurch gekennzeichnet, dass man eine Verbindung der Formel

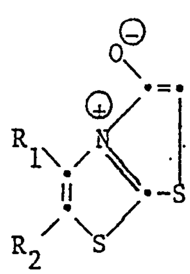 (XXIII)

der Basebehandlung unterwirft, wobei Umlagerung zu einer entsprechenden Verbindung der Formel I, worin in dem Rest $R_3$, $R_4$ 1,3-(2-Oxo)-propy-

len bedeutet, erfolgt, erforderlichenfalls ein verfahrensgemäss erhältliches Isomerengemisch in die reinen Isomeren auftrennt und gewünschtenfalls eine verfahrensgemäss erhältliche Verbindung in eine andere Verbindung der Formel I überführt und/oder eine verfahrensgemäss erhältliche freie Verbindung in ein Salz oder ein verfahrensgemäss erhältliches Salz in die freie Verbindung umwandelt.

Für die Basebehandlung geeignete Basen sind beispielsweise organische Stickstoffbasen, vorzugsweise tertiäre organische Stickstoffbasen, wie Triniederalkyl amine, z.B. Triäthylamin, oder vor allem aromatische tertiäre Stickstoffbasen, z.B. Pyridin. Die Basebehandlung erfolgt vorzugsweise in einem inerten Lösungsmittel oder einem Ueberschuss der verwendeten Base, erforderlichenfalls unter Kühlen oder Erwärmen und/oder unter Inertgas, wie Stickstoff.

Die Ausgangsstoffe der Formel XXIII weisen die gleichen pharmakologischen Eigenschaften in vergleichsweiser Wirkungsstärke auf wie die Verbindungen der Formel I. Sie sind, ebenso wie Verfahren zu ihrer Herstellung, sie enthaltende pharmazeutische Präparate und ihre Verwenndung, ebenfalls ein bevorzugter Gegenstand der Erfindung. Diese Verbindungen können hergestellt werden, indem man eine Verbindung der Formel

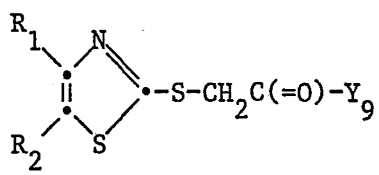 (XXIV),

worin $Y_9$ einen abspaltbaren Rest bedeutet, cyclisiert.

Abspaltbare Reste $Y_9$ sind beispielsweise gegebenenfalls veresterte Hydroxygruppen, wie mit anorganischen Säuren oder deren sauren Anhydriden bzw. sauren Estern verestertes Hydroxy, wie Halogen, Halogensulfinyl, Halogensulfonyl, Dihalogenphosphono und dergleichen, ferner mit Carbonsäuren, wie organischen Carbonsäuren oder Halbestern oder Halbanhydriden der Kohlensäure bzw. Thio- oder Dithiokohlensäure, verestertes Hydroxy, z.B. Niederalkanoyloxy, Niederalkoxycarbonyloxy, Halogencarbanyloxy oder Halogenthiocarbonyloxy.

Die Cyclisierung erfolt in üblicher Weise, vorteilhaft indem man eine Verbindung der Formel XXIV, worin $Y_9$ wie angegeben verestertes Hydroxy bedeutet, durch Umsetzung der entsprechenden Säure mit einem geeigneten Säureanhydrid, wie Phosgen,Phosphorpentachlorid, Thionylchlorid, Thiophosgen, einen Chlorameisensäureniederalkylester, einem Nideralkanoylchlorid oder vorzugsweise einem Niederalkansäureanhydrid in situ bildet und ohne Isolierung cyclisiert, erforderlichenfalls in Gegenwart eines basischen Kondensationsmittels, wie einer organischen Stickstoffbase, wie z.B. eines Triniederalkylamins oder von Pyridin, und/oder unter Erwärmen.

Eine bevorzugte Ausführungsform ist dadurch gekennzeichnet, dass man eine Säure der Formel XXIV oder ein Salz davon mit einem Säureanhydrid, z.B. mit Acetanhydrid behandelt. erforderlichenfalls in Gegenwart eines basischen Kondensationsmittels, z.B. von Triäthylamin.

Die neuen Verbindungen der Formel I können ferner hergestellt werden, indem man in einer Verbindung der Formel

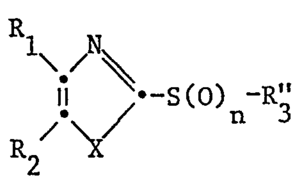

(XXV),

worin $R_3''$ einen in eine Gruppe $R_3$ überführbaren Rest bedeutet, $R_3''$ in die gewünschte Gruppe $R_3$ überführt, erforderlichenfalls ein verfahrensgemäss erhältliches Isomerengemisch in die reinen Isomeren auftrennt und gewünschtenfalls eine verfahrensgemäss erhältliche Verbindung in eine andere Verbindung der Formel I überführt und/oder eine verfahrensgemäss erhältliche freie Verbindung in ein Salz oder ein verfahrensgemäss erhältliches Salz in die freie Verbindung umwandelt.

In Reste $R_3$ überführbare Reste sind beispielsweise durch Carboxy substituierte Reste $R_3$, insbesondere 1- Carboxy-2-oxo-niederalkylreste oder Reste der Formel $-C(=O)-O-R_3$. Die Ueberführung dieser Reste in Reste $R_3$ erfolgt durch Abspaltung von Kohlendioxid.

Die Abspaltung von Kohlendioxid kann in üblicher Weise erfolgen, beispielsweise durch Säureeinwirkung, wie Behandlung mit einer Protonensäure, wie einer Mineralsäure, z.B. von Chlorwasserstoff- oder Schwefelsäure, vorteilhaft in einem Lösungs- oder Verdünnungsmittel, erforderlichenfalls unter Erwärmen, z.B. auf etwa 50 bis etwa 250°C.

So kann man Verbindungen der Formel XXV, worin $R_3''$ einen 1-Carboxy-2-oxo-niederalkylrest bedeutet, durch Erwärmen mit wässrigen Säurelösungen, z.B. mit gleichen Teilen etwa 15 %-iger Salzsäure und Essigsäure auf etwa 60 - 100°C, in Verbindungen der Formel I überführen, worin $R_3$ einen 2-Oxoniederalkylrest bedeutet.

Ferner kann man Verbindungen der Formel XXV, worin $R_3''$ einen Rest der Formel $-C(=O)-O-R_3$ darstellt, durch Erwärmen mit Chlorwasserstoff in Tetrahydrofuran auf etwa 40-80°C unter Kohlendioxidabspaltung in die entsprechenden Verbindungen der Formel I überführen.

Weitere in Reste $R_3$ überführbare Reste $R_3''$ sind beispielsweise gegebenenfalls in einer Ester-, Anhydrid- oder Salzform vorliegende Carboxyniederalkylreste der Formel $-C_nH_{2n}-COOH$, die in die Oxo- bzw. Hydroxygruppe in ω-Stellung tragenden Oxoniederalkylresten der Formel $-C_{n+1}H_{2n+1}=O$ bzw. Hydroxyniederalkylresten der Formel $-C_{n+1}H_{2n+2}-OH$ reduziert werden können.

Die Reduktion erfolgt in üblicher Weise, beispielsweise durch Umsetzung mit einem Hydridüberträger, der auf das α-ständige C-Atom Wasserstoff in anionischer Form überträgt, beispielsweise mit einem Leichtmetall- oder Dileichtmetallhydrides, z.B. mit Borhydrid-Aetherat

oder vorzugsweise Lithiumalminiumhydrid, vorteilhaft in einem Aether, wie einem Diniederalkyl- oder Niederalkylenäther, z.B. in Diäthyläther, Tertiärbutoxymethan oder Tetrahydrofuran, oder mittels Natriumborhydrid oder Natriumcyanoborhydrid, vorteilhaft in alkoholischer Lösung. Ebenso anwendbar sind Reduktionsmittel die nullwertige Metalle oder atomaren Wasserstoff übertragen. Dieser Prinzipien bedient man sich beispielsweise bei der metallischen Reduktion, die durch Einwirkung feinverteilter Metalle, z.B. von Zinkpulver, bewirkt werden kann, gleichermassen bei der Reduktion mit nascierendem Wasserstoff, der z.B. durch Säureeinwirkung auf unedle Metalle, wie von Essigsäure auf Zink, Eisen und Salzsäure oder Wassereinwirkung auf Natriumamalgam erzeugt werden kann. Durch die genannten Reduktionsmittel erhält man vorzugsweise Verbindungen der Formel I, worin $R_3$ einen Hydroxyniederalkylrest bedeutet. Salzformen von Carboxy sind dabei beispielsweise Basesalzformen, wie Alkali- oder Erdalkalimetallsalzformen, können aber auch Ammoniumsalzformen mit Ammoniak oder organischen Aminen sein. Anhydridformen sind beispielsweise gemischte Anhydridformen mit Halogenwasserstoffsäuren, können aber auch gemischte Anhydridformen mit organischen Carbonsäuren, wie Niederalkansäuren sein. Esterformen sind beispielsweise Niederalkylesterformen, können aber auch andere organische Esterformen, wie gegebenenfalls substituierte Phenylesterformen sein. Als weitere Esterformen kommen Lactonformen, z.B. γ-Lactonformen, in Betracht, bei deren Vorliegen in ω-Stellung und in einer niederen als der (ω-2)-Stellung zwei Hydroxygruppen aufweisende Reste $R_3$ gebildet werden. Die Reduktion von Reste $R_3''$, die eine in einer Salz-, Anhydrid- oder Esterform Carboxygruppe aufweisen, tragenden Verbindungen der Formel XXV kann aber auch auf der unter der Formel I fallenden Oxostufe aufgehalten werden, indem man selektive Reduktionsmittel verwendet. Solche sind für die Reduktion von gegebenenfalls in Salzform vorliegendem Carboxy beispielsweise Mono- oder Diniederalkylborhydride, z.B. 2-(2,3-Dimethyl-butyl)-borhydrid, für die Reduktion von Anhydridformen beispielsweise Wasserstoff in Gegenwart von Palladium oder Cyanwasserstoffsäure in Gegenwart von tertiären

aromatischen Stickstoffbasen, wie Chinolin, und für die Reduktion von Esterformen beispielsweise elektronegativ substituierte Aluminium- oder Alkalimetallaluminiumhydride, z.B. N-Methylpiperazino-aluminiumhydrid oder Natrium-bis-(2-methoxyäthoxy)-aluminiumhydrid, aber auch Dibutylaluminiumhydrid.

Als weitere Reduktionsmittel für die Reduktion von gegebenenfalls in einer Salz-, Anhydrid- oder Esterform vorliegenden Carboxy- zu Oxo- bzw. Hydroxygruppen kommen Organidüberträger, die auf das α-ständige C-Atom einen organischen Rest in anionischer Form übertragen, z.B. metallorganische Verbindungen, wie Verbindungen von aliphatischen, araliphatischen oder cycloaliphatischen organischen Verbindungen der Formel $R_o$-H (XXVI) mit Metalle, z.B. Verbindungen der Formel $R_o-M^I$, $R_o-M^{II}$-Hal oder $(R_o)_2M^{II}$, worin $M^I$ ein Metallatom der Gruppe 1A und $M^{II}$ ein Metallatom der Gruppen 2A und 2B des Periodischen Systems der Elemente darstellt und $M^I$ vorzugsweise für Natrium oder Lithium und $M^{II}$ vorzugsweise für Magnesium oder Cadmium steht. Die Reduktion mit diesen Reduktionsmitteln, bei der in einer Salz-, Anhydrid- oder Esterform vorliegende Carboxygruppen in tertiäre Hydroxygruppen überführt werden, erfolgt in üblicher Weise beispielsweise in einem inerten Lösungsmittel, wie einen Diniederalkyl- oder Niederalkylenäther, z.B. in Diäthyläther oder Tetrahydrofuran, erforderlichenfalls unter Kühlen oder Erwärmen und/oder unter Inertgas wie Stickstoff. Auch hier kann die Reduktion von gegebenenfalls in einer Salz-, Anhydrid- oder Esterform vorliegenden Carboxyverbindung gewünschtenfalls auf der Oxostufe angehalten werden, indem man milde Reduktionsmittel, z.B. eine Cadmium- oder Halogencadmium-Verbindung bzw. eine Halogenmagnesiumverbindung in Gegenwart eines Cadmium halogenides, verwendet oder bei tiefen Temperaturen, z.B. unter -25°C, arbeitet.

Weitere zu einer zumindestens eine an ein gesättigtes Kohlenstoffatom gebundene Hydroxygruppe aufweisenden Gruppe $R_3$ reduzierbare Reste $R_3''$ sind beispielsweise mindestens eine mit einem α-Phenylniederalkanol verätherte oder mit einer Carbonsäure, wie einer Niederalkansäure oder der Kohlensäure bzw. einem Halbester oder Halbamid derselben veresterte Hydroxygruppen, wie gegebenenfalls substituiertes Benzyloxy. gegebenenfalls halogeniertes Niederalkanoyloxy oder Niederalkoxycarbonyloxy, Benzyloxycarbonyloxy, Diniederalkylcarbamyloxy oder Carbonyldioxy, aufweisende Reste $R_3''$. Als solche kommen beispielsweise in Betracht: die Benzyloxy-, Benzyloxycarbonyloxy-, die gegebenenfalls halogenierte Niederalkanoyloxy- oder Niederalkoxycarbonyloxygruppe(n) bzw. die Carbonyldioxygruppe in höherer als der α-Stellung tragendes, gegebenenfalls substituiertes Mono- oder Dibenzyloxyniederalkyl, gegebenenfalls halogeniertes Mono- oder Diniederalkanoyloxyniederalkyl bzw. Mono- oder Diniederalkoxycarbonyloxy- oder Carbonyldioxyniederalkyl, ebenso entsprechende von Gruppen der Formel Ia abgeleitete Gruppen, die anstelle von $R_4$ einen die Benzyloxy-, gegebenenfalls halogenierte Niederalkanoyloxy- bzw. Niederalkoxycarbonyloxy oder Benzyloxycarbonyloxy in höherer als der α- und in niederer als der ω-Stellung tragendes Benzyloxy-, gegebenenfalls halogeniertes Niederalkanoyloxy- bzw. Niederalkoxycarbonyloxy- oder Benzyloxycarbonyloxyniederalkylen aufweisen. Weiterhin kommen Reste $R_3''$ der Formel

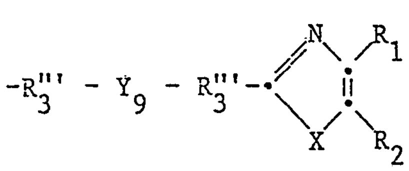

(XXVa),

worin $R_3'''$ einen zweiwertigen, die Oxygruppe in höherer als der α-Stellung tragenden, durch Oxy und zusätzlich gegebenenfalls wie angegeben substituierten aliphatischen, cycloaliphatischen oder cycloaliphatisch-aliphatischen Kohlenwasserstoffrest und $Y_9$ Carbonyl oder Thiocarbonyl

bedeutet. Als Reduktionsmittel kommen zur Reduktion von α-Phenylniederalkoxy und α-Phenylalkoxycarbonyloxygruppen sowie von Resten der Formel XXVa beispielsweise Wasserstoff in Gegenwart eines Hydrierkatalysators, wie von Platin oder Palladium, in Betracht, wobei man vorteilhaft in einem inerten Lösungsmittel, erforderlichenfalls bei erhöhtem Druck und/oder unter Kühlen oder Erwärmen, arbeitet. Mit Carbonsäuren veresterte Hydroxygruppen, auch α-Phenylniederalkoxycarbonyloxygruppen, können beispielsweise mittels Dichleichtmetallhydriden, z.B. mit Lithiumaluminiumhydrid, reduziert werden.

2-Halogen-niederalkoxycarbonyloxy (gegebenenfalls nach Umwandlung einer 2-Brom-niederalkoxycarbonyloxygruppe in eine 2-Jod-niederalkoxycarbonyloxygruppe), Aroylmethoxycarbonyloxy oder 4-Nitrobenzyloxycarbonyloxy kann z.B. durch Behandeln mit einem geeigneten chemischen Reduktionsmittel, wie Zink in Gegenwart einer geeigneten Carbonsäure, wie wässriger Essigsäure, abgespalten werden. Aroylmethoxycarbonyloxy kann auch durch Behandeln mit einem nucleophilen, vorzugsweise salzbildenden Reagens, wie Natriumthiophenolat, und 4-Nitro-benzyloxycarbonyloxy auch durch Behandeln mit einem Alkalimetall-, z.B. Natriumdithionit, gespalten werden.

Weitere Reste $R_3''$ sind zu einer mindestens eine an ein gesättigtes C-Atom gebundene Oxo- oder Hydroxygruppe aufweisenden Gruppe $R_3$ oxidierbare Reste, beispielsweise mindestens eine nicht-aromatische C-C-Doppelbindung aufweisende Reste $R_3''$, die in üblicher Weise z.B. durch Einwirkung eines oxidierenden Metalloxides oder Salzes einer oxidierenden Metallsäure an der Doppelbindung dihydroxyliert bzw. zu einem Oxo aufweisenden Rest $R_3$ gespalten oder durch Umsetzung mit Diboran und anschliessende Behandlung mit Wasserstoffperoxid monohydroxyliert werden können. Als oxidierendes Metalloxid kommt beispielsweise Chromtrioxid oder Osmiumtetroxid und als Salz einer oxidierenden Metallsäure beispielsweise Kaliumpermanganat in Betracht. Die Oxidation mit diesen Mitteln, ebenso die Umsetzung mit Diboran und anschliessende Behandlung mit Wasserstoffperoxid erfolgt in üblicher Weise, vorteilhaft in einem inerten Lösungsmittel, erforderlichenfalls unter Kühlen oder Erwärmen und/oder unter Inertgas, wie Stickstoff.

Weitere Reste $R_3''$ sind zu einer mindestens eine an ein gesättigtes C-Atom gebundene Hydroxygruppen aufweisenden Gruppe $R_3$ solvolysierbare Reste, beispielsweise Reste $R_3''$, die mindestens eine veresterte Hydroxygruppe, mindestens eine Silylgruppe oder eine Mono- oder Diarylniederalkylidendioxy- bzw. Mono- oder Diarylniederalkylidendithiogruppe aufweisen, ferner Reste der Formel XXVa, worin Carbonyl, Thiocarbonyl, Diniederalkoxy- bzw. Diniederalkylendioxymethylen oder Niederalkyliden, Mono- oder Diarylniederalkyliden bzw. Cycloalkyliden bedeutet.

Veresterte Hydroxygruppen sind beispielsweise Halogenatome, die vorzugsweise durch Umsetzung mit einem Salz einer organischen Carbonsäure, wie Natriumacetat, in organisches Acyloxy, wie Acetoxy, überführt werden, vor allem aber Acyloxygruppen. In Mono- oder Diarylniederalkylidendioxy- bzw. Mono- oder Diarylniederalkylidendithioresten bedeutet Aryl vorzugsweise Phenyl, das auch, z.B. durch Niederalkyl, Niederalkoxy und/oder Halogen, substituiert sein kann.

Acyloxygruppen sind beispielsweise von einer durch Halogen oder Aryl substituierten Alkancarbonsäure oder gegebenenfalls, z.B. durch Halogen, Niederalkoxy oder Nitro, substituierten Benzoesäure, oder einem Kohlensäurehalbester abgeleitete Acyloxygruppen. Solche Acyloxygruppen sind beispielsweise Halogenniederalkanoyloxy, wie 2-Halogenacetoxy, insbesondere 2-Chlor-, 2-Brom-, 2-Jod-, 2,2,2-Trifluor- oder 2,2,2-Trichloracetoxy, gegebenenfalls, z.B. durch Halogen, Niederalkoxy oder Nitro substituiertes Benzyloxy,

z.B. Benzoyloxy, 4-Chlorbenzoyloxy, 4-Methoxybenzoyloxy oder 4-Nitrobenzoyloxy, oder in 1-Stellung des Niederalkylrestes verzweigtes oder 1- oder 2-Stellung geeignet substituiertes Niederalkoxycarbonyloxy, insbesondere tert.-Niederalkoxycarbonyloxy, z.B. tert.-Butyloxycarbonyloxy, Arylmethoxycarbonyloxy mit einem oder zwei Arylreste, die vorzugsweise gegebenenfalls, z.B. durch Niederalkyl, insbesondere tert.-Niederalkyl, wie tert.-Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyloxy, z.B. 4-Nitrobenzyloxycarbonyloxy, oder substituiertes Diphenylmethoxycarbonyloxy, Aroylmethoxycarbonyloxy, worin die Aroylgruppen vorzugsweise gegebenenfalls, z.B. durch Halogen, wie Brom, substituiertes Benzoyl darstellt, z.B. Phenacyloxycarbonyloxy, 2-Halogen-niederalkoxycarbonyloxy, z.B. 2,2,2-Trichloräthoxycarbonyloxy, 2-Bromäthoxycarbonyloxy oder 2-Jodäthoxycarbonyloxy, oder 2-(trisubstituiertes Silyl)-äthoxycarbonyloxy, worin die Substituenten unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilyläthoxycarbonyloxy, wie 2-Trimethylsilyläthoxycarbonyloxy oder 2-(Di-n-butyl-methyl-silyl)-äthoxycarbonyloxy, oder 2-Triarylsilyläthoxycarbonyloxy, wie 2-Triphenylsilyläthoxycarbonyloxy.

Vor- und nachstehend bedeuten Arylreste vorzugsweise Phenyl, das, z.B. wie für $R_1$ bzw. $R_2$ angegeben und/oder durch Nitro, substituiert sein kann.

Weitere Acylreste sind auch entsprechende Reste organischer Phosphor-, Phosphon- oder Phosphinsäuren, wie Diniederalkylphosphoryloxy, z.B. Dimethylphosphoryloxy, Diäthylphosphoryloxy, Di-n-propylphosphoryloxy oder Diisopropylphosphoryloxy, Dicycloalkylphosphoryloxy, z.B. Dicyclohexylphosphoryloxy, gegebenenfalls substituiertes Diphenylphosphoryloxy, z.B. Diphenylphosphoryloxy, gegebenenfalls, z.B. durch Nitro substituiertes Di-phenylniederalkylphosphoryloxy, z.B. Dibenzylphosphoryloxy oder Di-4-nitrobenzylphosphoryloxy, gegebenenfalls substituiertes Phenyloxyphenylphosphonyloxy, z.B. Phenyloxy-phenylphosphonyloxy, Diniederalkylphosphinyloxy, z.B. Diäthylphosphinyloxy, oder gegebenenfalls substituiertes Diphenylphosphinyloxy, z.B. Diphenylphosphinyloxy.

Eine Silyloxygruppe ist in erster Linie eine organische Silyloxygruppe, worin das Silicium vorzugsweise Niederalkyl, insbesondere Methyl, ferner Niederalkoxy, z.B. Methoxy, und/oder Halogen, z.B. Chlor, als Substituenten enthält. Entsprechende Silyloxygruppen sind in erster Linie Triniederalkylsilyloxy, insbesondere Trimethylsilyloxy, ferner Dimethyl-tert.-butyl-silyloxy, Niederalkoxyniederalkyl-halogen-silyloxy, z.B. Methoxy-methyl-chlor-silyloxy, oder Diniederalkyl-halogensilyloxy, z.B. Dimethyl-chlor-silyloxy.

Die genannten, zu Hydroxy solvolysierbaren Gruppen aufweisende Reste $R_3''$ sind beispielsweise die Acyloxygruppe(n), Silyloxygruppe(n), oder Mono- oder Diarylniederalkylidendioxygruppe bzw. oder Mono- oder Diarylniederalkylidendithiogruppe in höhere als der α-Stellung tragendes Mono- oder Diacylniederalkyl, Mono- oder Disilyloxyniederalkyl, Mono- oder Diarylniederalkylidendioxyniederalkyl bzw. Mono- oder Diarylniederalkylidendithioniederalkyl, ferner Reste der Formel XXVa, worin $R_3'''$ die Oxogruppe in höherer als

der α-Stellung tragendes Oxyniederalkylen und $Y_9$ Carbonyl oder Thiocarbonyl bedeutet, ebenso entsprechende von Gruppen der Formel Ia abgeleitete Reste die als bzw. anstelle von $R_4$ die Acyloxygruppe(n), Silyloxygruppe(n), Niederalkylidendioxy- oder Mono- oder Diarylniederalkylidendioxygruppe bzw. Niederalkylidendithio- oder Mono- oder Diarylniederalkylidendithiogruppe in höherer als der α- und in niederer als der ω-Stellung tragendes Mono- oder Diacyloxyniederalkylen, Mono- oder Disilyloxyniederalkylen, Niederalkyliden- bzw. Mono- oder Diarylniederalkylidendioxyniederalkylen oder Niederalkyliden- bzw. Mono- oder Diarylniederalkylidendithioniederalkylen aufweisen.

Die Solvolyse erfolgt beispielsweise durch Hydrolyse (Behandlung mit Wasser), Alkoholyse (Behandlung mit einem Alkohol) oder Ammono- bzw. Aminolyse (Behandlung mit Ammoniak oder einem organischen Amin).

Die Hydrolyse wird in üblicher Weise durchgeführt, erforderlichenfalls in Gegenwart eines Hydrolysemittels, und/oder eines inerten Lösungsmittels, unter Kühlen oder Erwärmen und/oder unter Inertgas. Hydrolysemittel sind beispielsweise saure oder alkalische Mittel. Saure Mittel sind beispielsweise wie Mineralsäuren, Halogenwasserstoffsäuren, z.B. Chlor-, Brom- oder Jodwasserstoffsäure, oder Sauerstoffsäuren des Schwefels oder Phosphors bzw. deren saure Salze, wie Schwefelsäure, Kaliumhydrogensulfat oder Phosphorsäure, oder organische Carbon- oder Sulfonsäuren, z.B. Niederalkansäure, wie Essigsäure, Trifluoressigsäure oder Chloressigsäure, oder p-Toluolsulfonsäure. Basische Mittel sind beispielsweise Hydroxide oder Carbonate von Alkali- oder Erdalkalimetallen, wie Kalium-, Natrium- oder Calciumhydroxid, bzw. Kalium- oder Natriumcarbonat. Inerte Lösungsmittel sind beispielsweise polare, mit Wasser mischbare Lösungsmittel, wie Alkohole, z.B. Methanol oder Aethanol, Niederalkylenäther, wie Dioxan,

Diniederalkylketone, wie Aceton, tertiäre Amide, z.B. Dimethylformamid, N-Methylpyrrolidon, oder Hexamethylphosphorsäuretriamid, oder Diniederalkylsulfoxide, z.B. Dimethylsulfoxid.

Durch Alkoholyse können insbesondere mit organischen Carbonsäure veresterte Hydroxygruppen durch Umesterung freigesetzt werden. Die Umesterung erfolgt in üblicher Weise, beispielsweise in Gegenwart eines sauren oder basischen Mittels, erforderlichenfalls unter Kühlen oder Erwärmen und/oder unter Inertgas, wie Stickstoff. Als saure bzw. basische Mittel kommen z.B. die genannten in Betracht, als basische Mittel ferner Metallakoholate, wie Alkalimetall- oder Erdalkalimetallniederalkanolate, z.B. Natriummethanolat.

Aus den genannten Acyloxygruppen kann die Hydroxygruppe aber auch durch Aminolyse freigesetzt werden. Die Aminolyse erfolgt in üblicher Weise, erforderlichenfalls unter Kühlen oder Erwärmen und/oder unter Inertgas, wie Stickstoff.

2-Halogenniederalkanoyloxy kann aber auch durch Umsetzung mit Thioharnstoff zunächst in das Isothiuroniumderivat überführt und anschliessend durch Hydrolyse oder Alkoholyse desselben gespalten werden. 2-Substituiertes Silyläthoxycarbonyloxy lässt sich vorteilhaft durch Behandeln mit einem Alkalimetallfluorid, z.B. mit Kaliumfluorid, spalten. Phosphoro-, Phosphono- oder Phosphinogruppen können z.B. durch Behandeln mit einer Phosphor-haltigen Säure, wie einer Phosphor-, Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat oder Monomethylphosphonsäure, oder einem Anhydrid davon, wie Phosphorpentoxid, abgespalten werden.

Die Ausgangsstoffe können nach an sich bekannten Methoden hergestellt werden, beispielsweise durch Abspaltung von HY aus Verbindungen der Formel

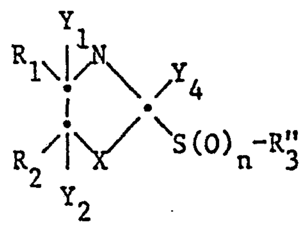

(XXVII),

worin $Y_1$, $Y_2$, $Y_3$ und $Y_4$ die unter der Formel II angegebenen Bedeutungen haben, durch Umsetzung von Verbindungen der Formeln

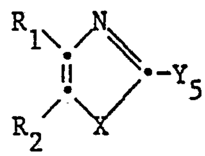

(XVI) und $Y_6$-$R''_3$ (XXVIII),

worin einer der Reste $Y_5$ und $Y_6$ eine reaktionsfähige veresterte Hydroxygruppe und der andere eine in Salzform vorliegende Gruppe der Formel $-S(O)_n-$ oder -S-SH bedeutet, oder, sofern $R''_3$ α-Phenylniederalkoxy, Mono- oder Diarylniederalkylidendioxy, Mono- oder Diarylniederalkylidendithio oder Silyloxy aufweist oder eine Gruppe der Formel XXVa darstellt, worin $Y_9$ Niederalkyliden, Mono- oder Diarylniederalkyliden oder Cycloalkyliden darstellt, einer der Reste $Y_5$ und $Y_6$ einen metallischen Rest und der andere eine reaktionsfähige veresterte Hydroxygruppe darstellt, oder sofern n für 1 oder 2 steht, veräthertes Hydroxy, oder, sofern n für 0 steht, veräthertes Mercapto bedeutet.

Verbindungen der Formel XXV, worin $R_3''$ an vicinale C-Atome gebundene Acyloxygruppen, vorzugsweise von organischen Carbonsäuren abgeleitete Acyloxygruppen, wie Niederalkanoyloxy, aufweist, können ferner durch oxidative Acyloxylierung der Doppelbindung in entsprechenden nicht-aromatische Doppelbindung(en) aufweisenden Verbindungen der Formeln I bzw. XXV hergestellt werden. Die oxidative Acyloxylierung erfolgt beispielsweise durch Behandeln mit einem Schwermetallsalz einer organischen Carbonsäure, z.B. mit Bleitetraacetat, oder mit einem Silbersalz einer organischen Carbonsäure in Gegenwart von Halogen, z.B. mit Silberacetat in Gegenwart von Jod oder Brom.

Verfahrensgemäss oder nach anderen vorstehend nicht erwähnten Verfahrensweisen erhältliche Verbindungen der Formel I können in an sich bekannter Weise in andere Verbindungen der Formel I umgewandelt werden.

So kann man beispielsweise gegebenenfalls verätherte oder veresterte Hydroxygruppen ineinander überführen.

Weist beispielsweise mindestens einer der Reste $R_1$, $R_2$ und $R_3$ eine Hydroxygruppe auf, so lässt sich diese auf üblichem Wege veräthern. So kann man Hydroxy als Bestandteil von $R_3$ mit einem Niederalkylierungsmittel, wie einem Niederalkanol, z.B. Methanol, in Gegenwart einer Säure, wie einer Mineralsäure, z.B. Schwefelsäure, oder eines Dehydratisierungsmittels, wie Dicyclohexylcarbodiimid, und Hydroxy

an einem Rest $R_1$ bzw. $R_2$ z.B. in Gegenwart von Basen, wie Alkalimetallhydroxiden oder -carbonaten, z.B. Natriumhydroxid oder Kaliumcarbonat, mit Hilfe von Diniederalkylsulfaten oder Diazoniederalkanen in entsprechende Niederalkoxygruppen überführt. Umgekehrt lassen sich Aether, wie Niederalkoxygruppen als Substituenten von $R_1$ bzw. $R_2$ abspalten, beispielsweise durch Behandeln mit Säuren, wie mit Lewissäuren, z.B. Bortribromid oder Mineralsäuren, z.B. von Jodwasserstoff.

Weiterhin lässt sich Hydroxy verestern, z.B. in Niederalkanoyloxy umwandeln, beispielsweise durch Umsetzung mit einer entsprechenden Niederalkancarbonsäure, wie Essigsäure, oder einem reaktionsfähigen Derivat, wie einem symmetrischen Anhydrid, davon oder einem Anhydrid mit einer Halogenwasserstoffsäure, erforderlichenfalls in Gegenwart eines Kondensationsmittels, ausgehend von Anhydriden z.B. eines basischen Kondensationsmittels, wie eines Alkalimetallhydroxides oder -carbonats oder einer tertiären Stickstoffbase, z.B. eines Triniederalkylamins oder von Pyridin, und ausgehend von einer Säure, z.B. in Gegenwart einer Säure, wie einer Protonensäure, z.B. Chlorwasserstoff-, Schwefel-, Phosphor- oder einer Sulfonsäure, oder einer Lewissäure, z.B. Bortrifluorid-Etherat.

Ferner kann man in Verbindungen der Formel I Reste $-S(O)_n-$ und/oder $-S(O)_m-$ worin n bzw. m für 0 steht, zu den entsprechenden Sulfinyl- oder Sulfonylresten, worin n bzw. m für 1 oder 2 steht, Reste $-S(O)_n-$ und/oder $-S(O)_m-$ worin n bzw. m für 1 steht, zu den entsprechenden Sulfonylresten, worin n 2 bedeutet, und/oder Heteroarylreste $R_1$, $R_2$, $R_1'$ und/oder $R_2'$ mit mindestens einem freien Ringstickstoffatom, wie Pyridylreste, N-oxidieren. Die Oxidation erfolgt vorzugsweise durch Einwirkung eines geeigneten

Oxidationsmittels, vorteilhaft in einem diesem gegenüber inerten Lösungsmittel, erforderlichenfalls unter Kühlen oder Erwärmen, z.B. im Temperaturbereich von etwa -30° bis +100°C, vorzugsweise bei etwa 0° bis 60°C, in einem geschlossenen Gefäss und/oder unter Intergas, wie Stickstoff. Geeignete Oxidationsmittel sind beispielsweise Peroxyverbindungen, wie Wasserstoffperoxid, organische Hydroperoxide, z.B. tert.-Butylhydroperoxid, organische Persäuren, wie aromatische oder aliphatische Percarbonsäuren, z.B. m-Chlorperoxybenzoesäure, Peroxyessigsäure oder Permonophthalsäure, oxidierende Schwermetallverbindungen, wie Chrom-VI- oder Mangan-IV bzw. Mangan-VII-verbindungen, z.B. Chromtrioxid, Chromsäure, Mangandioxid oder Kaliumpermanganat, oxidierende anorganische Sauerstoffsäuren, wie Sauerstoffsäuren des Stickstoffs, der Halogene oder Chalkogene, oder deren Anhydride oder Salze, z.B. Salpetersäure, Distickstofftetroxid, Selendioxid oder Natriummetaperjodat, ferner Ozon. Geeignete Lösungsmittel sind beispielsweise halogenierte Kohlenwasserstoffe, wie Halogenalkane, z.B. Kohlenstofftetrachorid, Chloroform oder Methylenchlorid, oder Carbonsäuren, wie Alkansäuren, z.B. Essigsäure, oder deren Anhydride.

In einer bevorzugten Ausführungsform dieses Oxidationsverfahrens kann man beispielsweise Thioäther der Formel I, worin n und m für 0 stehen, und/oder ein Rest $R_1'$, $R_2$, $R_1'$ und/oder $R_2'$ ein unsubstituiertes Ringstickstoffatom aufweist, durch Umsetzung mit einer organischen Persäure, z.B. mit m-Chlorperbenzoesäure, in einem Halogenalkan, z.B. in Chloroform, zu den entsprechenden Sulfinyl- oder Sulfonylverbindungen, worin n und/oder m für 1 oder 2 stehen, und gewünschtenfalls am Stickstoffatom oxidieren.

In einer anderen bevorzugten Ausführungsform kann man Thioäther der Formel I, worin n und/oder m für 0 steht, durch Behandeln mit Natriummetaperjodat, vorzugsweise in einem Halogenalkan,

z.B. in Kohlenstofftetrachlorid oder Chloroform, selektiv zu den entsprechenden Sulfoxiden, in denen n und/oder m für 1 steht, oder diese mit Wasserstoffperoxid in Essigsäure zu Sulfonen, worin n und/oder m für 2 steht, oxidieren.

Umgekehrt kann man in Verbindungen der Formel I, worin n und/oder m für 1 oder 2 steht und/oder Heteroarylreste $R_1$, $R_2$, $R_1'$ und/oder $R_2'$ N-oxidiert sind, die Gruppe -S(O)- bzw. $-S(O)_2$ zu Thio und/oder N-oxidierten Ringstickstoff reduzieren. Die Reduktion erfolgt durch Behandeln mit üblichen Reduktionsmitteln, z.B. mit nascierendem oder katalytisch aktiviertem Wasserstoff, wie Eisen oder Zink und Säure, wie Salzsäure, oder mit Wasserstoff in Gegenwart von Raney-Nickel, vorteilhaft in einem inerten Lösungsmittel, wie einem Niederalkanol oder mit Leichtmetallhydriden bzw. Dileichtmetallhydriden, z.B. mit Alkalimetallaluminium- oder Alkalimetallborhydriden, z.B. mit Natriumborhydrid oder Lithiumaluminiumhydrid, vorteilhaft in einem inerten Lösungsmittel, wie einem Aether, z.B. Diäthyläther oder Tetrahydrofuran, oder zur selektiven Reduktion von Gruppen N-Oxiden mit einer Phosphor-III-verbindung, wie einem Phosphin, z.B. Triphenylphosphin oder Tri-n-butylphosphin, oder einem Phosphorigsäureester, wie einem Triniederalkylphosphit, z.B. mit Trimethyl- oder Triäthylphosphit.

Ferner kann man in die Reste $R_1$, $R_2$, $R_1'$ und/oder $R_2'$ gegebenenfalls zusätzliche C-Substituenten einführen. So kann man in üblicher Weise halogenieren, z.B. durch Umsetzung mit Chlor oder Brom in Gegenwart von Eisen oder mittels N-Chlorsuccinimid. Ferner kann man in üblicher Weise, z.B. durch Umsetzung mit einem Alkylhalogenid, Alkanol oder Alken in Gegenwart von Aluminiumtrichlorid, alkylieren. Weiterhin kann man in üblicher Weise, z.B. mittels Salpetersäure/Schwefelsäure, nitrieren, die Nitrogruppe, z.B. mit Zinn-II-chlorid zu Amino reduzieren und diese mittels Natriumnitrit

und Tetrafluorborsäure in Fluor, mittels Salzsäure, Natriumnitrit und Kupfer-I-chlorid (-bromid) durch Chlor (Brom), mittels Natriumnitrit und Kaliumjodid durch Jod, mittels Natriumnitrit und einem Niederalkylmercaptan durch Niederalkylthio bzw. mittels Natriumnitrit in Hydroxy überführen.

Ferner kann man in Oxo aufweisenden Resten $R_3$ Oxo zu Hydroxy reduzieren bzw. reduktiv durch Wasserstoff ersetzen, beispielsweise durch Einwirkung von nascierendem Wasserstoff, beispielsweise erzeugt durch Behandlung unedler Metalle mit Protonendonatoren, z.B. von Zink mit Salz- oder Essigsäure, von amalgamiertem Aluminium oder Natriumamalgam mit Wasser oder von Natrium mit einem Alkohol, wie Methanol. Der reduktive Ersatz von Oxo durch Wasserstoff kann auch erfolgen durch Umsetzung mit Hydrazin in Gegenwart einer Metallbase, z.B. von Natrium- oder Kaliumhydroxid oder eines Alkalimetallalkoholates, wie von Natriummethanolat in einem hochsiedenden Alkohol, wie Aethylenglykol, Diäthylenglykol oder Diäthylenglykolmonomethyläther, vorzugsweise bei erhöhter Temperatur, z.B. bei etwa 150 bis 250°C. Oxo kann ferner in üblicher Weise zu Hydroxy reduziert werden, beispielsweise durch Umsetzung mit einem Leichtmetallhydrid, wie Boran-Tetrahydrofuran oder Diboran, oder einem Dileichtmetallhydrid, wie Natriumborhydrid, Natriumcyanoborhydrid oder Lithiumaluminiumhydrid, oder durch Umsetzung mit einem sekundären Alkohol, wie Isopropanol oder Cyclohexanol, in Gegenwart eines Aluminiumalkoholates. Die Reduktion von Oxo zu Hydroxy kann aber auch unter gleichzeitiger Einführung eines Kohlenwasserstoffrestes am Carbonylkohlenstoffatom erfolgen, indem man mit einer Niederalkylmetallverbindung, z.B. mit einem Niederalkyllithium oder Niederalkylmagnesiumhalogenid, umsetzt. In analoger Weise kann man durch Aldoladdition eines Ketons oder Aldehydes an eine aldehydische Oxogruppe unter Reduktion derselben zu Hydroxy einen in β-Stellung Oxo aufweisenden Rest einführen. Oxo kann ferner durch Behandlung mit

einem Orthocarbonsäureester, z.B. einem Orthoameisensäureniederalkylester, oder unter wasserabspaltenden Bedingungen und Säurekatalyse mit einem Niederalkanol, Niederalkandiol, Niederalkylmercaptan bzw. Niederalkyldimercaptan, in Diniederalkoxy, Niederalkylendioxy, Diniederalkylthio bzw. Niederalkylendithio überführt werden. Ebenso kann man Aldehyde der Formel I, worin $R_3$ eine ω-Oxogruppe aufweist, durch Umsetzung mit einem Metall-hydrogensulfit, z.B. mit Natriumhydrogensulfit(-bisulfit), in die Bisulfitadditionsprodukte, worin $R_3$ eine ω-Hydroxy- sowie eine in Salzform vorliegende ω-Sulfogruppe aufweist, umwandeln.

Weiterhin kann man in Hydroxy aufweisenden Resten $R_3$ die Hydroxygruppe(n) reduktiv durch Wasserstoff ersetzen, beispielsweise wie oben für den reduktiven Ersatz von Oxo durch Einwirkung von Wasserstoff angegeben. Hydroxy kann man ferner durch Umsetzung mit einer oxidierenden Schwermetallverbindung, z.B. mit Silberacetat oder Wismutoxid, mit Dimethylsulfoxid in Gegenwart von Trifluormethansulfonsäureanhydrid oder N-Chlorsuccinimid, oder mit einem Cyclohexanon, in Gegenwart eines Aluminiumalkoholats, wie Aluminiumisopropylat, zu Oxo oxidieren. Ferner kann man Hydroxyalkyl $R_3$ durch Säurebehandlung unter Wasserabspaltung in Alkenyl bzw. Niederalkoxyalkenyl durch saure Hydrolyse in Alkyl überführen.

In den Resten $R_3$ kann man ferner geminal gebundenes Diniederalkoxy, Diniederalkylthio, Niederalkylendioxy und Niederalkylendithio zu Oxo bzw. vicinal gebundenes Niederalkylidendioxy aufweisendes Niederalkyl zu Dihydroxyniederalkyl hydrolysieren, vorzugsweise säurekatalytisch.

Erfindungsgemäss erhältliche Stereoisomerengemische können in üblicher Weise in die Komponenten aufgetrennt werden.

So kann Diastereomerengemische auf Grund der Unterschiede der physikalischen Eigenschaften der Komponenten durch übliche physikalische Trennverfahren, wie Kristallisations-, Chromatographie-, Destillations- oder Phasenverteilungsverfahren in die Komponenten auftrennen.

Enantiomerengemische, wie Racemate, können durch Kristallisation aus optisch aktiven Lösungsmitteln, Chromatographie an optisch aktiven Feststoffen, Einwirkung von Mikroorganismen oder Umsetzung mit einer optisch aktiven Hilfsverbindung in Diastereomerengemische, z.B. mit einer optisch aktiven Säure in Gemische diastereomerer Säureadditionssalze, und Trennung derselben in die Diastereomeren aus denen die Enantiomeren in der jeweils üblichen Weise freigesetzt werden können an die Enantiomeren gespalten werden. Für diesen Zweck übliche optisch aktive Säuren sind z.B. D- oder L-Weinsäure, Di-o-Toluylweinsäure, Aepfelsäure,Mandelsäure, Camphersulfonsäuren oder Chinasäure.

Ferner können erhaltene freie Verbindungen in an sich bekannter Weise in Säureadditionssalze überführt werden, z.B. durch Umsetzen einer Lösung der freien Verbindung in einem geeigneten Lösungsmittel oder Lösungsmittelgemisch mit einer der vorgenannten Säuren oder mit einer Lösung davon, oder mit einem geeigneten Anionenaustauscher.

Erhaltene Säureadditonssalze können in an sich bekannter Weise in die freien Verbindungen umgewandelt werden, z.B. durch

Behandeln mit einer Base, wie einem Alkalimetallhydroxid, einem Metallcarbonat oder -hydrogencarbonat, oder Ammoniak, oder mit einem geeigneten Anionenaustauscher.

Erhaltene Säureadditonssalze können in an sich bekannter Weise in andere Säureadditionssalze überführt werden, z.B. durch Behandlung eines Salzes einer organischen Säure mit einem geeigneten Metallsalz, wie einem Natrium-, Barium- oder Silbersalz, einer Säure in einem geeigneten Lösungsmittel, in welchem ein sich bildendes anorganisches Salz unlöslich ist und damit aus dem Reaktionsgemisch ausscheidet, in andere Säureadditionssalze übergeführt werden.

Die Verbindungen, einschliesslich ihrer Salze können auch in Form der Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckmässig gegebenenfalls auch die entsprechenden Salze bzw. freien Verbindungen zu verstehen.

Die Erfindung betrifft auch diejenigen Ausführungsformen des Verfahrens, bei denen man von einer auf beliebiger Verfahrensstufe als Zwischenprodukte erhältlichen Verbindungen ausgeht und die fehlenden Verfahrensschritte ausführt, oder wobei ein Ausgangsstoff unter den Reaktionsbedingungen gebildet oder in Form eines Derivats davon, gegebenenfalls eines Salzes, verwendet wird.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe sowie Verfahren zu ihrer Herstellung bilden ebenfalls einen Gegenstand der vorliegenden Erfindung.

So weisen die als Ausgangsstoffe genannten Verbindungen der Formel XXIII die gleichen pharmakologischen Eigenschaften in vergleichbarer Wirkungsstärke auf wie die Verbindungen der Formel I und können deshalb ebenfalls als antirheumatische bzw. antinociceptive Arzneimittelwirkstoffe verwendet werden.

Die Erfindung betrifft dementsprechend ebenfalls Verbindungen der Formel XXIII, Verfahren zu ihrer Herstellung, diese enthaltende Präperate und ihre Verwendung als Arzneimittelwirkstoffe, wobei $R_1$ und $R_2$ insbesondere die für die eingangs als bevorzugt hervorgehobenen Verbindungen der Formel I angegebenen Bedeutungen haben.

Bei den erfindungsgemässen pharmazeutischen Präparaten, welche Verbindungen der Formel I oder pharmazeutisch verwendbare Salze davon enthalten, handelt es sich um solche zur enteralen wie oralen oder rektalen, und parenteralen Verabreichung sowie zur topischen Anwendung an Warmblüter(n), welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Im Normalfall ist für einen etwa 75 kg schweren Warmblüter bei oraler Applikation eine ungefähre Tagesdosis von etwa 10-100 mg, vorteilhaft in mehreren gleichen Teildosen verteilt, zu veranschlagen.

Die neuen pharmazeutischen Präparate enthalten z.B. von etwa 10 % bis etwa 80 %, vorzugsweise von etwa 20 % bis etwa 60 % des Wirkstoffs,Erfindungsgemäss pharmazeutische Präparate zur enteralen bzw. parenteralen Verabreichung sind z.B. solche in Dosiseinheitsformen wie Dragées, Tabletten, Kapseln oder Suppositorien,

ferner Ampullen. Diese werden in an sich bekannter Weise, z.B. mittels konventioneller Misch-, Granulier-, Dragier-, Lösungs- oder Lyopilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Starisäure oder Salze davon, wie Magnesium- oder Calciumstearat, und/oder Polyäthylenglykol. Dragée-Kerne werden mit geeigneten, gegebenenfalls Magensaftresistenten Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lachlösungen in geeigneten organischen Lösungsmitteln oder Lösungsmittelgemischen oder, zur Herstellung von Magensaftresistenten Ueberzügen, Lösungen von geeigneten Cellulosepräparaten, wie Acetylcellulosephthalat oder Hydroxypropylmethylcellulosephthalat, verwendet. Den Talbetten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglycolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglycole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoff in Frage.

Zur parenteralen Verabreichung eigenen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren enthalten.

Als topisch anwendbare pharmazeutische Präparate kommen in erster Linie Creme, Salben, Pasten, Schäume, Tinkturen und Lösungen, in Frage, die von etwa 0,5 bis etwa 20 % des Wirkstoffs enthalten.

Creme sind Oel-in-Wasser-Emulsionen, die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl.Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Aethylenoxidaddukten davon, wie Polyglycerinfettsäureester oder Polyoxyäthylenfettalkoholäther oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyäthylenglykole, ferner Konservierungsmittel, Riechstoffe, etc.

Salben sind Wasser-in-Oel-Emulsionen, die bis zu 70 %, vorzugsweise jedoch von etwa 20 % bis etwa 50 % Wasser oder wässrige Phasen enthalten. Als Fettphase kommen in erster Linie Kohlenwasserstoffe, z.b. Vaseline, Paraffinöl und/oder Hartparafine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyäthylenglykol, sowie Konservierungsmittel, Riechstoffe etc.

Fettsalben sind wasserfrei und enthalten als Grundlage insbesondere Kohlenwasserstoffe, z.B. Paraffin, Vaseline und/oder flüssige Paraffine, ferner natürliche oder partialsynthetische Fett, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Cereme und Salben mit sekretabsorbierenden Puderbestandteilen, wie Metalloxiden, z.B. Titanoxid oder Zinkoxid, ferner Talk und/oder Aluminiumsilikate, wleche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluoräthan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyäthylen-sorbitan-fettsäureester (Tweens), und solchen mit vorwiegend liphophilen Eigenschaften, wie Sorbitanfettsäureester (Spans). Dazu kommen die üblichen Zusätze, wie Konservierungsmittel, etc.

Tinkturen und Lösungen weisen meistens eine wässerig-äthanolische Grundlage auf, der u.a. Polyalkohole, z.B. Glycerin, Glyckole, und/oder Polyäthylenglykol, als Feuchthaltemittel zur Herabsetzung der Verdunstung, und rückfettende Substanzen, wie Fettsäureester mit niedrigen Polyäthylenglycolen, d.h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel von der Emulgierung in einer der beiden Phasen gelöst; bei Verarbeitung als Suspension wird er nach der Emulgierung mit einem Teil der Grundlage vermischt und dann dem Rest der Formulierung beigegeben.

Die vorliegende Erfindung betrifft ebenfalls die Verwendung der Verbindungen der Formeln I und II und der Salze von solchen Verbindungen mit salzbildenden Eigenschaften, vorzugsweise zur Behandlung von Entzündungen, in erster Linie von entzündlichen chronischen Erkrankungen des rheumatischen Formenkreises, besonders der chronischen Arthritis.

Die nachfolgenden Beispiele illustrieren die oben beschriebene Erfindung; sie sollen jedoch diese in ihrem Umfang in keiner Weise einschränken. Temperaturen sind in Celsiusgraden, Drucke in mbar angegeben.

Beispiel 1: 5 g 4,5-Bis-phenyl-thiazolin-2-thion werden in 50 ml Hexamethylphosphorsäuretriamid gelöst, auf 5° abgekühlt und unter Rühren mit Natriumhydrid (0,89 g der 50%-igen Suspension in Mineralöl, mit Hexan entölt) versetzt. Man lässt 30 Minuten bei Raumtemperatur rühren, tropft dann langsam 3,18 g Aethyljodid hinzu, lässt 30 Minuten bei Raumtemperatur nachrühren, giesst auf 1000 ml Eiswasser und schüttelt zweimal mit Diäthyläther aus. Die Aetherphasen werden vereinigt, mit Wasser gewaschen, über Magnesiumsulfat getrocknet und zur Trockne eingedampft. Das zurückbleibende hellgelbe Oel wird in 200 ml Toluol/Hexan (1:1) gelöst und über 10 g Kieselgel filtriert. Nach Eindampfen des Eluates bleibt das 2-Aethylthio-4,5-bis-phenyl-thiazol als Oel zurück.

Das Ausgangsmaterial kann z.B. folgendermassen hergestellt werden: 22,48 g 2-Brom-1,2-bis-phenyl-äthanon werden in 90 ml 95%-igem Aethanol suspendiert und durch Erwärmen auf 45° in Lösung gebracht. Die Lösung wird auf 20° gekühlt, mit 27 g Ammoniumdithiocarbaminat versetzt und 15 Minuten zum Rückfluss erhitzt. Dann kühlt man im Eisbad ab, saugt den gebildeten Niederschlag ab, wäscht mit kaltem Aethanol nach, digeriert dreimal mit Wasser, saugt erneut ab, löst in 2n-Natronlauge, filtriert und fällt mit 2n-Salzsäure aus. Das ausgefallene 4,5-Bis-phenyl-thiazolin-2-thion wird aus Essigsäureäthylester umkristallisiert. Es schmilzt bei 224-226°.

In analoger Weise erhält man:

2-Methylthio-4,5-bis-phenyl-thiazol, Smp. 73-74°, und
2-Propylthio-4,5-bis-phenyl-thiazol, Smp. 35-36°.

Beispiel 2: Eine auf 5° gekühlte Lösung von 1,694 g Aethyldithiocarbaminat in 25 ml Aethanol wird unter Rühren mit 1,106 g Pyridin und 3,68 g 2-Brom-1,2-bis-phenyl-äthanon versetzt. Man lässt 30 Minuten bei 5° und 90 Minuten bei Raumtemperatur nachrühren, dampft

unter vermindertem Druck zur Trockne ein und verteilt den Rückstand zwischen Methylenchlorid und Wasser. Die wässrige Phase wird mit n-Salzsäure angesäuert und erneut zweimal mit Methylenchlorid ausgeschüttelt. Die organischen Phasen werden vereinigt, über Magnesiumsulfat getrocknet und unter vermindertem Druck zur Trockne eingedampft. Das zurückbleibende Oel wird an 100 g Kieselgel mit Toluol/Hexan (1:1) als Elutionsmittel chromatographisch gereinigt. Nach dem Eindampfen bleibt das 2-Aethylthio-4,5-bis-phenyl-thiazol als Oel zurück.

Beispiel 3: In einer auf 0 bis 5° gekühlten Lösung von 0,575 g Lithiumaluminiumhydrid in 25 ml Diäthyläther werden unter Rühren innerhalb von 30 Minuten 4,93 g 2-Methoxycarbonylmethylthio-4,5-bis-phenyl-thiazol zugetropft. Man lässt 1 Stunde nachrühren, fügt vorsichtig zunächst 0,6 ml und dann weitere 50 ml Wasser hinzu, säuert mit n-Salzsäure an, trennt die Aetherschicht ab und wäscht die Wasserphase mit Diäthyläther nach. Die ätherischen Lösungen werden vereinigt, über Magnesiumsulfat getrocknet und zur Trockne eingedampft. Der Rückstand wird in Toluol gelöst und an 90 g Kieselgel chromatographisch gereinigt. Die mit Toluol/Essigsäureäthylester eluierten Fraktionen werden vereinigt und unter vermindertem Druck eingedampft. Man erhält das 2-(2-Hydroxyäthylthio)-4,5-bis-phenyl-thiazol vom Smp. 77-79°.

Das Ausgangsmaterial kann z.B. folgendermassen hergestellt werden: 6 g 4,5-Bis-phenyl-thiazolin-2-thion werden in 50 ml Hexamethylphosphorsäuretriamid gelöst, auf 5° abgekühlt und unter Rühren mit Natriumhydrid (0,89 g der 50%-igen Suspension in Mineralöl, mit Hexan entölt) versetzt. Man lässt 30 Minuten bei Raumtemperatur rühren, tropft langsam 3,57 g Bromessigsäuremethylester hinzu, lässt 1 Stunde bei Raumtemperatur nachrühren, giesst in 500 ml Eiswasser, schüttelt zweimal mit je 300 ml Essigsäureäthylester aus, wäscht mit Wasser,

trocknet über Magnesiumsulfat und dampft unter vermindertem Druck zur Trockne ein. Das zurückbleibende Oel wird in Toluol gelöst und an 50 g Kieselgel chromatographisch gereinigt. Das Eluat mit Toluol/Essigsäureäthylester (95:5) wird unter vermindertem Druck eingedampft. Man erhält 2-Methoxycarbonylmethyl-4,5-bis-phenyl-thiazol vom Smp. 65-67°.

Beispiel 4: In analoger Weise wie in Beispiel 1 beschrieben kann man ferner

2-Methylthio-4,5-bis-(p-chlorphenyl)-thiazol, Smp. 94-95°,
2-Aethylthio-4,5-bis(p-chlorphenyl)-thiazol, Smp. 79-81°,
2-Propylthio-4,5-bis-(p-chlorphenyl)-thiazol, Smp. 63-64°, und
2-(2-Hydroxyäthylthio)-4,5-bis-(p-chlorphenyl)-thiazol, Smp. 122-124°,

herstellen. Das als Ausgangsmaterial dienende 4,5-Bis-(p-chlorphenyl)-thaizolin-2-thion kann z.B. durch Umsetzung von 2-Brom-1,2-bis-(p-chlorphenyl)-äthanon mit Ammoniumdithiocarbaminat in analoger Weise wie in Beispiel 1 beschrieben hergestellt werden. Es schmilzt bei 241-243°.

Beispiel 5: Eine auf 5° gekühlten Lösung von 6 g 5-Phenyl-4-(3-pyridyl)-thiazolin-2-thion in 40 ml Hexamethylphosphorsäuretriamid wird unter Rühren mit Natriumhydrid (1,06 g der 50%igen Suspension in Mineralöl, entölt mit Hexan) versetzt. Man erhöht die Innentemperatur auf 10°, tropft langsam 3,15 g Methyljodid hinzu, lässt 15 Minuten bei Raumtemperatur nachrühren und giesst in 500 ml Eiswasser. Das ausgeschiedene Oel kristallisiert nach einigem Stehenlassen aus. Man nimmt in Essigsäureäthylester auf und schüttelt die Wasserphase zweimal mit je 100 ml Essigsäureäthylester nach. Die organischen Phasen werden zweimal mit je 200 ml Wasser gewaschen, vereinigt, über Magnesiumsulfat getrocknet und unter vermindertem Druck eingedampft. Das zurückbleibende Oel wird mit 30 ml Diäthyl-

äther verrieben, worauf Kristallisation erfolgt. Das kristalline 2-Methylthio-5-phenyl-4-(3-pyridyl)-thiazol wird abgesaugt. Es schmilzt bei 95-95°.

Das Ausgangsmaterial kann beispielsweise folgendermassen hergestellt werden.

120 g 2-Phenyl-1-(3-pyridyl)-äthanonhydrobromid werden in 1000 ml Essigsäure auf 75° erwärmt. Dann wird das Heizbad entfernt und unter Rühren eine Lösung von 75,8 g Brom in 30 ml Essigsäure in Portionen von je 5 ml hinzugegeben. Man lässt unter Nachrühren allmählich auf 60° abkühlen, kühlt dann mittels eines Eisbades auf 20° ab, saugt ab, wäscht zunächst einmal mit 100 ml kalter Essigsäure und dann zweimal mit je 200 ml Diäthyläther und trocknet unter vermindertem Druck. Man erhält das 2-Brom-2-phenyl-1-(3-pyridyl)-äthanon-hydrobromid vom Smp. 216-219°.

42,0 g desselben werden unter Rühren in 500 ml Methanol (95%ig) gelöst, auf 10° abgekühlt und innerhalb von 5 Minuten portionenweise mit 14,93 g Ammoniumdithiocarbamat versetzt. Man kühlt noch 5 Minuten, entfernt das Eisbad, gibt nach 45 Minuten erneut 14,93 g Ammoniumdithiocarbaminat hinzu, erhitzt dann 90 Minuten zum Sieden und dampft zur Trockne ein. Der Eindampfrückstand wird mit 200 ml Wasser und 65 ml 2n-Natronlauge digeriert, abgesaugt und mit wenig Wasser gewaschen. Man löst in 100 ml 2n-Natronlauge, filtriert und säuert mit 2n-Salzsäure auf pH 5 an. Die ausgefallenen Kristalle werden abgesaugt zweimal mit wenig Eiswasser gewaschen, trockengesaugt und dann zweimal mit je 30 ml Aceton und dann zweimal mit je 50 ml Diäthyläther gründlich gewaschen. Man erhält das 5-Phenyl-4-(3-pyridyl)-thiazolin-2-thion vom Smp. 229-232°.

Beispiel 6: Eine auf 5° gekühlte Lösung von 6 g 5-Phenyl-4-(3-pyridyl)-thiazolin-2-thion in 40 ml Hexamethylphosphorsäuretriamid wird unter Rühren mit Natriumhydrid (1,06 g der 50%igen Suspension in Mineralöl, entölt mit Hexan) versetzt. Man fügt tropfenweise 3,46 g Aethyljodid hinzu, lässt 15 Minuten bei Raumtemperatur nachrühren und giesst in 500 ml Eiswasser. Das ausgefallene Oel wird in 100 ml Essigsäureäthylester aufgenommen. Die erhaltene Lösung wird sechsmal mit je 50 ml 2n-Ammoniumchloridlösung ausgezogen. Die Auszüge werden vereinigt, mit Natronlaug auf pH = 10 gebracht und zweimal mit je 100 ml Essigsäureäthylester ausgezogen. Die organischen Phasen werden vereinigt, über Magnesiumsulfat getrocknet und unter vermindertem Druck zur Trockne eingedampft. Der Eindampfrückstand wird in 30 ml Toluol gelöst und an 30 g Kieselgel chromatographisch gereinigt. Aus dem Eluat mit Toluol/Essigsäureäthylester (10:1) kristallisiert nach einigem Stehenlassen in der Kältekammer das 2-Aethylthio-5-phenyl-4-(3-pyridyl)-thiazol vom Smp. 41-42°.

Beispiel 7: Eine auf 5° gekühlte Lösung von 7 g 5-Phenyl-4-(3-pyridyl)-thiazolin-2-thion in 40 ml Hexamethylphosphorsäuretriamid wird unter Rühren mit Natriumhydrid (1,24 g der 50%-igen Suspension in Mineralöl, mit Hexan entölt) versetzt. Man erhöht die Innentemperatur auf 10°, tropft 4,45 g 2-Jodäthanol hinzu, lässt 15 Minuten bei Raumtemperatur nachrühren und giesst auf 500 ml Eiswasser und schüttelt zweimal mit je 100 ml Essigsäureäthylester aus. Die organische Phase werden vereinigt, zweimal mit je 100 ml 0,1n-Natronlauge gewaschen, über Magnesiumsulfat getrocknet und unter vermindertem Druck zur Trockne eingedampft. Der Eindampfrückstand wird durch Verreiben mit 30 ml Diäthyläther zur Kristallisation gebracht. Man lässt 1 Stunde rühren und saugt das kristalline 2-(2-Hydroxyäthylthio)-5-phenyl-4-(3-pyridyl)-thiazol vom Smp. 109-110° ab.

Beispiel 8: 10 g 4,5-Bis-(p-methoxyphenyl)-thiazolin-2-thion werden in 50 ml Hexamethylphosphorsäuretriamid gelöst, auf 5° abgekühlt und unter Rühren mit Natriumhydrid (1,45 g der 50%-igen Suspension in Mineralöl, mit Hexan entölt) versetzt. Man lässt 30 Minuten bei Raumtemperatur nachrühren, tropft 5,22 g 2-Jodäthanol hinzu, lässt 15 Minuten bei Raumtemperatur nachrühren und giesst auf ein Gemisch von 30 ml 2-n-Salzsäure und 400 ml Eiswasser. Das ausgeschiedene Oel kristallisiert nach einigem Stehenlassen und wird in 200 ml Methylenchlorid aufgenommen. Die Lösung wird über Magneisumsulfat getrocknet und zur Trockne eingedampft. Zur Kristallisation wird mit Diäthyläther verrieben. Man erhält das 2-(2-Hydroxyäthylthio)-4,5-bis-(p-methoxyphenyl)-thiazol vom Smp. 83-84°.

Das Ausgangsmaterial kann z.B. folgendermassen hergestellt werden:

4,92 g Ammoniumthiocarbaminat werden in 70 ml 96%-igem Aethanol suspendiert. Man kühlt auf 5° ab und gibt unter Rühren innerhalb von 10 Minuten portionsweise 15 g 2-Brom-1,2-bis-(p-methoxyphenyl)-äthanon hinzu, wobei man die Innentemperatur durch Kühlen auf etwa 10° hält. Man lässt 1 Stunde bei 10° nachrühren, gibt nochmals 2,4 g Ammoniumdithiocarbaminat hinzu und lässt nochmals 3 Stunden bei Raumtemperatur rühren. Dann giesst man in 500 ml Eiswasser, saugt das Rohprodukt ab und wäscht mit Eiswasser nach..Zur Reinigung wird das Rohprodukt in 50 ml Aethanol suspendiert und zum Rückfluss erhitzt, wobei zunächst Auflösung und nach 90 Minuten erneute Ausfällung erfolgt. Man lässt abkühlen, saugt ab und wäscht mit kaltem Aethanol nach. Man erhält das 4,5-Bis-(p-methoxyphenyl)-thiazolin-2-thion vom Smp. 226-230°.

In analoger Weise kann man ferner
2-Propylthio-4,5-bis-(p-methoxyphenyl)-thiazol, Smp. 58-60°, herstellen.

Beispiel 9: In analoger Weise wie in Beispiel 3 beschrieben erhält man durch Umsetzung von 4,5-Bis-(p-methoxyphenyl)-thiazolin-2-thion mit Bromessigsäuremethylester und anschliessende Reduktion mit Lithiumaluminiumhydrid das

2-(2-Hydroxyäthylthio)-4,5-bis-(p-methoxyphenyl)-thiazol, Smp. 82-84°.

Beispiel 10: Man löst unter Stickstoff 0,217 g Aethylmercaptan in 10 ml Hexamethylphosphorsäuretriamid, kühlt auf 10° ab und gibt unter Rühren portionsweise 170 mg 50%-ige Natriumhydridsuspension (in Mineralöl, mit Hexan entölt) hinzu. Man lässt 15 Minuten bei Raumtemperatur nachrühren, fügt 1,0 g 2-Chlor-4,5-bis-(p-methoxyphenyl)-thiazol hinzu und lässt 1 Stunde bei Raumtemperatur rühren. Dann giesst man auf 300 ml 10%-ige Kochsalzlösung, digeriert bis zur Kristallisation, saugt ab, wäscht zweimal mit Wasser nach und saugt auf der Nutsche trocken. Das Rohprodukt wird aus Methanol umkristallisiert. Man erhält 2-Aethylthio-4,5-bis-(p-methoxyphenyl)-thiazol vom Smp. 73-75°.

Das als Ausgangsmaterial dienende 2-Chlor-4,5-bis(p-methoxyphenyl)-thiazol kann beispielsweise folgendermassen hergestellt werden.

100 g 2-Brom-1,2-bis-(p-methoxyphenyl)-äthanon werden mit 700 ml 96%-igem Aethanol und 25,4 g Natriumthiocyanat vermischt und 2 Stunden auf 45° erwärmt. Dann dampft man unter vermindertem Druck zur Trockne ein, gibt 500 ml Eiswasser hinzu, saugt ab und wäscht zweimal mit Eiswasser nach. Das Rohprodukt wird in 1500 ml Methylenchlorid aufgenommen, vom restlichen Wasser abgetrennt und über Magnesiumsulfat getrocknet. Dann engt man auf etwa 200 ml ein, lässt abkühlen, saugt erneut ab, wäscht mit wenig Methylenchlorid nach und trocknet unter vermindertem Druck. Man erhält 1,2-Bis-(p-methoxyphenyl)-2-isothiocyano-äthanon vom Smp. 155°.

10 g desselben werden in 150 ml Diäthyläther suspendiert und unter Rühren auf 15 - 20° abgekühlt. Dann leitet man so langsam gasförmigen Chlorwasserstoff ein, dass die Reaktionstemperatur 20° nicht übersteigt. Nach etwa 45 Minuten erhält man eine klare, gelbliche Lösung. Man hält bei 15° und leitet weitere 7 Stunden etwas Chlorwasserstoff ein, lässt über Nacht bei 15° stehen, giesst auf 300 ml Eis, destilliert den Diäthyläther ab, saugt den kristallinen Niederschlag ab, wäscht zweimal mit Wasser nach und saugt auf der Nutsche trocken. Das Rohprodukt wird aus 100 ml Hexan umkristallisiert. Man erhält 2-Chlor-4,5-bis-(p-methoxyphenyl)-thiazol vom Smp. 92-94°.

Beispiel 11: 2,2 g 4,5-Bis(p-methoxyphenyl)-thiazol werden in 10 ml Tetrahydrofuran gelöst und auf -60° abgekühlt. Dann tropft man innerhalb von 5 Minuten 5 ml einer 1,6-molaren Lösung von Butyllithium in Hexan hinzu, rührt 10 Minuten bei -60° nach entfernt das Kältebad, gibt langsam 1,8 g Dimethyldisulfid, gelöst in 20 ml Tetrahydrofuran hinzu un lässt unter Rühren auf Raumtemperatur erwärmen, gibt 50 ml konzentrierter Ammonchloridlösung hinzu, trennt die organische Phase ab, dampft zur Trockne ein, nimmt in Methylenchlorid auf, wäscht mit Wasser, trocknet über Magnesiumsulfat und dampft erneut zur Trockne ein. Man erhält 2-Methylthio-4,5-bis-(p-methoxyphenyl)-thiazol vom Smp. 102-104°.

Das Ausgangsmaterial kann z.B. folgendermassen hergestellt werden.

Eine Lösung von 7 g 2-Brom-1,2-bis-(p-methoxyphenyl)-äthanon in 20 ml Benzol wird mit 7 g Phosphorpentasulfid in 20 ml Formamid versetzt und 1 Stunde zum Sieden erhitzt. Man lässt abkühlen, neutralisiert mit 10%-iger Natronlauge und schüttelt mit Chloroform aus. Die organische Phase wird abgetrennt, mit Wasser gewaschen, über Magnesiumsulfat getrocknet und eingedampft. Man erhält 4,5-Bis-(p-methoxyphenyl)-thiazol vom Smp. 98-99°, das ohne weitere Reinigung eingesetzt werden kann.

Beispiel 12: 100 g 4,3-Bis-(p-methoxyphenyl)-thiazolin-2-thion werden unter Rühren in 320 ml 2-n Natronlauge gelöst. Dann gibt man 45 ml 2-Bromäthanol hinzu, lässt 15 Minuten bei Raumtemperatur rühren, gibt 500 ml Methylenchlorid hinzu, filtriert über Diatomerenerde, trennt die organische Schicht ab, und wäscht mit 200 ml 2n-Natronlauge aus. Die vereinigten Wasserphasen werden zweimal mit Methylenchlorid nachextrahiert. Die organischen Phasen werden vereinigt, über Magnesiumsulfat getrocknet und unter vermindertem Druck eingedampft. Das zurückbleibende Oel wird in 700 ml heissem Diäthyläther gelöst, 1 Stunde gerührt und bei 10° zur Kristallisation gestellt. Man saugt ab, wäscht zweimal mit je 150 ml eiskaltem Diäthyläther nach und trocknet unter vermindertem Druck. Man erhält das 2-(2-Hydroxyäthylthio)-4,5-bis-(p-methoxyphenyl)-thiazol vom Smp. 82-83°.

Beispiel 13: 5,0 g 4,5-Bis-(p-methoxyphenyl)-thiazolin-2-thion werden in 30 ml 2n-Natronlauge gelöst und mit 50 g Eis und 100 ml Methylenchlorid versetzt. Zu der heftig gerührten Lösung gibt man 2,65 g Brom, gelöst in 5 ml Methylenchlorid hinzu und lässt 5 Minuten nachrühren. Die organische Schicht wird abgetrennt und mit 100 ml Wasser gewaschen. Die vereinigten wässrigen Pahsen werden zweimal mit je 50 ml Methylenchlorid ausgewaschen. Die organischen Phasen werden vereinigt, über Magnesiumsulfat getrocknet unter vermindertem Druck eingedampft. Das zurückbleibende Oel wird aus Diäthyläther kristallisiert, abgesaugt, mit Diäthyläther nachgewaschen unter vermindertem Druck getrocknet und aus Diäthyläther umkristallisiert. Man erhält 2,2-Bis-[4,5-bis-(p-methoxyphenyl)-thiazolyl]-disulfid vom 155-158°.

Beispiel14 : 5,0 g 4,5-Bis-(p-methoxyphenyl)-thiazolyl-(2)-thioglykolsäure (Smp. 143-145°) werden unter Stickstoff mit 35 ml Acetanhydrid versetzt und langsam zum Rückfluss erhitzt. Man lässt 5 Minuten sieden und kühlt sofort auf 5° ab. Die ausgeschiedenen

Kristalle werden abgesaugt, mit Aether gewaschen und unter vermindertem Druck getrocknet. Man erhält 5,6-Bis-(p-methoxyphenyl)-thiazolo-[2,3-b]thiazolium-3-oxylat der Formel

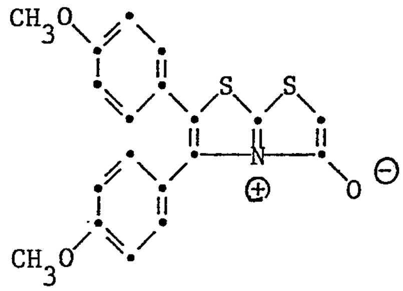

vom Smp. 165-168°. In analoger Weise erhält man, ausgehend von 4,5-Bis-phenyl-thiazolyl-(2)-thioglykolsäure bzw. 4,5-Bis-(p-chlorphenyl)-thiazolyl-(2)-thioglykolsäure das 5,6-Bis-phenyl-thiazolo[2,3-b]-thiazolium-3-oxylat bzw. 5,6-Bis-(p-chlorphenyl)-thiazolo[2,3-b]-thiazolium-3-oxylat.

Beispiel 15: 0,1 g 5,6-Bis-(p-methoxyphenyl)-thiazolo[2,3-b]-thiazolium-3-oxylat werden in 5 ml Pyridin suspendiert und unter Rühren in einem vorgeheizten Oelbad 5 Minuten zum Rückfluss erhitzt. Man giesst in 100 ml Eiswasser und digeriert bis zur Kristallisation des zunächst ölig ausgeschiedenen Rohprodukts. Man nutscht ab, saugt auf der Nutsche trocken, läst in 50 ml Methylenchlorid, trocknet über Magnesiumsulfat, verrührt mit 1 g Kieselgel, saugt ab, wäscht zweimal mit Methylenchlorid und dampft die vereinigten Methylenchloridlösungen zur Trockne ein. Das Rohprodukt wird aus 10 ml Methanol kristallisiert. Man saugt ab und trocknet unter vermindertem Druck bis zur Gewichtskonstanz. Das 1,3-Bis-[4,5-bis-(p-methoxyphenyl)-thiazolyl-(2)-thio]-propan-2-on schmilzt bei 130-133°.

Beispiel 16: 20,0 g 4,5-Bis-(p-methoxyphenyl)-thiazolin-2-thion werden bei 35° in 100 ml Hexamethylphosphorsäuretriamid in Lösung gebracht. Man lässt auf 20° abkühlen, gibt zunächst 11,2 g 2-Bromacetaldehyd-dimethylacetal und dann portionsweise 2,9 g 50%-ige Natriumhydrid-Suspension (in Mineralöl, mit Hexan entölt) hinzu, wobei die Reaktionstemperatur durch Kühlen auf 25-30° gehalten wird. Man lässt 1 Stunde bei Raumtemperatur nachrühren, giesst auf Eis, bringt das ausgeschiedene Oel mit Diäthyläther in Lösung und trennt die organische Phase ab. Die Wasserphase wird nochmals mit 200 ml Diäthyläther ausgeschüttelt. Die organischen Phasen werden vereinigt, mit 200 ml 2n-Natronlauge gewaschen, über Magnesiumsulfat getrocknet und eingedampft. Das ölige Rohprodukt wird aus Hexan kristallisiert und mit Methanol angeschlämmt. Man erhält 2-[4,5-Bis-(p-methoxyphenyl)-thiazolyl-(2)-thio]-acetaldehyd-dimethylacetal vom Smp. 60-61°.

Beispiel 17: 7,0 g 2-[4,5-Bis-(p-methoxyphenyl)-thiazolyl-(2)-thio]-acetaldehyd-dimethylacetal werden in 50 ml Essigsäure, 25 ml Schwefelsäure und 25 ml Wasser vermischt, 30 Minuten heftig gerührt und dann in 1000 ml Eiswasser eingerührt. Das ausgeschiedene Oel wird in 200 ml Essigsäureäthylester aufgenommen. Man schüttelt mit weiteren 200 ml Essigsäureäthylester nach, wäscht die vereinigten organischen Phasen mit Wasser und dann zweimal mit kalter Natriumhydrogencarbonatlösung, trocknet über Magnesiumsulfat und dampft zur Trockne ein. Das Rohprodukt wird mit Diäthyläther kristallisiert. Man erhält 2-[4,5-Bis-(p-methoxyphenyl)-thiazolyl-(2)-thio]-acetaldehyd vom Smp. 75-76°.

Beispiel 18: 0,10 g 2-[4,5-Bis-(p-methoxyphenyl)-thiazolyl-(2)-thio]-acetaldehyd werden in 3 ml Methanol gelöst, mit 0,01 g Natriumborhydrid versetzt und 15 Minuten gerührt. Man dampft unter vermindertem Druck zur Trockne ein, digeriert mit 20 ml Eiswasser. Nach einiger Zeit erfolgt Kristallisation. Nach 2 Stunden wird abgesaugt, mit Wasser gewaschen und trocken gesaugt. Man erhält 2-[2-Hydroxyäthylthio]-4,5-bis-(p-methoxyphenyl)-thiazol vom Smp. 82-83°.

Beispiel 19: 5,0 g 4,5-Bis-(p-methoxyphenyl)-thiazolin-2-thion werden unter Stickstoff bei 45° in 50 ml Hexamethylphosphorsäuretriamid gelöst, portionsweise mit 0,73 g 50%-iger Natriumhydrid-Suspension (in Mineralöl, mit Hexan entölt) versetzt und 1 Stunde bei Raumtemperatur gerührt. Dann werden 2,22 g 3-Brompropanol zugetropft. Man lässt 90 Minuten bei Raumtemperatur nachrühren, giesst in ein Gemisch von 400 ml Eiswasser und 20 ml 2n-Salzsäure, rührt zweimal mit je 100 ml Essigsäureäthylester aus, wäscht zweimal mit je 100 ml Wasser, trocknet über Natriumsulfat und dampft unter vermindertem Druck ein. Das ölige Rohprodukt wird in 30 ml Toluol gelöst und an einer mit 30 g Kieselgel gefüllten Säule mit Toluol als Laufmittel chromatographiert. Die Fraktion 800 - 1200 ml werden vereinigt, zur Trockne eingedampft und 3 Stunden unter 0,01 mbar getrocknet. Man erhält analysenreines 2-(3-Hydroxypropylthio)-4,5-bis-(p-methoxyphenyl)-thiazol in Form eines hellgelben Oeles, vom $R_f$-Wert=0,25 (Kieselgel-Dünnschicht/Toluol + Essigsäureäthylester 3:1).

Beispiel 20: 5,0 g 2-(2-Hydroxyäthylthio)-4,5-bis-(p-methoxyphenyl)-thiazol werden in 100 ml Methylenchlorid gelöst und unter Rühren protionsweise mit 2,56 g m-Chlorperbenzoesäure versetzt. Man lässt 15 Minuten nachrühren und schüttelt sodann zweimal mit verdünnter Sodalösung aus. Die Wasserphase wird mit Methylenchlorid nachgewaschen. Die organischen Phasen werden vereinigt, über Magnesiumsulfat getrocknet und zur Trockne eingedampft. Das Rohprodukt wird in 10 ml Essigsäureäthylester gelöst, mit 30 ml Diäthyläther ausgefällt, abgesaugt, mit Diäthyläther gewaschen, unter vermindertem Druck getrocknet, zur weiteren Reinigung mit 50 ml Diäthyläther angeschlämmt, erneut abfiltriert und unter 0,01 mbar bei 100° bis zur Gewichtskonstanz getrocknet. Man erhält das 2-(2-Hydroxyäthansulfinyl)-4,5-bis-(p-methoxyphenyl)-thiazol in Form eines hellgelben Oels vom $R_f$-Wert = 0,12 (Kieselgel-Dünnschicht/Toluol + Essigsäureäthylester 1:1); IR-Spektrum: $V_{SO}$ = 1060 $cm^{-1}$ (3 % in $CH_2Cl_2$).

Beispiel 21: 6,0 g 2-(2-Hydroxyäthylthio)-4,5-bis-(p-methoxyphenyl)-thiazol werden in 100 ml Methylenchlorid gelöst und portionsweise mit 6,09 g m-Chlorperbenzoesäure versetzt.Man lässt 10 Minuten bei Raumtemperatur rühren erwärmt dann 1 Stunde zum Rückfluss, bis Kaliumjodidpapier nicht mehr gefärbt wird. Man lässt abkühlen, wäscht mit eiskalter n-Natronlauge, zieht diese mit Methylendichlorid aus, vereinigt die organischen Phasen, trocknet über Magnesiumsulfat und dampft zur Trockne ein. Das Rohprodukt wird in 20 ml Toluol/Essigsäureäthylester gelöst und an 60 g Kieselgel chromatographisch gereinigt. Man fängt die ersten 150 ml Eluat auf, dampft zur Trockne ein und trocknet bei 100° unter 0,018 mbar bis zur Gewichtskonstanz. Man erhält analysenreines 2-(2-Hydroxyäthansulfonyl)-4,5-bis-(p-methoxyphenyl)-thiazol als hellgelbes Oel vom $R_f$-Wert = 0,21 (Kieselgel-Dünnschicht/Toluol + Essigsäureäthylester 3:1); JR-Spektrum: $v_{SO_2}$ = 1170 $cm^{-1}$, 1225 $cm^{-1}$ (3 % in $CH_2Cl_2$).

Beispiel 22: 0,23 g Natrium werden unter Stickstoff in 30 ml Aethanol gelöst. Man lässt auf Raumtemperatur abkühlen, fügt unter Rühren 3 g 4,5-Bis-(p-methoxyphenyl)-thiazolin-2-thion hinzu und leitet nach vollständiger Auflösung Aethylenoxid ein, wobei man die Reaktionstemperatur durch gelindes Kühlen auf 25-30° hält. Nach 30-minütigem Einleiten sollte dünnschichtchromatographisch kein Thion mehr nachweisbar sein. Alsdann fügt man 10 ml 2n-Ammonchlorid hinzu, destilliert unter verminderten Druck das Aethanol ab und schüttelt den Eindampfrückstand mit einem Gemisch von je 50 ml Wasser und Methylenchlorid. Man trennt die organische Phase ab, schüttelt mit Methylenchlorid nach, trocknet über Magnesiumsulfat und dampft zur Trockne ein. Das ölige Rohprodukt wird durch Anreiben mit 20 ml Diäthyläther zur Kristallisation gebracht und unter vermindertem Druck getrocknet. Man erhält das 2-(2-Hydroxyäthylthio)-4,5-bis-(p-methoxyphenyl)-thiazol vom Smp. 82-83°.

Beispiel 23: 15,0 g 4,5-Bis-(p-methoxyphenyl)-thiazolin-2-thion werden in 50 ml 2n-Natronlauge gelöst, unter Stickstoff mit 15,4 g Bromacetaldehyd-dimethylacetal (2,2-Dimethoxy-1-bromäthan) versetzt und unter Rühren auf 80° erwärmt. Man lässt 2,5 Stunden bei 80° rühren, kühlt auf Raumtemperatur ab, schüttelt mit 100 ml Toluol aus, trennt die organische Schicht ab und schüttelt die Wasserphase mit 50 ml Toluol nach. Die organischen Phasen werden vereinigt, zweimal mit je 30 ml 2n-Natronlauge und anschliessend mit 30 ml 2n-Salzsäure gewaschen, über Magnesiumsulfat getrocknet, unter vermindertem Druck eingedampft und sorgfältig getrocknet. Das Rohprodukt wird aus Hexan/Methanol (50 ml + 5 ml) kristallisiert. Man erhält 2-[4,5-Bis-(p-methoxyphenyl)-thiazolyl-(2)-thio]-acetaldehyd-dimethylacetal vom Smp. 60-61°.

Beispiel 24: 7,0 g 2-[4,5-Bis-(p-methoxyphenyl)-thiazolyl-(2)-thio]-acetaldehyd-dimethylacetal werden in 200 ml Methylenchlorid gelöst, auf 5° abgekühlt, mit 3,53 g m-Chlorbenzoesäure (90%ig) versetzt und 15 Minuten bei 5° gerührt. Man fügt 100 g Eis und 50 ml 2n-Natronlauge hinzu; trennt die organische Phase ab, wäscht mit 50 ml 2n-Natronlauge nach und rührt die Wasserphase zweimal mit je 50 ml Methylenchlorid aus. Die organischen Phasen werden vereinigt, über Magnesiumsulfat getrocknet und zur Trockne eingedampft. Das Rohprodukt wird an 110 g Kieselgel chromatographisch gereinigt, wobei mit Toluol/Essigester (9:1) 2-[4,5-Bis-o-methoxyphenyl)-thiazolyl-(2)-sulfonyl]-acetaldehyd-dimethylacetal und mit Toluol/Essigester (1:1) 2-[4,5-Bis-(p-methoxyphenyl)-thiazolyl-(2)-sulfinyl]-acetaldehyd-dimethylacetal als Oele eluiert werden.

Beispiel 25: 5,75 g 2-[4,5-Bis-(p-methoxyphenyl)-thiazolyl-(2)-sulfinyl]-acetaldehyd-dimethylacetal werden in 5 ml Essigsäure gelöst. Dann werden unter Rühren innerhalb von 5 Minuten 30 ml eines Gemisches von 2 Teilen Essigsäure, 1 Teil Schwefelsäure und 1 Teil Wasser zugetropft. Man lässt 30 Minuten bei Raumtemperatur rühren, verdünnt mit

100 ml Methylenchlorid und giesst in 200 ml Eiswasser. Die organische Schicht wird abgetrennt und mit 200 ml Eiswasser gewaschen. Die Wasserphase wird noch dreimal mit je 50 ml Methylenchlorid ausgeschüttelt. Die organischen Phasen werden vereinigt, über Magnesiumsulfat getrocknet und zur Trockne eingedampft. Das Rohprodukt wird in 200 ml Methylenchlorid gelöst und mit 5 g Kieselgel verrührt. Man filtriert ab, dampft zur Trockne ein und trocknet unter vermindertem Druck. Man erhält den 2-[4,5-Bis-(p-methoxyphenyl)-thiazolyl-(2)-sulfinyl]-acetaldehyd in Form eines hochviskosen Oels, IR-Spektrum: $v_{c=o} = 1720\ cm^{-1}$, $v_{s=o} = 1080\ cm^{-1}$ ($CCl_4$).

Beispiel 26: 8,0 g 2-[4,5-Bis-(p-methoxyphenyl)-thiazolyl-(2)-thio]-acetaldehyd-dimethylacetal werden in 200 ml Methylenchlorid gelöst, portionsweise mit 8,08 g m-Chlorperbenzoesäure versetzt und 2 Stunden bei Raumtemperatur gerührt. Man schüttelt zweimal mit je 100 ml kalter n-Natronlauge aus, wäscht die wässrigen Auszüge zweimal mit je 50 ml Methylenchlorid, vereinigt die organischen Phasen, trocknet diese über Magnesiumsulfat und dampft zur Trockne ein. Der Eindampfrückstand wird in 200 ml Toluol/Essigester (10:1) gelöst, mit 10 g Kieselgel verrührt, abfiltriert und erneut eingedampft. Man erhält 2-[4,5-Bis-(p-methoxyphenyl)-thiazolyl-(2)-sulfonyl]-acetaldehyd-dimethylacetal in Form eines gelblichen Oels.

Beispiel 27: 7,15 g 2-[4,5-Bis-(p-methoxyphenyl)-thiazolyl-(2)-sulfonyl]-acetaldehyd-dimethylacetal werden wie in Beispiel 25 beschrieben zu 2-[4,5-Bis-(p-methoxyphenyl)-thiazolyl-(2)-sulfonyl]-acetaldehyd (hochviskoses Oel) hydrolysiert, IR-Spektrum: $v_{c=o} = 1720\ cm^{-1}$, $v_{s=o} = 1145\ cm^{-1} + 1325\ cm^{-1}$ ($CCl_4$).

Beispiel 28: 4,0 g 2-[4,5-Bis-(p-methoxyphenyl)-thiazol-(2)-thio]-2-formyl-essigsäureäthylester werden in 50 ml Essigsäure gelöst, mit 10 ml 43%iger Bromwasserstoffsäure versetzt und 4 Stunden auf 80° erhitzt. Man dampft unter vermindertem Druck zur Trockne ein, löst in 200 ml Essigester, wäscht zweimal mit je 50 ml gesättigter Natriumhydrogencarbonatlösung und mit Wasser, trocknet über Magnesiumsulfat

und dampft zur Trockne ein. Das Rohprodukt wird aus Hexan/Diäthyläther (55 ml + 10 ml) umkristallisiert. Man erhält 2-[4,5-Bis-(p-methoxyphenyl)-thiazolyl-(2)-thio]-acetaldehyd vom Smp. 75-76°.

Das Ausgangsmaterial kann z.B. folgendermassen hergestellt werden:

3,3 g 4,5-Bis-(p-methoxyphenyl)-thiazolin-2-thion werden in 50 ml Hexamethylphosphorsäuretriamid gelöst, auf 5° abgekühlt und unter Stickstoff mit Natriumhydrid (0,5 g der 50%igen Suspension in Mineralöl, mit Hexan entölt) in das Natriumsalz überführt. Man lässt auf Raumtemperatur erwärmen, tropft 2,0 g 2-Brom-formylessigsäureäthylester hinzu, lässt 1 Stunde bei 25° rühren, giesst auf 750 ml Eiswasser und nimmt in Diäthyläther auf. Die Aetherphase wird mit Wasser gewaschen, über Magnesiumsulfat getrocknet und zur Trockne eingedampft. Man erhält 2-[4,5-Bis-(p-methoxyphenyl)-thiazol-(2)-thio]-2-formylessigsäureäthylester, der ohne weitere Reinigung eingesetzt werden kann.

Beispiel 29: 1,49 g Kaliumpyrosulfit werden in 5 ml Wasser gelöst. Dann fügt man eine Lösung von 2,5 g 2-[4,5-Bis-(p-methoxyphenyl)-thiazolyl-(2)-thio]-acetaldehyd in 40 ml Aethanol hinzu, lässt 30 Minuten bei Raumtemperatur und 15 Minuten bei 5-10° rühren, saugt ab, wäscht zweimal mit kalten Aethanol und zweimal mit Diäthyläther und trocknet unter vermindertem Druck. Man erhält das Kaliumsalz der 2-[4,5-Bis-(p-methoxyphenyl)-thiazolyl-(2)-thio]-1-hydroxy-äthansulfonsäure. Es schmilzt bei 165° (Zers.).

Beispiel 30: 3,28 g 2,2-Bis-[4,5-bis-(p-methoxyphenyl)-thiazolyl]-disulfid in 50 ml Tetrahydrofuran werden tropfenweise zu einer Propylmagnesiumbromidlösung (bereitet aus 0,24 g mit Jod aktiviertem Magnesium und 0,9 ml Propylbromid in 10 ml Tetrahydrofuran) hinzugefügt. Man erwärmt 90 Minuten zum Rückfluss, lässt abkühlen, tropft 100 ml gesättigte Ammonchloridlösung hinzu und schüttelt

dreimal mit je 100 ml Diäthyläther aus. Die Aetherlösungen werden vereinigt, dreimal mit je 100 ml Wasser gewaschen, über Magnesiumsulfat getrocknet und zur Trockne eingedampft. Das Rohprodukt wird an 15 g Kieselgel unter Verwendung von Toluol/Essigester (10:1) als Elutionsmittel gereinigt und aus Methanol umkristallisiert. Man erhält das 2-Propylthio-4,5-bis-(p-methoxyphenyl)- thiazol vom Smp, 58-60°.

Beispiel 31: 5,0 g 2-(2-Hydroxyäthylthio)-4,5-bis-(p-methoxyphenyl)-thiazol werden in 50 ml Acetanhydrid suspendiert, unter Rühren zum Sieden erhitzt und 15 Minuten am Rückfluss gekocht. Man dampft unter vermindertem Druck zur Trockne ein, versetzt zweimal mit je 30 ml Xylol, dampft jeweils erneut ein, um Reste von Acetanhydrid zu entfernen und trocknet unter vermindertem Druck. Man erhält das 2-(2-Acetoxyäthylthio)-4,5-bis-(p-methoxyphenyl)-thiazol in Form eines Oeles vom $R_f$ = 0,33 (Kieselgel Toluol/Essigester 10+1), IR-Spektrum: $v_{c=o}$ = 1730 $cm^{-1}$ ($CCl_4$).

Beispiel 32: 0,5 g 4,5-Bis-(p-methoxyphenyl)-thiazolin-2-thion werden in 2,6 ml 2n-Natronlauge unter Rühren gelöst. Man fügt 0,367 g Allylbromid hinzu, lässt 10 Minuten bei Raumtemperatur rühren, schüttelt zweimal mit je 10 ml Toluol aus, wäscht die vereinigten wässrigen Phasen zweimal mit je 5 ml 2n-Natronlauge, trocknet über Magnesiumsulfat, dampft unter vermindertem Druck ein und kristallisiert aus Hexan. Man erhält das 2-Allylthio-4,5-bis-(p-methoxyphenyl)-thiazol vom Smp. 57-58°.

Beispiel 33: 0,2 g 2-[4,5-Bis-(p-methoxyphenyl)-thiazol-(2)-thio]-acetaldehyd werden in 50 ml Toluol gelöst. Dann fügt man unter lebhaftem Rühren 1 ml Aethylenglykol und anschliessend 0,2 g p-Toluolsulfonsäure hinzu. Man erhitzt 40 Stunden am Wasserabscheider, kühlt auf 20° ab, saugt ab, kristallisiert aus Hexan/Benzol nach und trocknet unter vermindertem Druck bis zur

Gewichtskonstanz. Man erhält das 2-[4,5-Bis-(p-methoxyphenyl)-thiazolyl-(2)-thio]-acetaldehyd-äthylenacetal als viskoses Oel.

Beispiel 34: In analoger Weise wie in den Beispielen 1-33 oder in der Beschreibung beschrieben kann man ferner herstellen:

2-(1,1,2,2-Tetrafluoräthylthio)-4,5-bis-phenyl-thiazol,
2-(1,1,2,2-Tetrafluoräthylthio)-4,5-bis-(p-chlorphenyl)-thiazol,
2-(1,1,2,2-Tetrafluoräthylthio)-4,5-bis-(p-methoxyphenyl)-thiazol,
2-(3-Hydroxypropansulfinyl)-4,5-bis-(p-methoxyphenyl)-thiazol,
2-(3-Hydroxypropansulfonyl)-4,5-bis-(p-methoxyphenyl)-thiazol,
2-(2-Hydroxyäthansulfonyl)-4,5-phenyl-thiazol,
2-(2-Hydroxyäthansulfonyl)-4,5-bis-(p-chlorphenyl)-thiazol,
2-Methansulfonyl-4,5-bis-(p-methoxyphenyl)-thiazol,
2-Aethansulfonyl-4,5-bis-(p-methoxyphenyl)-thiazol,
2-(2-Hydroxyäthylthio)-4-(p-methoxyphenyl)-5-(4-pyridyl)-thiazol,
2-(2-Hydroxyäthylthio)-4-phenyl-5-(3-pyridyl)-thiazol,
2-(2-Hydroxyäthansulfonyl)-4,5-bis-(p-methoxyphenyl)-thiazol.
2-Propargylthio-4,5-bis-(p-methoxyphenyl)-thiazol,
2-Aethinylthio-4,5-bis-(p-methoxyphenyl)-thiazol,
2-(2-Methoxyäthylthio)-4,5-bis-(p-methoxyphenyl)-thiazol,
2-(2-Methylthioäthylthio)-4,5-bis-(p-methoxyphenyl)-thiazol,
3-[4,5-Bis-(p-methoxyphenyl)-thaizolyl-(2)-thio]-propionaldehyd,
3-[4,5-Bis-(p-methoxyphenyl)-thiazolyl-(2)-thio]-propionaldehyd-dimethylacetal,
2-(2,3-Dimethoxypropylthio)-4,5-bis-(p-methoxyphenyl)-thiazol,
2-(2,3-Dihydroxypropylthio)-4,5-bis-(p-methoxyphenyl)-thiazol,
2-(2,3-Isopropylidendioxypropylthio)-4,5-bis-(p-methoxyphenyl)-thiazol,
2-(2,2-Aethylendithioäthylthio)-4,5-bis-(p-methoxyphenyl)-thiazol,
2-(2-Hydroxyäthansulfinyl)-5-phenyl-4-(3-pyridyl)-thiazol,
2-(2-Hydroxyäthansulfonyl)-5-phenyl-4-(3-pyridyl-thiazol,

2-Methansulfinyl-5-phenyl-4-(3-pyridyl)-thiazol,

2-Methansulfonyl-5-phenyl-4-(3-pyridyl)-thaizol,

2-Aethansulfinyl-5-phenyl-4-(3-pyridyl)-thiazol,

2-Aethansulfonyl-5-phenyl-4-(3-pyridyl)-thiazol,

2-(2-Hydroxyäthansulfinyl)-5-phenyl-4-(3-pyridyl)-thiazol,

2-(2-Hydroxyäthansulfonyl)-5-phenyl-4-(3-pyridyl)-thiazol,

2-(1,1,2,2-Tetrafluoräthylthio)-5-phenyl-4-(2-pyridyl)-thiazol,

2-Methylthio-4-phenyl-5-(3-pyridyl)-thiazol,

2-Aethylthio-4-phenyl-5-(3-pyridyl)-thiazol,

1,3-Bis-[5-phenyl-4-(3-pyridyl)-thiazolyl-(2)-thio]-propan-2-ol,

1,3-Bis-[5-phenyl-4-(3-pyridyl)-thiazolyl-(2)-thio]-propan-2-on,

2,2'-Bis-[5-phenyl-4-(3-pyridyl)-thiazolyl]-disulfid,

2-(2-Hydroxyäthylthio)-4-phenyl-5-(2-thienyl)-thiazol,

2-Methansulfinyl-4-phenyl-5-(3-pyridyl)-thiazol,

2-Methansulfonyl-4-phenyl-5-(3-pyridyl)-thiazol,

2-Aethansulfonyl-4-phenyl-5-(3-pyridyl)-thiazol,

2-Aethansulfinyl-4-phenyl-5-(3-pyridyl)-thiazol,

2-(2-Hydroxyäthansulfinyl)-4-phenyl-5-(3-pyridyl)-thiazol und

2-(2-Hydroxyäthansulfonyl)-4-phenyl-5-(3-pyridyl)-thiazol.

Beispiel 35: 1,2 g 2-(2-Bromäthylthio)-4,5-(bis-p-methoxyphenyl)-thiazol werden in 10 ml Polyäthylenglykol gelöst, mit 0,5 ml Wasser und 0,5 g Kaliumhydroxid versetzt, 30 Minuten auf 120° erwärmt, auf Raumtemperatur abgekühlt, mit 50 ml Wasser verdünnt und 2 Stunden bei +5° stehengelassen. Man filtriert die ausgefallenen Kristalle ab und kristallisiert aus Diäthyläther/Hexan um. Man erhält das 2-(2-Hydroxyäthylthio)-4,5-bis-(p-methoxyphenyl)-thiazol vom Smp. 82-83°.

Das Ausgangsmaterial kann z.B. folgendermassen erhalten werden:

2,0 g 4,5-Bis-(p-methoxyphenyl)-thiazolin-2-thion werden in 10,4 ml 2n-Natronlauge gelöst. Man fügt 2,3 g Dibromäthan hinzu und lässt 4 Stunden bei Raumtemperatur rühren. Dann trennt man die organische Schicht ab, schüttelt die Wasserphase mit 10 ml Toluol nach, wäscht die vereinigten organischen Phasen zweimal mit je 5 ml 2n-Natronlauge, trocknet über Magnesiumsulfat und dampft unter vermindertem Druck zur Trockne ein. Man erhält das 2-(2-Bromäthylthio)-4,5-bis-(p-methoxyphenyl)-thiazol, $R_f$ = 0,65 (Kieselgel, Toluol/Essigester 10:1).

Beispiel 36: 0,8 g 2-(2-Bromäthylthio)-4,5-bis-(p-methoxyphenyl)-thiazol werden in 20 ml Toluol gelöst. Man gibt 2 ml einer 30%igen Lösung von Tetrabutylammoniumhydroxid in Wasser hinzu und erhitzt unter lebhaftem Rühren 2 Stunden zum Rückfluss. Dann lässt man abkühlen, trennt die organische Phase ab, wäscht zweimal mit je 10 ml Wasser, trocknet über Natriumsulfat und dampft zur Trockne ein. Das Rohprodukt wir an einer Kieselgelsäule mit Toluol/Essigester (10:1) als Laufmittel chromatographisch gereinigt. Die nach Ausweis des Dünnschichtchromatogramms das 2-Vinylthio-4,5-bis-(p-methoxyphenyl)-thiazol enthaltenden Fraktionen werden eingedampft und bis zur Gewichtskonstanz getrocknet. Man erhält das 2-Vinylthio-4,5-bis-(p-methoxyphenyl)-thiazol als hellgelbes Oel.

Beispiel 37: Tabletten enthaltend 25 mg Wirkstoff, z.B. 2-(2-Hydroxyäthylthio)-4,5-bis-(p-methoxyphenyl)-thiazol, können folgendermassen hergestellt werden:

| Bestandteile (für 1000 Tabletten) | |
|---|---|
| Wirkstoff | 25,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 38: Tabletten enthaltend 10 mg Wirkstoff, z.B. 2-(2-Hydroxyäthylthio-4,5-bis-(p-methoxyphenyl)-thiazol, können folgendermassen hergestellt werden:

| Bestandteile (für 1000 Tabletten) | |
|---|---|
| Wirkstoff | 10,0 g |
| Lactose | 100,7 g |
| Weizenstärke | 7,5 g |
| Polyäthylenglykol 6000 | 5,0 g |

| | |
|---|---|
| Talkum | 5,0 g |
| Magnesiumstearat | 1,8 g |
| entmineralisiertes Wasser | q.w. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der STärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 39: Tabletten enthaltend 75 mg Wirkstoff, z.B. 2-(2-Hydroxyäthylthio-4,5-bis-(p-methoxyphenyl)-thiazol, können folgendermassen hergestellt werden:

| Bestandteile (für 1000 Tabletten) | |
|---|---|
| Wirkstoff | 75 ,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser

suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 40: In analoger Weise wie in den Beispielen 37-39 beschrieben kann man auch Tabletten enthaltend eine andere gemäss einem der Beispiele 1-36 anderen erhältlichen Verbindungen herstellen.

Patentansprüche

1. Ein neues 2,4,5-trisubstituiertes Thiazol der Formel

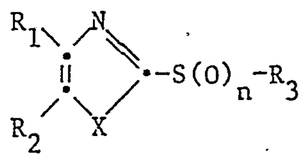

(I),

worin $R_1$ und $R_2$ unabhängig voneinander unsubstituierte oder durch aliphatische Kohlenwasserstoffreste, freies, veräthertes oder verestertes Hydroxy, veräthertes, gegebenenfalls S-oxidiertes Mercapto, aliphatisch substituiertes Amino und/oder Trifluormethyl substituierte Aryl- oder gegebenenfalls N-oxidierte Heteroarylreste bedeuten, X Thio bedeutet, n für 0, 1 oder 2 steht, und $R_3$ einen unsubstituierten oder durch veräthertes oder verestertes Hydroxy, veräthertes gegebenenfalls S-oxidiertes Mercapto und/oder in höherer als der α-Stellung durch Oxo und/oder Hydroxy substituierten, gegebenenfalls zusätzlich zu endständig gebundenem Hydroxy oder in Salzform vorliegendes Sulfo aufweisenden, aliphatischen, einen unsubstituierten oder im Arylteil wie für $R_1$ bzw. $R_2$ angegeben substituierten araliphatischen oder einen unsubstituierten oder in höherer als der α-Stellung durch freies, veräthertes oder verestertes Hydroxy substituierten cycloaliphatischen oder cycloaliphatisch-aliphatischen Kohlenwasserstoffrest bedeutet oder eine Gruppe der Formel

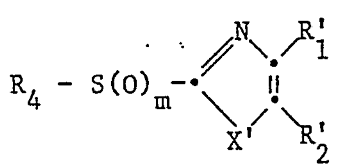

(Ia)

darstellt, in der m für 0, 1 oder 2 steht, $R_4$ einen unsubstituierten oder durch veräthertes oder verestertes Hydroxy, gegebenenfalls S-oxidiertes veräthertes Mercapto oder in höherer als der α- und in niederer als der ω-Stellung durch Oxo und/oder Hydroxy substituierten aliphatischen, gegebenenfalls durch Oxa oder Thia, welches auch S-oxodiert sein kann, unterbrochenen Kohlenwasserstoffrest oder, sofern n und m für 0 stehen, eine direkte Bindung darstellt,

und $R_1'$ und $R_2'$ eine der für $R_1$ und $R_2$ angegebenen Bedeutungen hat, mit der Massgabe, dass in Verbindungen, worin n für 0 oder 1 steht, mindestens einer der Reste $R_1$ und $R_2$ von unsubstituierten oder durch Alkyl, Alkoxy, Halogen und/oder Trifluormethyl substituiertem Phenyl verschieden ist, wenn $R_3$ Alkyl bedeutet, mit der weiteren Massgabe, dass in Verbindungen, worin n für 0 steht, mindestens einer der Reste $R_1$ und $R_2$ einen unsubstituierten oder wie angegeben substituierten Heteroarylrest bedeutet, wenn $R_3$ $\omega$-Hydroxy- oder $\omega$-Oxoniederalkyl mit mehr als 3 Kohlenstoffatomen darstellt, bzw. mindestens einer der Reste $R_1$ und $R_2$ einen unsubstituierten oder wie angegeben substituierten Heteroarylrest oder beide Reste $R_1$ und $R_2$ zugleich p-Methoxyphenyl bedeuten, wenn $R_3$ $\omega$-Hydroxy- oder $\omega$-Oxoniederalkyl mit 2 oder 3 Kohlenstoffatomen darstellt, oder ein Salz davon.

2. Eine Verbindung gemäss Anspruch 1. der Formel I, worin $R_1$ und $R_2$ unabhängig voneinander unsubstituiertes oder durch Niederalkyl, Niederalkoxy bzw. an vicinalen C-Atomen Niederalkylidendioxy, Hydroxy, Halogen, Niederalkanoyloxy, unsubstituiertes oder durch Niederalkyl, Niederalkoxy und/oder Halogen substituiertes Benzoyl- oder Pyridoyloxy, Niederalkylthio bzw. an vicinalen C-Atomen Niederalkylidendithio, Niederalkansulfinyl, Niederalkansulfonyl, Amino, Mono- oder Diniederalkylamino, 3- bis 7-gliedriges Alkylen- bzw. 3-Aza-, 3-Oxa- oder 3-Thianiederalkylenamino, Nitro und/oder Trifluormethyl substituiertes Aryl mit 6 bis und mit 10 C-Atomen oder monocyclische, ein Sauerstoff- oder Schwefelatom und gegebenenfalls zusätzlich ein Stickstoffatom aufweisende 5-gliedrige bzw. gegebenenfalls N-oxidierte ein oder zwei Stickstoffatom(e) aufweisende 6-gliedriges Heteroaryl bedeuten, X Thio darstellt, n für 0, 1 oder 2 steht, $R_3$ Niederalkyl, Niederalkenyl oder Niederalkinyl, Mono- oder Diniederalkoxyniederalkyl, Niederalkylendioxyniederalkyl, Niederalkylidendioxyniederalkyl, die Niederalkanoyloxygruppe(n) in höherer als der $\alpha$-Stellung tragendes Mono- oder Diniederalkanoyloxyniederalkyl, Polyhalogenniederalkyl, Mono- oder

Diniederalkylthioniederalkyl, Niederalkansulfinyl- bzw. Niederalkansulfonylniederalkyl, Niederalkylendithioniederalkyl, die Hydroxygruppe(n) bzw. die Niederalk(anoyl)oxygruppe in höherer als der α-Stellung tragendes Mono- oder Dihydroxyniederalkyl, Hydroxyniederalkoxyniederalkyl, Niederalkanoyloxyniederalkoxyniederalkyl, Niederalkoxyniederalkoxyniederalkyl, Niederalkylthioniederalkoxyniederalkyl, Hydroxyniederalkylthioniederalkyl, Niederalkoxyniederalkylthioniederalkyl oder Niederalkanoyloxyniederalkylthioniederalkyl, die Oxogruppe in höherer als der α-Stellung tragendes Oxoniederalkyl, unsubstituiertes oder wie für $R_1$ und $R_2$ angegeben substituiertes Phenylniederalkyl, 3- bis 8-gliedriges Cycloalkyl bzw. Cycloalkylniederalkyl oder die Hydroxy- bzw. Niederalk(anoyl)oxygruppe in höherer als der α-Stellung tragendes Hydroxycycloalkyl, Niederalkoxycycloalkyl, Niederalkanoyloxyniederalkyl, Cycloalkyl-(hydroxy)-niederalkyl bedeutet oder einen Rest der Formel Ia darstellt in der m für 0,1 oder 2 steht, $R_4$ Niederalkylen, über ein gesättigtes C-Atom gebundenes Niederalkenylen, Niederalkoxyniederalkylen, die Niederalkanoyloxygruppe in höherer als der α- und in niederer als der ω-Stellung tragendes Mono- oder Diniederalkanoyloxyniederalkylen, Polyhalogenniederalkylen, Niederalkylthioniederalkylen, oder die Hydroxy- bzw. Oxogruppe in höherer als der α- und in niederer als der ω-Stellung tragendes Mono- oder Dihydroxy- bzw. Oxoniederalkylen, Oxaniederalkylen oder gegebenenfalls S-oxidiertes Thianiederalkylen bedeutet oder, sofern n und m für 0 stehen, eine direkte Bindung darstellt und $R_1'$ und $R_2'$ eine der für $R_1$ bzw. $R_2$ angegebenen Bedeutungen haben, mit der Massgabe, dass in Verbindungen, worin n für 0 oder 1 steht, mindestens einer der Reste $R_1$ und $R_2$ von unsubstituierten oder durch Niederalkyl, Niederalkoxy, Halogen und/oder Trifluormethyl substituiertem Phenyl verschieden ist, wenn $R_3$ Niederalkyl bedeutet, und mit der weiteren Massgabe, dass in Verbindungen, worin n für 0 steht, mindestens einer der Reste $R_1$ und $R_2$ unsubstituiertes oder wie angegeben substituiertes Heteroaryl gemäss der vorstehenden Definition bedeutet,

wenn $R_3$ ω-Hydroxy- oder ω-Oxoniederalkyl mit mehr als 3 C-Atomen darstellt, bzw. mindestens einer der Reste $R_1$ und $R_2$ Heteroaryl gemäss der vorstehenden Definition bedeutet oder beide Reste $R_1$ und $R_2$ zugleich für p-Methoxyphenyl stehen, wenn $R_3$ ω-Hydroxy- oder ω-Oxoniederalkyl mit 2 oder 3 C-Atomen darstellen, oder ein Salz davon.

3. Eine Verbindung gemäss Anspruch 1 der Formel I, worin $R_1$ und $R_2$ unabhängig voneinander unsubstituiertes oder durch Niederalkyl, Niederalkoxy bzw. an vicinalen C-Atomen Niederalkylidendioxy, Hydroxy, Halogen, Niederalkanoyloxy, unsubstituiertes oder durch Niederalkyl, Niederalkoxy und/oder Halogen substituiertes Benzoyl- oder Pyridoyloxy, Niederalkylthio bzw. an vicinalen C-Atomen Niederalkylidendithio, Niederalkansulfinyl, Niederalkansulfonyl, Amino, Mono- oder Diniederalkylamino, 3- bis 7-gliedriges Alkylen- bzw. 3-Aza-, 3-Oxa- oder 3-Thianiederalkylenamino, Nitro und/oder Trifluormethyl substituiertes Aryl mit 6 bis und mit 10 C-Atomen oder monocyclische, ein Sauerstoff- oder Schwefelatom und gegebenenfalls zusätzlich ein Stickstoffatom aufweisende 5-gliedrige bzw. gegebenenfalls N-oxidierte ein oder zwei Stickstoffatom(e) aufweisende 6-gliedriges Heteroaryl bedeuten, X Thio darstellt, n für 0, 1 oder 2 steht, $R_3$ Niederalkyl, über ein gesättigtes C-Atom gebundenes Niederalkenyl oder Niederalkinyl, Mono- oder Diniederalkoxyniederalkyl, Niederalkylendioxyniederalkyl, Niederalkylidendioxyniederalkyl, die Niederalkanoyloxygruppe(n) in höherer als der α-Stellung tragendes Mono- oder Diniederalkanoyloxyniederalkyl, Polyhalogenniederalkyl, Mono- oder Diniederalkylthioniederalkyl, Niederalkansulfinyl- bzw. Niederalkansulfonylniederalkyl, Niederalkylendithioniederalkyl, die Hydroxygruppe(n) bzw. die Niederalk(anoyl)oxygruppe in höherer als der α-Stellung tragendes Mono- oder Dihydroxyniederalkyl, Hydroxyniederalkoxyniederalkyl, Niederalkanoyloxyniederalkoxyniederalkyl, Niederalkoxyniederalkoxyniederalkyl, Niederalkylthioniederalkoxyniederalkyl, Hydroxyniederalkylthioniederalkyl, Niederalkoxyniederalkylthioniederalkyl oder Niederalkanoyloxyniederalkylthioniederalkyl, die Oxogruppe in höherer als der α-Stellung tragendes Oxoniederalkyl, unsubstituiertes oder wie für $R_1$ und $R_2$ angegeben substituiertes Phenylniederalkyl, 3- bis 8-gliedriges Cycloalkyl bzw. Cycloalkylniederalkyl oder die Hydroxy- bzw. Niederalk(anoyl)oxygruppe in höherer als der α-Stellung tragendes Hydroxycycloalkyl, Niederalkoxycycloalkyl, Niederalkanoyloxyniederalkyl, Cycloalkyl-(hydroxy)-niederalkyl bedeutet oder einen Rest der Formel Ia darstellt, in der m für 0,1 oder 2 steht, $R_4$ Niederalkylen, über ein gesättigtes C-Atom gebundenes Niederalkenylen, Niederalkoxyniederalkylen, die Niederalkanoyloxygruppe in höherer als der α- und in niederer als der ω-Stellung tragendes Mono- oder Diniederalkanoyloxyniederalkylen, Polyhalogenniederalkylen, Niederalkylthioniederalkylen, oder die Hydroxy- bzw. Oxogruppe in höherer als der α- und in niederer als der ω-Stellung tragendes Mono- oder Dihydroxy- bzw. Oxoniederalkylen, Oxaniederalkylen oder gegebenenfalls S-oxidiertes Thianiederalkylen bedeutet oder, sofern n und m für 0 stehen, eine direkte Bindung darstellt und $R'_1$ und $R'_2$ eine der für $R_1$ bzw. $R_2$ X angegebenenen Bedeutungen haben, mit der Massgabe, dass in Verbindungen, worin n für 0 oder 1 steht, mindestens einer der Reste $R_1$ und $R_2$ von unsubstituierten oder durch Niederalkyl, Niederalkoxy, Halogen und/oder Trifluormethyl substituiertem Phenyl verschieden ist, wenn $R_3$ Niederalkyl bedeutet, und mit der weiteren Massgabe, dass in Verbindungen, worin n für 0 steht, mindestens einer der Reste $R_1$ und $R_2$ Heteroaryl gemäss der vorstehenden Definition ist, wenn $R_3$ ω-Hydroxy- oder ω-Oxoniederalkyl bedeutet, oder ein Salz davon.

4. Eine Verbindung gemäss Anspruch 1 der Formel I, worin $R_1$ und $R_2$ unabhängig voneinander unsubstituiertes oder durch Niederalkyl, Niederalkoxy, Hydroxy, Halogen, Niederalkanoyloxy, Niederalkylthio, Niederalkansulfonyl, Diniederalkylamino, 3- bis 7-gliedriges Alkylen- bzw. 3-Aza-, 3-Oxa- oder 3-Thianiederalkylenamino und/oder Trifluormethyl substituiertes Phenyl oder unsubstituiertes oder durch Niederalkyl, Niederalkoxy oder Hydroxy substituiertes Furyl, Pyridyl bzw. 1-Oxidopyridyl bedeuten, X Thio darstellt, n für 0, 1 oder 2 steht, $R_3$ Niederalkyl, über ein gesättigtes C-Atom gebundenes Niederalkenyl oder Niederalkinyl, Mono- oder Diniederalkoxyniederalkyl, Niederalkylendioxyniederalkyl, Niederalkylidendioxyniederalkyl, die Niederalkanoyloxygruppe(n) in höherer als der α-Stellung tragendes Mono- oder Diniederalkanoyloxyniederalkyl, Mono- oder Diniederalkylthioniederalkyl, die Hydroxygruppe(n), die Niederalkanoyloxygruppe bzw. die Niederalkoxygruppe in höherer als der α-Stellung tragendes Mono- oder Dihydroxyniederalkyl, Hydroxyniederalkoxyniederalkyl oder Niederalkanoyloxyniederalkoxyniederalkyl, Niederalkoxyniederalkoxyniederalkyl, Niederalkylthioniederalkoxyniederalkyl, Hydroxyniederalkylthioniederalkyl, Niederalkoxyniederalkylthioniederalkyl oder Niederalkanoyloxyniederalkylthioniederalkyl, in Salzform vorliegendes ω-Hydroxy-ω-sulfoniederalkyl, ω-Hydroxy-ω-sulfo-niederalkenyl und ω-Hydroxy-ω-sulfo-niederalkinyl, ebenso/oder die Oxogruppe in höherer als der α-Stellung tragendes Oxoniederalkyl bedeutet oder einen Rest der Formel Ia darstellt, in der m für 0, 1 oder 2 steht, $R_4$ Niederalkylen, die Hydroxy-, Niederalkanoyloxy- oder Oxogruppe in höherer als der α- und in niederer als der ω-Stellung tragendes Hydroxyniederalkylen, Niederalkanoyloxyniederalkylen oder Oxoniederalkylen bedeutet oder, sofern n und m für 0 stehen, eine direkte Bindung darstellt und $R_1'$ und $R_2'$ eine der für $R_1$ bzw. $R_2$ angegebenen Bedeutungen haben,mit der Massgabe, dass in Verbindungen, worin n für 0 oder 1 steht, mindestens einer der Reste $R_1$ und $R_2$ von unsubstituiertem oder durch Niederalkyl, Niederalkoxy, Halogen und/oder Trifluormethyl substituiertem Phenyl verschieden ist, wenn $R_3$ Niederalkyl bedeutet, und mit der weiteren Massgabe, dass in Verbindungen, worin n für 0 steht, mindestens einer der Reste $R_1$ und $R_2$ unsubstituiertes oder wie angegeben substituiertes Furyl, Thienyl, Pyridyl oder 1-Oxidopyridyl bedeutet, wenn $R_3$ ω-Hydroxy- oder ω-Oxoniederalkyl mit mehr als 3 C-Atomen darstellt, bzw. mindestens einer der Reste $R_1$ und

$R_2$ unsubstituiertes oder wie angegeben substituiertes Furyl, Thienyl, Pyridyl oder 1-Oxidopyridyl bedeutet, oder beide Reste $R_1$ und $R_2$ zugleich p-Methoxyphenyl darstellen, wenn $R_3$ $\omega$-Hydroxy- oder $\omega$-Oxoniederalkyl mit 2 oder 3 C-Atomen bedeutet, oder ein Salz davon.

5. Eine Verbindung gemäss Anspruch 1 der Formel I, worin $R_1$ und $R_2$ unabhängig voneinander unsubstituiertes oder durch Niederalkoxy, Hydroxy, Halogen, Niederalkanoyloxy, Niederalkylthio, Niederalkansulfinyl, Diniederalkylamino und/oder Trifluormethyl substituiertes Phenyl oder unsubstituiertes Thienyl oder unsubstituiertes oder durch Hydroxy substituiertes , gegebenenfalls N-oxidiertes Pyridyl bedeuten, X Thio dars ellt, n für 0, 1 oder 2 steht, $R_3$ Niederalkyl, über ein gesättigtes C-Atom gebundenes Niederalkenyl oder Niederalkinyl, Niederalkoxyniederalkyl, die Niederalkanoyloxygruppe in höherer als der $\alpha$-Stellung tragendes Niederalkanoyloxyniederalkyl, Niederalkylthioniederalkyl, die Hydroxygruppe bzw. die Niederalk(anoyl)oxygruppe in höherer als der $\alpha$-Stellung tragendes Hydroxyniederalkyl, Hydroxyniederalkoxyniederalkyl oder Niederalkanoyloxyniederalkoxyniederalkyl, in Salzform vorliegendes $\omega$-Hydroxy-$\omega$-sulfoniederalkyl, Niederalkoxyniederalkoxyniederalkyl, Niederalkylthioniederalkoxyniederalkyl, oder die Oxogruppe in höherer als der $\alpha$-Stellung tragendes Oxoniederalkyl bedeutet, mit der Massgabe, dass in Verbindungen, worin n für 0 oder 1 steht, mindestens einer der Reste $R_1$ und $R_2$ von unsubstituierten oder durch Niederalkyl, Niederalkoxy, Halogen und/oder Trifluormethyl substituiertem Phenyl verschieden ist, wenn $R_3$ Niederalkyl bedeutet, und mit der weiteren Massgabe, dass in Verbindungen, worin n für 0 steht, mindestens einer der Reste $R_1$ und $R_2$ unsubstituiertes oder wie angegeben substituiertes Phenyl oder Pyridyl ist, wenn $R_3$ $\omega$-Hydroxy- oder $\omega$-Oxoniederalkyl mit mehr als 3 C-Atomen ist, bzw. mindestens einer der Reste $R_1$ und $R_2$ unsubstituiertes oder wie angegeben substituiertes Phenyl oder Pyridyl ist, oder beide zugleich p-Methoxyphenyl bedeuten, wenn $R_3$ $\omega$-Hydroxy- oder $\omega$-Oxoniederalkyl mit 2 oder 3 C-Atomen bedeutet, oder ein Salz davon .

6. Eine Verbindung gemäss Anspruch 1 der Formel I, worin mindestens einer der Reste $R_1$ und $R_2$ gegebenenfalls N-oxidiertes Pyridyl oder Thienyl und der andere unsubstituiertes oder durch Niederalkoxy mit bis und 4 C-Atomen, Niederalkylthio mit bis und mit 4 C-Atomen, Halogen der Atomnummer bis und mit 35 Hydroxy und/oder Trifluormethyl substituiertes Phenyl bedeutet, X Thio darstellt, n für 0, 1 oder 2 steht und $R_3$ Niederalkyl bis und mit 4 C-Atomen, die Oxo-, Hydroxy-, Niederalkanoyloxy-, Niederalkoxy- oder Niederalkylendioxygruppe(n) in höherer als der α-Stellung tragendes Oxo- oder Hydroxyniederalkyl mit jeweils bis und mit 4 C-Atomen, Niederalkanoyloxyniederalkyl mit jeweils bis und mit 4 C-Atomen, Mono- oder Diniederalkoxyniederalkyl mit jeweils bis und mit 4 C-Atomen, oder Niederalkylendioxyniederalkyl mit jeweils bis und mit 4 C-Atomen oder in Salzform vorliegendes ω-Hydroxy-ω-sulfo-niederalkyl mit 2 bis 4 C-Atomen bedeutet oder ein Salz davon.

7. Eine Verbindung gemäss Anspruch 1 der Formel I, worin mindestens einer der Reste $R_1$ und $R_2$ gegebenenfalls N-oxidiertes Pyridyl oder Thienyl und der andere unsubstituiertes oder durch Niederalkoxy mit bis und 4 C-Atomen, Niederalkylthio mit bis und mit 4 C-Atomen, Halogen der Atomnummer bis und mit 35 Hydroxy und/oder Trifluormethyl substituiertes Phenyl bedeutet, X Thio darstellt, n für 0, 1 oder 2 steht und $R_3$ Niederalkyl bis und mit 4 C-Atomen, Polyhalogenniederalkyl, worin Halogen die Atomnummer bis und mit 35 und Niederalkyl bis und mit 4 C-Atome aufweist, die Hydroxy-, Niederalkanoyloxy-, Niederalkoxy-, Niederalkylthio-, Niederalkansulfinyl- bzw. Niederalkansulfonylgruppe in höherer als der α-Stellung tragendes Hydroxyniederalkyl mit 2 bis und mit 7 C-Atomen, Niederalkoxyniederalkyl mit jeweils bis und mit 4 C-Atomen oder Niederalkylthio-, Niederalkanusulfinyl- oder Niederalkansulfonyl mit jeweils bis und mit 4 C-Atomen oder die Hydroxygruppe sowie die Hydroxyniederalkoxy- bzw. Hydroxyniederalkylthiogruppe in höherer als der α-Stellung tragendes Hydroxyniederalkoxyniederalkyl bzw. Hydroxyniederalkylthioniederalkyl bedeutet, oder ein Salz davon.

8. Eine Verbindung genäss Anspruch 1 der Formel I, worin $R_1$ und $R_2$ unabhängig voneinander unsubstituiertes oder durch Niederalkoxy mit bis und mit 4 C-Atomen, Niederalkylthio mit bis und mit 4 C-Atomen, Halogen der Atomnummer bis und mit 35, Hydroxy und/oder Trifluormethyl substituiertes Phenyl bedeuten, X Thio darstellt, n für 0, 1 oder 2 steht und $R_3$ die Niederalkanoyloxy-, Niederalkoxy- bzw. Niederalkylendioxygruppe(n) in höhere als der α-Stellung tragendes Niederalkanoyloxyniederalkyl mit jeweils bis und mit 4 C-Atomen, Mono- oder Di-Niederalkoxyniederalkyl bzw. Niederalkylendioxyniederalkyl mit jeweils bis und mit 4 C-Atomen oder in Salzform vorliegendes ω-Hydroxy-ω-sulfoniederalkyl mit 2 bis 4 C-Atomen bedeutet, oder, sofern n für 2 steht, Niederalkyl mit 2 bis und mit 7 C-Atomen oder, sofern n für 1 oder 2 steht, ω-Hydroxyniederalkyl oder ω-Oxoniederalkyl mit 2 bis und mit 4 C-Atomen darstellt, oder, sofern n für 0 steht und beide Reste $R_1$ und $R_2$ p-Methoxyphenyl darstellen, ω-Hydroxy- oder ω-Oxoniederalkyl mit 2 bis 3 C-Atomen bedeuten oder ein Salz davon.

9. Eine Verbindung gemäss Anspruch 1 der Formel I, worin $R_1$ und $R_2$ unabhängig voneinander unsubstituiertes oder durch Niederalkoxy mit bis und mit 4 C-Atomen, Niederalkylthio mit bis und mit 4 C-Atomen, Halogen der Atomnummer bis und mit 35, Hydroxy und/oder Trifluormethyl substituiertes Phenyl bedeuten, X Thio darstellt, n für 0 oder 2 steht und $R_3$ Polyhalogenniederalkyl, worin Halogen die Atomnummer bis und mit 35 und Niederalkyl bis und mit 4 C-Atome aufweist, die Niederalkanoyloxy., Niederalkoxy-, Niederalkylthio-, Niederalkansulfinyl- bzw. Niederalkansulfonylgruppe in höhere als der α-Stellung tragendes Niederalkanoyloxyniederalkyl mit jeweils bis und mit 4 C-Atomen, Niederalkoxyniederalkyl mit jeweils bis und mit 4 C-Atomen oder Niederalkylthio-, Niederalkansulfinyl- oder Niederalkansulfonyl mit jeweils bis und mit 4 C-Atomen, oder die Hydroxygruppe sowie die Hydroxyniederalkyl- bzw. Hydroxyniederalkylthiogruppe in höherer als der α-Stellung tragendes Hydroxyniederalkoxyniederalkyl bzw. Hydroxyniederalkylthioniederalkyl

oder die Hydroxygruppe in höherer als der α- und in niederer als der ω-Stellung tragendes Hydroxyniederalkyl mit 2 bis und mit 4 C-Atomen bedeutet, oder, sofern n für 2 steht, Niederalkyl mit 2 bis und mit 7 C-Atomen oder, sofern n für 1 oder 2 steht, ω-Hydroxyniederalkyl mit 2 bis und mit 7 C-Atomen darstellt, oder ein Salz davon.

10. Eine Verbindung gemäss Anspruch 1 der Formel I, worin mindestens einer der Reste $R_1$ und $R_2$ Pyridyl oder Thienyl und der andere unsubstituiertes oder durch Niederalkoxy mit bis und mit 4 C-Atomen oder Halogen der Atomnummer bis und mit 35 substituiertes Phenyl bedeutet, X Thio darstellt, n für 0, 1 oder 2 steht und $R_3$ Niederalkyl mit bis und mit 4 C-Atomen, in Salzform vorliegendes ω-Hydroxy-ω-sulfoniederalkyl mit 2 bis 4 C-Atomen, ω-Hydroxyniederalkyl mit 2 bis 4 C-Atomen, ω,ω-Diniederalkoxyniederalkyl mit bis und mit 4 C-Atomen, ω-Niederalkanoyloxyniederalkyl mit je bis und mit 4 C-Atomen oder ω-Oxoniederalkyl mit bis und mit 4 C-Atomen bedeutet, oder ein Salz davon.

11. Eine Verbindung gemäss Anspruch 1 der Formel I, worin mindestens einer der Reste $R_1$ und $R_2$ Pyridyl oder Thienyl und der andere unsubstituiertes oder durch Niederalkoxy mit bis und mit 4 C-Atomen oder Halogen der Atomnummer bis und mit 35 substituiertes Phenyl bedeutet, X Thio darstellt, n für 0, 1 oder 2 steht, und $R_3$ Niederalkyl mit bis und mit 4 C-Atomen, ω-Hydroxyniederalkyl mit 2 bis 4 C-Atomen, 2- oder 3-Niederalkoxyniederalkyl bzw. 2- oder 3-Niederalkylthioniederalkyl mit bis und mit 4 C-Atomen oder 2- oder 3-(ω-Hydroxyniederalkoxy)- bzw. 2- oder 3-(ω-Hydroxyniederalkylthio)-niederalkyl mit bis und mit 4 C-Atomen bedeutet, oder ein Salz davon.

12. Eine Verbindung gemäss Anspruch 1 der Formel I, worin $R_1$ und $R_2$ unabhängig voneinander unsubstituiertes oder durch Niederalkoxy mit bis und mit 4 C-Atomen oder Halogen der Atomnummer bis und mit 35 substituiertes Phenyl bedeuten, X Thio darstellt, n für 0, 1 oder 2

steht und $R_3$ in Salzform vorliegendes ω-Hydroxy-ω-sulfo-niederalkyl mit 2 bis 4 C-Atomen, ω-Diniederalkoxyniederalkyl bzw. ω-Niederalkanoyloxyniederalkyl mit je bis und mit 4 C-Atomen oder ω-Niederalkylendioxyniederalkyl mit je bis und mit 4 C-Atomen bedeutet oder, sofern n für 2 steht, Niederalkyl mit bis und mit 4 C-Atomen bedeutet oder, sofern n für 1 oder 2 steht, ω-Hydroxyniederalkyl bzw. ω-Oxoniederalkyl mit 2 bis und mit 4 C-Atomen darstellt, oder, sofern n für 0 steht, und $R_1$ und $R_2$ zugleich p-Methoxyphenyl bedeuten, ω-Hydroxy- oder ω-Oxoniederalkyl mit 2 oder 3 C-Atomen bedeuten, oder ein Salz davon.

13. Eine Verbindung gemäss Anspruch 1 der Formel I, worin $R_1$ und $R_2$ unabhängig voneinander unsubstituiertes oder durch Niederalkoxy mit bis und mit 4 C-Atomen oder Halogen der Atomnummer bis und mit 35 substituiertes Phenyl bedeuten, X Thio darstellt, n für 0, 1 oder 2 steht und $R_3$ 2- oder 3-Niederalkoxyniederalkyl bzw. 2- oder 3-Niederalkylthioniederalkyl mit bis und mit 4 C-Atomen oder 2- oder 3-(ω-Hydroxyniederalkoxy)- bzw. 2- oder 3-(ω-Hydroxyniederalkylthio)-niederalkyl mit bis und mit 4 C-Atomen bedeutet oder, sofern n für 2 steht, Niederalkyl mit bis und mit 4 C-Atomen, bedeutet oder, sofern n für 1 oder 2 steht, ω-Hydroxyniederalkyl mit 2 bis und mit 4 C-Atomen darstellt, oder ein Salz davon.

14. 2-Methylthio-4,5-bis-phenyl-thiazol oder ein Salz davon.

15. 2-Propylthio-4,5-bis-phenyl-thiazol oder ein Salz davon.

16. 2-Methylthio-4,5-bis-(p-chlorphenyl)-thiazol oder ein Salz davon.

17. 2-Aethylthio-4,5-bis-(p-chlorphenyl)-thiazol oder ein Salz davon.

18. 2-Propylthio-4,5-bis-(p-chlorphenyl)-thiazol oder ein Salz davon.

19. 2-Methylthio-5-phenyl-4-(3-pyridyl)-thiazol oder ein Salz davon.

20. 2-Aethylthio-5-phenyl-4-(3-pyridyl)-thiazol oder ein Salz davon.

21. 2-(2-Hydroxyäthylthio)-5-phenyl-4-(3-pyridyl)-thiazol oder ein Salz davon.

22. 2-Methylthio-4,5-bis-(p-methoxyphenyl)-thiazol oder ein Salz davon.

23. 2-(2-Hydroxyäthylthio)-4,5-bis-(p-methoxyphenyl)-thiazol oder ein Salz davon.

24. 2-Aethylthio-4,5-bis-(p-methoxyphenyl)-thiazol oder ein Salz davon.

25. 2-Propylthio-4,5-bis-(p-methoxyphenyl)-thiazol oder ein Salz davon.

26. 2-(1,1,2,2-Tetrafluoräthylthio)-4,5-bis-phenyl)-thiazol oder ein Salz davon.

27. 2-(1,1,2,2-Tetrafluoräthylthio)-4,5-bis-(p-chlorphenyl)-thiazol oder ein Salz davon.

28. 2-(1,1,2,2-Tetrafluoräthylthio)-4,5-bis-(p-methoxyphenyl)-thiazol oder ein Salz davon.

29. 2-(3-Hydroxypropylthio)-4,5-bis-(p-methoxyphenyl)-thiazol oder ein Salz davon.

30. 2-(3-Hydroxypropansulfinyl)-4,5-bis-(p-methoxyphenyl)-thiazol oder ein Salz davon.

31. 2-(3-Hydroxypropansulfonyl)-4,5-bis-(p-methoxyphenyl)-thiazol oder ein Salz davon.

32.2-(2-Hydroxyäthansulfonyl)-4,5-bis-phenyl-thiazol oder ein Salz davon.

33. 2-(2-Hydroxyäthansulfonyl)-4,5-bis-(p-chlorphenyl)-thiazol oder ein Salz davon.

34. 2-(2-Hydroxyäthansulfonyl)-4,5-bis-(p-methoxyphenyl)-thiazol oder ein Salz davon.

35. 2-Methansulfonyl-4,5-bis-(p-methoxyphenyl)-thiazol oder ein Salz davon.

36. 2-Aethansulfonyl-4,5-bis-(p-methoxyphenyl)-thiazol oder ein Salz davon.

37. 2-Methansulfinyl-5-phenyl-4-(3-pyridyl)-thiazol oder ein Salz davon.

38. 2-Methansulfonyl-5-phenyl-4-(3-pyridyl)-thiazol oder ein Salz davon.

39. 2-Aethansulfinyl—5-phenyl-4-(3-pyridyl)-thiazol oder ein Salz davon.

40. 2-Aethansulfonyl)-5-phenyl-4-(3-pyridyl)-thiazol oder ein Salz davon.

41. 2-(2-Hydroxyäthansulfinyl)-5-phenyl-4-(3-pyridyl)-thiazol oder ein Salz davon.

42. 2-(2-Hydroxyäthansulfonyl)-5-phenyl-4-(3-Pyridyl)-thiazol oder ein Salz davon.

43. 2-(1,1,2,2-Tetrafluoräthylthio)-5-phenyl-4-(3-pyridyl)-thiazol oder ein Salz davon.

44. 2-Methylthio-4-phenyl-5-(3-pyridyl)-thiazol oder ein Salz davon.

45. 2-Aethylthio-4-phenyl-5-(3-pyridyl)-thiazol oder ein Salz davon.

46. 2-(2-Hydroxyäthylthio)-4-phenyl-5-(3-pyridyl)-thiazol oder ein Salz davon.

47. 1,3-Bis-{2-[5-phenyl-4-(3-pyridyl)-thiazolyl]-thio}-2-hydroxypropan oder ein Salz davon.

48. 1,3-Bis-{2-[5-phenyl-4-(3-pyridyl)-thiazolyl]-thio}-2-oxopropan oder ein Salz davon.

49. 2,2'-Bis-[5-phenyl-4-(3-pyridyl)-thiazolyl]-disulfid oder ein Salz davon.

50. 2,2'-Bis-[4,5-bis-(p-methoxyphenyl)-thiazolyl]-disulfid oder ein Salz davon.

51. 2-Methansulfinyl-4-phenyl-5-(3-pyridyl)-thiazol oder ein Salz davon.

52. 2-Methansulfonyl-4-phenyl-5-(3-pyridyl)-thiazol oder ein Salz davon.

53. 2-Aethansulfonyl-4-phenyl-5-(3-pyridyl)-thiazol oder ein Salz davon.

54. 2-Aethansulfinyl-4-phenyl-5-(3-pyridyl)-thiazol oder ein Salz davon.

55. 2-(2-Hydroxyäthansulfinyl)-4-phenyl-5-(3-pyridyl)-thiazol oder ein Salz davon.

56. 2-(2-Hydroxyäthansulfonyl)-4-phenyl-5-(3-pyridyl)-thiazol oder ein Salz davon.

57. 1,3-Bis-[4,5-bis-(p-methoxyphenyl)-thiazolyl-(2)-thio]-propan-2-on oder ein Salz davon.

58. 2-[4,5-Bis-(p-methoxyphenyl)-thiazolyl-(2)-thio]-acetaldehyd oder ein Salz davon.

59. 2-[4,5-Bis-(p-methoxyphenyl)-thiazolyl-(2)-thio]-acetaldehyd-dimethylacetal oder ein Salz davon.

60. 2-(2-Hydroxyäthansulfinyl)-4,5-bis-(p-methoxyphenyl)-thiazol oder ein Salz davon.

61. 2-[4,5-Bis-(p-methoxyphenyl)-thiazolyl-(2)-sulfinyl]-acetaldehyd oder ein Salz davon.

62. 2-[4,5-Bis-(p-methoxyphenyl)-thiazolyl-(2)-sulfonyl]-acetaldehyd oder ein Salz davon.

63. 2-(2-Hydroxyäthylthio)-4-(p-methoxyphenyl)-5-(4-pyridyl)-thiazol oder ein Salz davon.

64. 2-(2-Hydroxyäthylthio)-4-phenyl-5-(3-pyridyl)-thiazol oder ein Salz davon.

65. 2-[4,5-Bis-(p-methoxyphenyl)-thiazolyl-(2)-sulfinyl]-acetaldehyd-dimethylacetal oder ein Salz davon.

66. 2-[4,5-Bis-(p-methoxyphenyl)-thiazolyl-(2)-sulfonyl]-acetaldehyd-dimethylacetal oder ein Salz davon.

67. Kalium-2-[4,5-Bis-(p-methoxyphenyl)-thiazolyl-(2)-thio]-1-hydroxy-äthansulfonat.

68. 2-(2-Acetoxyäthylthio)-4,5-bis-(p-methoxyphenyl)-thiazol oder ein Salz davon.

69. 2-Allylthio-4,5-bis-(p-methoxyphenyl)-thiazol oder ein Salz davon.

70. 2-Vinylthio-4,5-bis-(p-methoxyphenyl)-thiazol oder ein Salz davon.

71. 2-Aethinylthio-4,5-bis-(p-methoxyphenyl)-thiazol oder ein Salz davon.

72. 2-(2-Methoxyäthylthio)-4,5-bis-(p-methoxyphenyl)-thiazol oder ein Salz davon.

73. 2-(2-Methylthioäthylthio)-4,5-bis-(p-methoxyphenyl)-thiazol oder ein Sakz davon.

74. 2-(2,3-Dimethoxypropylthio)-4,5-bis-(p-methoxyphenyl)-thiazol oder ein Salz davon.

75. 2-(2,3-Dihydroxypropylthio)-4,5-bis-(p-methoxyphenyl)-thiazol oder ein Salz davon.

76. 2-(2,3-Isopropylidendioxypropylthio)-4,5-bis-(p-methoxyphenyl)-thiazol oder ein Salz davon.

77. 2-(2,2-Aethylidendithioäthylthio)-4,5-bis-(p-phenoxyphenyl)-thiazol oder ein Salz davon.

78. 2-(2,2-Aethylidendioxyäthylthio)-4,5-bis-(p-methoxyphenyl)-thiazol oder ein Salz davon.

79. 3-[4,5-Bis-(p-methoxyphenyl)-thiazolyl-(2)-thio]-propionaldehyd oder ein Salz davon.

80. 3-[4,5-Bis-(p-methoxyphenyl)-thiazolyl-(2)-thio]-propionaldehyd-dimethylacetal oder ein Salz davon.

81. Die in den Beispielen 1 bis 32 genannten neuen Verbindungen.

82. Verfahren zur Herstellung neuer 2,4,5-trisubstituierter Azole gemäss Anspruch 1, dadurch gekennzeichnet, dass man aus einer Verbindung der Formel

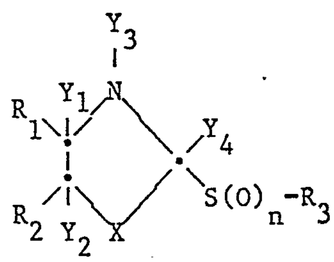 (II),

worin einer der Reste $Y_1$ und $Y_2$ einen abspaltbaren Rest Y und der andere ein Wasserstoffatom bedeutet und $Y_3$ und $Y_4$ gemeinsam eine zusätzliche Bindung darstellen, oder worin $Y_4$ einen abspaltbaren Rest Y und $Y_3$ Wasserstoff bedeutet und $Y_1$ und $Y_2$ eine zusätzliche Bindung darstellen, oder aus einem Salz davon unter Einführung einer zusätzlichen Bindung HY abspaltet, oder Verbindungen der Formel

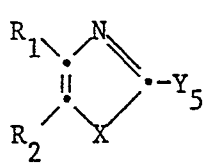 (XVI) und $Y_6-R_3$ (XII),

worin $Y_5$ eine gegebenenfalls in Salzform vorliegende Gruppe der Formel $-S(O)_n-H$ oder -S-SH und $Y_6$ reaktionsfähiges verestertes Hydroxy oder, sofern $R_3$ in α,β-Stellung mindestens eine zusätzliche C-C-Bindung aufweist, veräthertes Hydroxy, bedeutet oder $Y_5$ reaktionsfähiges verestertes Hydroxy und $Y_6$ eine gegebenenfalls in Salzform vorliegende Gruppe der Formel -SH- oder -S-SH darstellt, oder worin einer der Reste $Y_5$ und $Y_6$ einen metallischen Rest, der andere eine Gruppe $-S(O)_n-Y_7$ bedeutet, in der $Y_7$ reaktionsfähiges verestertes Hydroxy darstellt oder, sofern n für 1 oder 2 steht, veräthertes Hydroxy oder, sofern n für 0 steht, veräthertes Mercapro bedeutet und $R_3$ einen unsubstituierten oder durch verätherte Hydroxy- und/oder Mercaptogruppen substituierten, gegebenenfalls durch Oxa oder Thia unterbrochenen aliphatischen, einen unsubstituierten oder ein Arylteil gegebenenfalls wie für $R_1$ bzw. $R_2$ angegeben substituierten aliphatischen oder einen unsubstituierten oder durch veräthertes Hydroxy substituierten cycloaliphatischen oder cycloaliphatisch-aliphatischen Kohlenwasserstoffrest bedeutet oder aufweist, miteinander kondensiert oder eine Verbindung der Formel

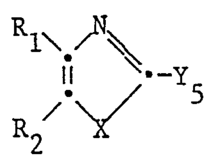

(XVI),

worin $Y_5$ Mercapto ist, oder ein Salz davon mit einem von einer Verbindung der Formel $R_3$-H (XIX) abgeleiteten vicinalen Epoxidderivat zu einer Verbindung der Formel I, worin $R_3$ in β-Stellung eine Hydroxygruppe aufweist und n für 0 steht, oder mit einer Verbindung der Formel $R_3'$-H (XX), worin $R_3'$ ein von $R_3$ abgeleiteter mindestens eine C-C-Doppel- oder C-C-Dreifachbindung aufweisenden Rest bedeutet, zu einer Verbindung der Formel I, worin $R_3$ α,β-gesättigt oder α,β-einfach ungesättigt ist und n für 0 steht, umsetzt, oder in einer Verbindung der Formel

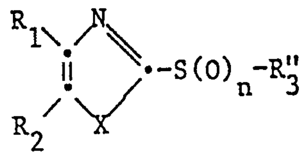 (XXV),

worin $R_3''$ einen in einen Rest $R_3$ überführbaren Rest bedeutet, $R_3''$ in die Gruppe $R_3$ überführt oder zur Herstellung von Verbindungen der Formel I, worin $R_3$ eine Gruppe der Formel Ia darstellt, n und m für 0 stehen und $R_4$ eine direkte Bindung darstellt, Verbindungen der Formeln

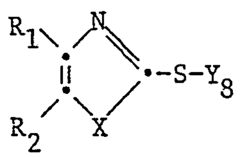

(XXI)

und

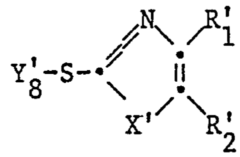

(XXII),

worin die Reste $Y_8$ und $Y_8'$ unter Ausbildung einer Bindung abspaltbare Gruppen bedeuten, miteinander kondensiert, oder zur Herstellung von Verbindungen der Formel I, worin X Thio und $R_3$ eine Gruppe der Formel Ia bedeutet, in der X Thio, $R_1'$ einen Rest $R_1$, $R_2'$ einen Rest $R_2$ und m 0 darstellt, eine Verbindung der Formel

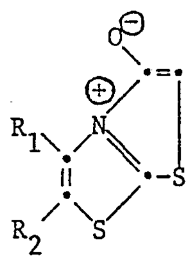 (XXIII)

der Basebehandlung unterwirft, wobei Umlagerung zu einer entsprechenden Verbindung der Formel I, worin in dem Rest $R_3$ der Gruppe $R_4$ 1,3-(2-Oxo)-propylen bedeutet, jeweils erforderlichenfalls ein verfahrensgemäss erhältliches Isomerengemisch in die reinen Isomeren auftrennt und gewünschtenfalls jeweils eine verfahrensgemäss erhältliche Verbindung in eine andere Verbindung der Formel I überführt

und/oder eine verfahrensgemäss erhältliche freie Verbindung in ein Salz oder ein verfahrensgemäss erhältliches Salz in die freie Verbindung umwandelt.

83. Verfahren nach Anspruch 82, dadurch gekennzeichnet, dass man von einer auf beliebiger Verfahrensstuffe als Zwischenprodukte erhältlichen Verbindungen ausgeht und die fehlenden Verfahrensschritte ausführt, oder einen Ausgangsstoff unter den Reaktionsbedingungen bildet oder in Form eines Derivats davon, gegebenenfalls eines Salzes, verwendet.

84. Das Verfahren der Beispiele 1 bis 32 und die danach bzw. nach dem Verfahren gemäss Anspruch 82 oder 83 erhältlichen neuen Verbindungen.

85. Ein 2,4,5-trisubstituiertes Thiazol der Formel

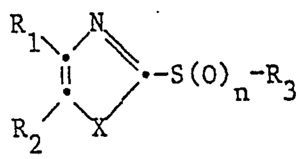 (I),

worin $R_1$ und $R_2$ unabhängig voneinander unsubstituierte oder durch aliphatische Kohlenwasserstoffreste, freies, veräthertes oder verestertes Hydroxy, veräthertes, gegebenenfalls S-oxidiertes Mercapto, aliphtisch substituiertes Amino und/oder Trifluormethyl substituierte Aryl- oder gegebenenfalls N-oxidierte Heteroarylreste bedeuten, X Thio bedeutet, n für 0, 1 oder 2 steht und $R_3$ einen unsubstituierten oder durch veräthertes oder verestertes Hydroxy, veräthertes gegebenenfalls S-oxidiertes Mercapto und/oder in höherer als der α-Stellung durch Oxo und/oder Hydroxy substituierten, gegebenenfalls zusätzlich zu endständig gebundenem Hydroxy in Salzform vorliegendes Sulfo aufweisenden aliphatischen, einen unsubstituierten oder im Arylteil wie für $R_1$ bzw. $R_2$ angegeben substituierten arali-

phatischen oder einen unsubstituierten oder in höherer als der α-Stellung durch freies, veräthertes oder verestertes Hydroxy substituierten cycloaliphatischen oder cycloaliphatisch-aliphatischen Kohlenwasserstoffrest bedeutet oder eine Gruppe der Formel

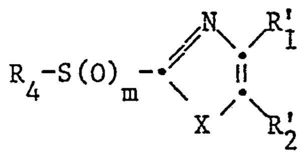

(Ia)

darstellt, in der m für 0, 1 oder 2 steht, $R_4$ einen unsubstituierten oder durch veräthertes oder verestertes Hydroxy, gegebenenfalls S-oxidiertes veräthertes Mercapto oder in höherer als der α- und in niederer als der ω-Stellung durch Oxo und/oder Hydroxy substituierten aliphatischen, gegebenenfalls durch Oxa oder Thia, welches auch S-oxidiert sein kann, unterbrochenen Kohlenwasserstoffrest, oder, sofern n und m für 0 stehen, eine direkte Bindung darstellt und $R_1'$ und $R_2'$ die für $R_1$ bzs. $R_2$ angegebenen Bedeutungen haben, mit der Massgabe, dass in Verbindungen, worin n für 1 steht, mindestens einer der Reste $R_1$ und $R_2$ von unsubstituierten oder durch Alkyl, Alkoxy, Halogen und/oder Trifluormethyl substituiertem Phenyl verschieden ist, wenn $R_3$ Alkyl bedeutet, oder ein pharmazeutisch verwendbares Salz davon zur Anwendung in einem Verfahren zur therapeutischen Behandlung des menschlichen oder tierischen Körpers.

86. Eine Verbindung gemäss Anspruch 85 der Formel I, worin $R_1$ und $R_2$ unabhängig voneinander unsubstituiertes oder durch Niederalkyl, Niederalkoxy bzw. an vicinalen C-Atomen Niederalkylidendioxy, Hydroxy, Halogen, Niederalkanoyloxy, unsubstituiertes oder durch Niederalkyl, Niederalkoxy und/oder Halogen substituiertes Benzoyl- oder Pyridoyloxy, Niederalkylthio bzw. an vicinalen C-Atomen Niederalkylidendithio, Niederalkansulfinyl, Niederalkansulfonyl, Amino, Mono- oder Diniederalkylamino, 3- bis

7-gliedriges Alkylen- bzw. 3-Aza-, 3-Oxa- oder 3-Thianiederalkylenamino, Nitro und/oder Trifluormethyl substituiertes Aryl mit 6 bis und mit 10 C-Atomen oder monocyclische, ein Sauerstoff- oder Schwefelatom und gegebenenfalls zusätzlich ein Stickstoffatom aufweisende 5-gliedrige bzw. gegebenenfalls N-oxidierte ein oder zwei Stickstoffatom(e) aufweisende 6-gliedriges Heteroaryl bedeuten, X Thio darstellt, n für 0, 1 oder 2 steht, $R_3$ Niederalkyl, über ein gesättigtes C-Atom gebundenes Niederalkenyl oder Niederalkinyl, Mono- oder Diniederalkoxyniederalkyl, Niederalkylendioxyniederalkyl, Niederalkylidendioxyniederalkyl, die Niederalkanoyloxygruppe(n) in höherer als der α-Stellung tragendes Mono- oder Diniederalkanoyloxyniederalkyl, Polyhalogenniederalkyl, Mono- oder Diniederalkylthioniederalkyl, Niederalkansulfinyl- bzw. Niederalkansulfonylniederalkyl, Niederalkylendithioniederalkyl, die Hydroxygruppe(n) bzw. die Niederalk(anoyl)oxygruppe in höherer als der α-Stellung tragendes Mono- oder Dihydroxyniederalkyl, Hydroxyniederalkoxyniederalkyl, Niederalkanoyloxyniederalkoxyniederalkyl, Niederalkoxyniederalkoxyniederalkyl, Niederalkylthioniederalkoxyniederalkyl, Hydroxyniederalkylthioniederalkyl, Niederalkoxyniederalkylthioniederalkyl oder Niederalkanoyloxyniederalkylthioniederalkyl, die Oxogruppe in höherer als der α-Stellung tragendes Oxoniederalkyl, unsubstituiertes oder wie für $R_1$ und $R_2$ angegeben substituiertes Phenylniederalkyl, 3- bis 8-gliedriges Cycloalkyl bzw. Cycloalkylniederalkyl oder die Hydroxy- bzw. Niederalk(anoyl)oxygruppe in höherer als der α-Stellung tragendes Hydroxycycloalkyl, Niederalkoxycycloalkyl, Niederalkanoyloxyniederalkyl, Cycloalkyl-(hydroxy)-niederalkyl bedeutet oder einen Rest der Formel Ia darstellt in der m für 0,1 oder 2 steht, $R_4$ Niederalkylen, über ein gesättigtes C-Atom gebundenes Niederalkenylen, Niederalkoxyniederalkylen, die Niederalkanoyloxygruppe in höherer als der α- und in niederer als der ω-Stellung tragendes Mono- oder Diniederalkanoyloxyniederalkylen, Polyhalogenniederalkylen, Niederalkylthioniederalkylen, oder die Hydroxy- bzw. Oxogruppe in höherer als der α- und in niederer als der ω-Stellung

tragendes Mono- oder Dihydroxy- bzw. Oxoniederalkylen, Oxaniederalkylen oder gegebenenfalls S-oxidiertes Thianiederalkylen bedeutet oder, sofern n und m für 0 stehen, eine direkte Bindung darstellt und $R_1'$ und $R_2'$ eine der für $R_1$ bzw. $R_2$ angegebenenen Bedeutungen haben, mit der Massgabe, dass in Verbindungen, worin n für 1 steht, mindestens einer der Reste $R_1$ und $R_2$ von unsubstituierten oder durch Niederalkyl, Niederalkoxy, Halogen und/oder Trifluormethyl substituiertem Phenyl verschieden ist, wenn $R_3$ Niederalkyl bedeutet, oder ein pharmazeutisch verwendbares Salz davon zur Anwendung gemäss Anspruch 85..

87. Eine Verbindung gemäss Anspruch 85 der Formel I, worin $R_1$ und $R_2$ unabhängig voneinander unsubstituiertes oder durch Niederalkyl, Niederalkoxy, Hydroxy, Halogen, Niederalkanoyloxy, Niederalkylthio, Niederalkansulfonyl, Diniederalkylamino, 3- bis 7-gliedriges Alkylen- bzw. 3-Aza-, 3-Oxa- oder 3-Thianiederalkylenamino und/oder Trifluormethyl substituiertes Phenyl oder unsubstituiertes oder durch Niederalkyl, Niederalkoxy oder Hydroxy substituiertes Furyl, Pyridyl bzw. 1-Oxidopyridyl bedeuten, X Thio ist, n für 0, 1 oder 2 steht, $R_3$ Niederalkyl, über ein gesättigtes C-Atom gebundenes Niederalkenyl oder Niederalkinyl, Mono- oder Diniederalkoxyniederalkyl, Niederalkylendioxyniederalkyl, Niederalkylidendioxyniederalkyl, die Niederalkanoyloxygruppe(n) in höherer als der α-Stellung tragendes Mono- oder Diniederalkanoyloxyniederalkyl, Mono- oder Diniederalkylthioniederalkyl, die Hydroxygruppe(n), die Niederalk(anoyl)-oxygruppen bzw. die Niederalkylendioxygruppe in höherer als der α-Stellung tragendes Mono- oder Dihydroxyniederalkyl, Hydroxyniederalkoxyniederalkyl oder Niederalkanoyloxyniederalkoxyniederalkyl, Niederalkoxyniederalkoxyniederalkyl, Niederalkylthioniederalkoxyniederalkyl, Hydroxyniederalkylthioniederalkyl, Niederalkoxyniederalkylthioniederalkyl oder Niederalkanoyloxyniederalkylthioniederalkyl, in Salzform vorliegendes ω-Hydroxy-ω'-sulfoniederalkyl, ω-Hydroxy-ω'-sulfo-niederalkenyl und ω-Hydroxy-ω-sulfo-niederalkinyl, ebenso/oder die Oxogruppe in höherer als der α-Stellung tragendes Oxoniederalkyl bedeutet oder einen Rest der Formel Ia darstellt, in der m für 0, 1 oder 2 steht, $R_4$ Niederalkylen, die Hydroxy-, Niederalkanoyloxy- oder Oxogruppe in höherer als der α- und in niederer als der ω-Stellung tragendes Hydroxyniederalkylen, Niederalkanoyloxyniederalkylen oder Oxoniederalkylen bedeutet oder, sofern n und m für 0 stehen, eine direkte Bindung darstellt und $R_1'$ und $R_2'$ eine der für $R_1$ bzw. $R_2$ angegebenen Bedeutungen haben,, mit der Massgabe, dass in Verbindungen, worin n für 1 steht, mindestens einer der Reste $R_1$ und $R_2$ von unsubstituierten oder durch Niederalkyl, Niederalkoxy, Halogen und/oder Trifluormethyl substituiertem Phenyl verschieden ist, wenn $R_3$ Niederalkyl bedeutet, oder ein pharmazeutisch verwendbares Salz davon zur Anwendung gemäss Anspruch 85.

88. Eine Verbindung gemäss Anspruch 85, worin $R_1$ und $R_2$ unabhängig voneinander unsubstituiertes oder durch Niederalkoxy mit bis und mit 4 C-Atomen, Niederalkylthio mit bis und mit 4 C-Atomen, Halogen der Atomnummer bis und mit 35, Hydroxy und/oder Trifluormethyl substituiertes Phenyl bedeuten, X Thio darstellt, n für 0 oder 2 steht und $R_3$ Niederalkyl bis und mit 4 C-Atomen, die Oxo-, Hydroxy-, Niederalkanoyloxy-, Niederalkoxy- oder Niederalkylendioxygruppe(n) in höherer als der α-Stellung tragendes Hydroxyniederalkyl mit 2 bis und mit 4 C-Atomen, Niederalkanoyloxyniederalkyl mit jeweils bis und mit 4 C-Atomen, Oxoniederalkyl mit bis und mit 4 C-Atomen oder Mono- oder Diniederalkoxyniederalkyl bzw. Niederalkylendioxyniederalkyl mit jeweils bis und mit 4 C-Atomen in jedem Alkyl(en)teil oder in Salzform vorliegendes ω-Hydroxy-ω-sulfo-niederalkyl mit 2 bis 4 C-Atomen, wie 2-Hydroxy-2-sulfo-äthyl, bedeutet, oder ein pharmazeutisch verwendbares Salz davon zur Anwendung gemäss Anspruch 85.

89. Eine Verbindung gemäss Anspruch 85, worin $R_1$ und $R_2$ unabhängig voneinander unsubstituiertes oder durch Niederalkoxy mit bis und mit

4 C-Atomen oder Halogen der Atomnummer bis und mit 35 substituiertes Phenyl bedeuten, X Oxy oder Tyio darstellt, n für 0, 1 oder 2 steht und $R_3$ ω-Hydroxyniederalkyl mit 2 bis 4 C-Atomen, 2- oder 3-Niederalkoxyniederalkyl bzw. 2- oder 3-Niederalkylthioniederalkyl mit bis und mit 4 C-Atomen oder 2- oder 3-( -Hydroxyniederalkoxy)- bzw. 2- oder 3-(ω-Hydroxyniederalkylthio)-niederalkyl mit bis und mit 4 C-Atomen bedeutet oder ω,ω-Diniederalkoxyniederalkyl mit jeweils bis und mit 4 C-Atomen in jedem Alkylteil, oder ω-Niederalkanoyloxyniederalkyl mit je bis und mit 4 C-Atomen bedeutet oder, sofern n für 0 oder 2 steht, Niederalkyl mit bis und mit 4 C-Atomen darstellt, oder ein pharmazeutisch verwendbares Salz davon zur Anwendung gemäss Anspruch 85.

90. 2-Hydroxyäthylthio-4,5-bis-phenyl-thiazol oder ein pharmazeutisch verwendbares Salz davon zur Anwendung gemäss Anspruch 85.

91. 2-Aethylthio-4,5-bis-phenyl-thiazol oder ein pharmazeutisch verwendbares Salz davon zur Anwendung gemäss Anspruch 85.

92. 2-(2-Hydroxyäthylthio)-4,5-bis-(p-chlorphenyl)-thiazol oder ein pharmazeutisch verwendbares Salz davon zur Anwendung gemäss Anspruch 85.

93. Eine Verbindung der Formel

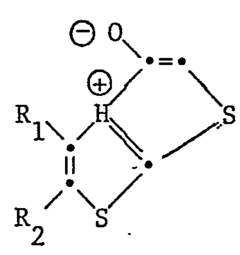

(XXIII),

worin $R_1$ und $R_2$ unabhängig voneinander unsubstituierte oder durch aliphatische Kohlenwasserstoffreste, freies, veräthertes oder verestertes Hydroxy, veräthertes, gegebenenfalls S-oxidiertes Mercapto, aliphatisch substituiertes Amino und/oder Trifluormethyl substituierte Aryl- oder gegebenenfalls N-oxidierte Heteroarylreste bedeuten.

94. Eine Verbindung gemäss Anspruch 93, worin $R_1$ und $R_2$ unabhängig voneinander unsubstituiertes oder durch Niederalkyl, Niederalkoxy bzw. an vicinalen C-Atomen Niederalkylidendioxy, Hydroxy, Halogen, Niederalkanoyloxy, unsubstituiertes oder durch Niederalkyl, Niederalkoxy und/oder Halogen substituiertes Benzoyl- oder Pyridoyloxy, Niederalkylthio bzw. an vicinalen C-Atomen Niederalkylidendithio, Niederalkansulfinyl, Niederalkansulfonyl, Amino, Mono- oder Diniederalkylamino, 3- bis 7-gliedriges Alkylen- bzw. 3-Aza-, 3-Oxa- oder 3-Thianiederalkylenamino, Nitro und/oder Trifluormethyl substituiertes Aryl mit 6 bis und mit 10 C-Atomen oder monocyclische, ein Sauerstoff- oder Schwefelatom und gegebenenfalls zusätzlich ein Stickstoffatom aufweisende 5-gliedrige bzw. gegebenenfalls N-oxidierte ein oder zwei Stickstoffatom(e) aufweisende 6-gliedriges Heteroaryl bedeuten.

95. Eine Verbindung gemäss Anspruch 93, worin mindestens einer der Reste $R_1$ und $R_2$ Pyridyl oder Thienyl und der andere unsubstituiertes oder durch Niederalkoxy mit bis und mit 4 C-Atomen oder Halogen der Atomnummer bis und mit 35 substituiertes Phenyl bedeutet.

96. Eine Verbindung gemäss Anspruch 93, worin $R_1$ und $R_2$ unabhängig voneinander unsubstituiertes oder durch Niederalkoxy mit bis und mit 4 C-Atomen oder Halogen der Atomnummer bis und mit 35 substituiertes Phenyl bedeuten.

97. 5,6-Bis-(p-methoxyphenyl)-thiazolo[2,3-b]thiazolium-oxylat.

98. 5,6-Bis-phenyl-thiazolo[2,3-b]thiazolium-oxylat.

99. 5,6-Bis-(p-chlorphenyl)-thiazolo[2,3-b]thiazolium-oxylat.

100. Verfahren zur Herstellung von Verbindungen gemäss Anspruch 93 dadurch gekennzeichnet, dass man eine Verbindung der Formel

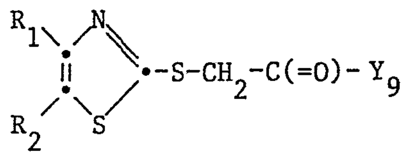

worin $Y_9$ einen abspaltbaren Rest bedeutet, cyclisiert und gewünschtenfalls eine verfahrensgemäss erhältliche Verbindung in eine andere Verbindung der Formel XXIII überführt.

101.Eine Verbindung gemäss einem der Ansprüche 1 bis 80 in freier Form oder in pharmazeutisch verwendbarer Salzform oder eine Verbindung gemäss einem der Ansprüche 93-99 zur Anwendung in einem Verfahren zur therapeutischen Behandlung des menschlichen oder tierischen Körpers.

102. Pharmazeutische Präparate enthaltend eine Verbindung gemäss einem der Ansprüche 3,7,9,11 und 13 bis 56 und gegebenenfalls in pharmazeutisch verwendbarer Salzform neben üblichen pharmazeutischen Hilfs- und/oder Trägerstoffen.

103. Pharmazeutische Präparate enthaltend eine Verbindung gemäss einem der Ansprüche 1, 2, 4 bis 6, 8, 10, 12, 57 bis 80 und 93 bis 99 gegebenenfalls in pharmazeutisch verwendbarer Salzform neben üblichen pharmazeutischen Hilfs- und/oder Trägerstoffen.

104. Eine Verbindung gemäss einem der Ansprüche 1 bis 80 und 85 bis 99 als antinociceptives Mittel.

105. Antirheumatische pharmazeutische Präparate enthaltend eine Verbindung gemäss einem der Ansprüche 1 bis 80 und 85-89 neben üblichen pharmazeutischen Hilfs- und/oder Trägerstoffen.

106. Verfahren zur Behandlung rheumatischer Erkrankungen, dadurch gekennzeichnet, dass man einem einer solchen Behandlung bedürftigen Warmblüterorganismus ein pharmazeuitsches Präparat gemäss einem der Ansprüche 102, 103 und 104 verabfolgt.